# EUROPÄISCHE PATENTSCHRIFT

(11) **EP 3 713 417 B1**
(45) Veröffentlichungstag und Bekanntmachung des Hinweises auf die Patenterteilung: **17.01.2024**
(21) Anmeldenummer: 18800199.4
(22) Anmeldetag: 15.11.2018
(51) Int. Cl.: A01N 43/653, A01N 43/713, C07D 327/04, C07D 405/12, C07D 409/12, C07D 411/12, C07D 417/04, C07D 249/14, C07D 257/06, C07C 323/62

(54) **HERBIZID WIRKSAME BIZYKLISCHE BENZAMIDE**
HERBICIDALLY ACTIVE BICYCLIC BENZAMIDES
BENZAMIDES BICYCLIQUES À ACTION HERBICIDE EFFICACE

(30) Priorität: 20.11.2017 EP 17202474
(43) Veröffentlichungstag der Anmeldung: 30.09.2020
(73) Patentinhaber: Bayer Aktiengesellschaft, 51373 Leverkusen (DE)
(72) Erfinder: MEMMEL, Frank, 67583 Guntersblum (DE); BRAUN, Ralf, 76857 Ramberg (DE); WALDRAFF, Christian, 61118 Bad Vilbel (DE); KARIG, Gunter, 65439 Flörsheim am Main (DE); DÖRNER-RIEPING, Simon, 61267 Neu-Anspach (DE); HÄUSER-HAHN, Isolde, 51375 Leverkusen (DE); MACHETTIRA, Anu, Bheemaiah, 60326 Frankfurt am Main (DE); DIETRICH, Hansjörg, 65835 Liederbach am Taunus (DE); GATZWEILER, Elmar, 61231 Bad Nauheim (DE); ROSINGER, Christopher, Hugh, 65719 Hofheim (DE)
(74) Vertreter: BIP Patents
(86) Internationale Anmeldenummer: PCT/EP2018/081327
(87) Internationale Veröffentlichungsnummer: WO 2019/096884

(56) Entgegenhaltungen:
- EP-A1- 0 918 056
- WO-A1-94/18179
- WO-A1-2012/123409
- WO-A2-2013/076315
- D'ARCANGELIS S T ET AL: "2-(1,3-Benzodithiol-2-ylidene)-1,3-benzox athiole (Dibenzo-oxatrithiafulvalene, DBOTTF): The First Tetraheterofulvalene containing Oxygen", TETRAHEDRON LETTERS, ELSEVIER, AMSTERDAM , NL, vol. 37, no. 17, 22 April 1996 (1996-04-22), pages 2931-2934, XP004029684, ISSN: 0040-4039, DOI: 10.1016/0040-4039(96)00450-9

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Speziell betrifft sie substituierte bizyklische Benzamide, Verfahren zu ihrer Herstellung und ihre Verwendung als Herbizide.

WO 2013/076315 A2 und WO 2014/184073 A1 beschreiben jeweils substituierte N-(Tetrazol-5-yl)- und N-(Triazol-5-yl)arylcarboxamide, die in 2,3- oder bevorzugt in 3,4-Position anelliert sind. WO 2012/123409 A1 offenbart in 3,4-Position mit nichtaromatischen Heterocyclen anellierte Arylcarboxamide. Die herbizide Wirkung dieser bekannten Verbindungen, insbesondere bei niedrigen Aufwandmengen, bzw. deren Verträglichkeit gegenüber Kulturpflanzen bleiben verbesserungswürdig.

Aus den genannten Gründen besteht weiterhin ein Bedarf nach wirkungsstarken Herbiziden und/oder Pflanzenwachstumsregulatoren für die selektive Anwendung in Pflanzenkulturen oder die Anwendung auf Nichtkulturland, wobei diese Wirkstoffe vorzugsweise weitere vorteilhafte Eigenschaften in der Anwendung haben sollten, wie zum Beispiel eine verbesserte Verträglichkeit gegenüber Kulturpflanzen.

Ein Gegenstand der vorliegenden Erfindung ist daher die Bereitstellung von Verbindungen mit herbizider Wirkung (Herbizide), die bereits bei relativ niedrigen Aufwandmengen gegen wirtschaftlich wichtige Schadpflanzen hochwirksam sind und vorzugsweise bei guter Wirksamkeit gegen Schadpflanzen selektiv in Kulturpflanzen eingesetzt werden können und dabei vorzugsweise eine gute Verträglichkeit gegenüber Kulturpflanzen zeigen. Bevorzugt sollten diese herbiziden Verbindungen insbesondere effektiv und effizient gegen ein breites Spektrum an Ungräsern sein, und vorzugsweise zusätzlich eine gute Wirksamkeit gegen viele Unkräuter aufweisen.

Überraschenderweise wurde nun gefunden, dass die nachfolgenden Verbindungen der Formel (I) und deren Salze eine ausgezeichnete herbizide Wirksamkeit gegen ein breites Spektrum wirtschaftlich wichtiger mono- und dikotyler annueller Schadpflanzen aufweisen.

Gegenstand der vorliegenden Erfindung sind daher Verbindungen der allgemeinen Formel (I) 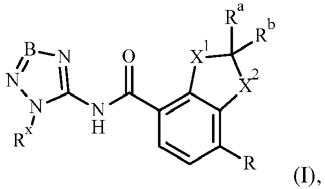 und deren agrochemisch verträglichen Salze, in welchen die Symbole und Indizes folgende Bedeutungen haben:
- B: bedeutet N oder CH,
- X¹, X²: bedeuten unabhängig voneinander jeweils jeweils O oder S(O)ₙ,
- R: bedeutet Wasserstoff, Halogen, (C₁-C₆)-Alkyl, Halogen-(C₁-C₆)-alkyl, (C₃-C₆)-Cycloalkyl, R'O oder R²(O)ₙS,
- R^{a}, R^{b}: bedeuten unabhängig voneinander jeweils Wasserstoff, Fluor, Chlor, Hydroxy, (C₁-C₆)-Alkyl, Halogen-(C₁-C₆)-alkyl, (C₁-C₆)-Alkyloxy, (C₁-C₆)-Alkylthio, Cyano, oder
- R^{a} und R^{b}: bilden gemeinsam mit dem Kohlenstoffatom, an dem sie gebunden sind eine Carbonyl- oder eine Thiocarbonylgruppe,
- R^{x}: bedeutet (C₁-C₆)-Alkyl, (C₁-C₆)-Alkyl-O-(C₁-C₆)-alkyl oder Phenyl,
- R¹: bedeutet (C₁-C₆)-Alkyl oder Halogen-(C₁-C₆)-alkyl,
- R²: bedeutet (C₁-C₆)-Alkyl,
- n: bedeutet 0, 1 oder 2.

Die Verbindungen der Formel (I) können Salze bilden. Salzbildung kann durch Einwirkung einer Base auf solche Verbindungen der Formel (I) erfolgen, die ein acides Wasserstoffatom tragen. Geeignete Basen sind beispielsweise organische Amine, wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R‴]+, worin R bis R‴ jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Alkyl bedeutet gesättigte, geradkettige oder verzweigte Kohlenwasserstoffreste mit der jeweils angegebenen Anzahl von Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Durch Halogen substitiertes Alkyl bedeutet geradkettige oder verzweigte Alkylgruppen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

Cycloalkyl bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Adamantan-1-yl und Adamantan-2-yl.

Im Falle von substituiertem Cycloalkyl werden auch spirocyclische aliphatische Systeme umfaßt, wie beispielsweise Spiro[2.2]pent-1-yl, Spiro[2.3]hex-1-yl und Spiro[2.3]hex-4-yl, 3-Spiro[2.3]hex-5-yl.

Alkoxy bedeutet gesättigte, geradkettige oder verzweigte Alkoxyreste mit der jeweils angegebenen Anzahl von Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Durch Halogen substitiertes Alkoxy bedeutet geradkettige oder verzweigte Alkoxyreste mit der jeweils angegebenen Anzahl von Kohlenstoffatomen, wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlor-fluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-1,2-difluorethoxy, 2,2-Dichlor-2-fluorethoxy, 2,2,2-Trichlorethoxy, Pentafluor-ethoxy und 1,1,1-Trifluorprop-2-oxy.

Die Bezeichnung "Halogen" bedeutet Fluor, Chlor, Brom oder Iod. Wird die Bezeichnung für einen Rest verwendet, dann bedeutet "Halogen" ein Fluor-, Chlor-, Brom- oder Iodatom.

Die Verbindungen der Formel (I) können je nach Art und Verknüpfung der Substituenten als Stereoisomere vorliegen. Sind beispielsweise ein oder mehrere asymmetrisch substiuierte Kohlenstoffatome und/oder Sulfoxide vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden.

Die Erfindung betrifft auch alle Stereoisomeren und deren Gemische, die von der Formel (I) umfaßt, jedoch nicht spezifisch definiert sind. Im Folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Ist eine Gruppe mehrfach durch Reste substituiert, so bedeutet dies, dass diese Gruppe durch einen oder mehrere gleiche oder verschiedene der genannten Reste substituiert ist.

In allen nachfolgend genannten Formeln haben die Substituenten und Symbole, sofern nicht anders definiert, dieselbe Bedeutung wie unter Formel (I) beschrieben. Pfeile in einer chemischen Formel bedeuten die Verknüpfungsorte zum restlichen Molekül.

Im Folgenden werden, jeweils für die einzelnen Substituenten, bevorzugte, besonders bevorzugte und ganz besonders bevorzugte Bedeutungen beschrieben. Die übrigen Substituenten der allgemeinen Formel (I), welche nachfolgend nicht genannt werden, weisen die oben genannte Bedeutung auf.

Bevorzugt sind Verbindungen der allgemeinen Formel (I), wobei die Symbole und Indices folgende Bedeutungen haben:
- B: bedeutet N oder CH,
- X¹, X²: bedeuten unabhängig voneinander jeweils O oder S(O)ₙ,
- R: bedeutet Wasserstoff, Chlor, Brom, Iod, (C₁-C₃)-Alkyl, Halogen-(C₁-C₃)-alkyl, (C₃-C₆)-Cycloalkyl, R'O oder R²(O)ₙS,
- R^{a}, R^{b}: bedeuten unabhängig voneinander jeweils Wasserstoff, Fluor, Chlor, Hydroxy, (C₁-C₆)-Alkyl, Halogen-(C₁-C₆)-alkyl, (C₁-C₆)-Alkyloxy, (C₁-C₆)-Alkylthio, Cyano, oder
- R^{a} und R^{b}: bilden gemeinsam mit dem Kohlenstoffatom, an dem sie gebunden sind eine Carbonyl- oder eine Thiocarbonylgruppe,
- R^{x}: bedeutet (C₁-C₃)-Alkyl oder (C₁-C₃)-Alkyl-O-(C₁-C₃)-alkyl,
- R¹: bedeutet (C₁-C₃)-Alkyl oder Halogen-(C₁-C₃)-alkyl,
- R²: bedeutet (C₁-C₃)-Alkyl,
- n: bedeutet 0, 1 oder 2.

Besonders bevorzugt sind Verbindungen der allgemeinen Formel (I), wobei die Symbole und Indices folgende Bedeutungen haben:
- B: bedeutet N oder CH,
- X¹, X²: bedeuten unabhängig voneinander jeweils O oder S(O)ₙ,
- R: bedeutet Wasserstoff, Chlor, Brom, Iod, Methyl, Ethyl, Cyclopropyl, Trifluormethyl, Difluormethyl, Pentafluorethyl, Methylsulfanyl, Methylsulfinyl, Methylsulfonyl, Ethylsulfonyl oder Methoxy,
- R^{a}, R^{b}: bedeuten unabhängig voneinander jeweils Wasserstoff, Fluor, Chlor, Methyl, Ethyl, Trifluormethyl, Difluormethyl, Methyoxy, Ethoxy, Methylthio, Ethylthio, Cyano, oder
- R^{a} und R^{b}: bilden gemeinsam mit dem Kohlenstoffatom, an dem sie gebunden sind eine Carbonyl- oder eine Thiocarbonylgruppe,
- R^{x}: bedeutet Methyl, Ethyl, Propyl, Methoxymethyl, Methoxyethyl oder 2-Methoxy-2-methyl-1-propyl,
- n: bedeutet 0, 1 oder 2.

Im Rahmen der vorliegenden Erfindung ist es möglich, die einzelnen bevorzugten und besonders bevorzugten Bedeutungen für die Substituenten beziehungsweise Indices B, X¹, X², R, R^{a}, R^{b}, R^{x}, R¹, R² und n beliebig miteinander zu kombinieren.

Das heißt, dass Verbindungen der allgemeinen Formel (I) von der vorliegenden Erfindung umfasst sind, in welchen beispielsweise der Substituent X¹ eine bevorzugte Bedeutung aufweist und die Substituenten B, X², R, R^{a}, R^{b}, R", R¹und R² sowie der Index n die allgemeine Bedeutung aufweisen oder aber der Substituent R eine bevorzugte Bedeutung aufweist, der Substituent R^{a} eine besonders bevorzugte Bedeutung aufweist und die übrigen Substituenten eine allgemeine Bedeutung aufweisen.

Verbindungen der Formel (II) sind ebenfalls neu und eignen sich sehr gut als Intermediate zur Herstellung einiger der erfindungsgemäßen Verbindungen der Formel (I). Ein weiterer Gegenstand vorliegender Erfindung sind somit Verbindungen der Formel (II), 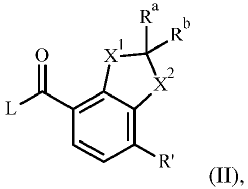 worin die Symbole und Indizes folgende Bedeutungen haben:
- L: bedeutet Halogen oder R³O,
- R³: bedeutet Wasserstoff oder (C₁-C₆)-Alkyl,
- X¹, X²: unabhängig voneinander jeweils O oder S(O)ₙ, wobei X¹ und X² nicht beide gleichzeitig O oder S(O)ₙ sind,
- R': bedeutet SO₂Me, SO₂Et, Difluormethyl, Trifluormethyl, 1,1,2,2-Tetrafluorethyl oder Pentafluorethyl,
- R^{a}, R^{b}: bedeuten unabhängig voneinander jeweils Wasserstoff, Fluor, Chlor, Hydroxy, (C₁-C₆)-Alkyl, Halogen-(C₁-C₆)-alkyl, (C₁-C₆)-Alkyloxy, (C₁-C₆)-Alkylthio, Cyano, oder
- R^{a} und R^{b}: bilden gemeinsam mit dem Kohlenstoffatom, an dem sie gebunden sind eine Carbonyl- oder eine Thiocarbonylgruppe,
- n: bedeutet 0, 1 oder 2.
- Bevorzugt sind: Verbindungen (II), worin die Symbole und Indizes folgende Bedeutungen haben:
- L: bedeutet Chlor, Methoxy, Ethoxy oder Hydroxy,
- X¹, X²: bedeuten unabhängig voneinander jeweils O oder S(O)ₙ, wobei X¹ und X² nicht beide gleichzeitig O oder S(O)ₙ sind,
- R': bedeutet SO₂Me, SO₂Et, Trifluormethyl, Difluormethyl oder Pentafluorethyl,
- R^{a}, R^{b}: bedeuten unabhängig voneinander jeweils Wasserstoff, Fluor, Chlor, Methyl, Ethyl, Trifluormethyl, Difluormethyl, Methyoxy, Ethoxy, Methylthio, Ethylthio, Cyano, oder
- R^{a} und R^{b}: bilden gemeinsam mit dem Kohlenstoffatom, an dem sie gebunden sind eine Carbonyl- oder eine Thiocarbonylgruppe,
- n: bedeutet 0, 1 oder 2.

Verbindungen der Formel (III) sind ebenfalls neu und eignen sich sehr gut als Intermediate zur

Herstellung einiger der erfindungsgemäßen Verbindungen der Formel (I). Die Verbindungen der Formel (III) sind nicht per se Gegenstand der Erfindung.

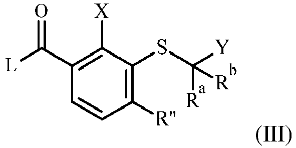

In Formel (III) haben die Symbole und Indizes folgende Bedeutungen:
- L: bedeutet Halogen oder R³O,
- R³: bedeutet Wasserstoff oder (C₁-C₆)-Alkyl,
- X: bedeutet Hydroxy, SR⁴ oder Halogen,
- Y: bedeutet Halogen,
- R": bedeutet Wasserstoff, (C₁-C₆)-Alkyl, Halogen-(C₁-C₆)-alkyl, (C₃-C₆)-Cycloalkyl, R'O, R²(O)ₙS,
- R^{a}, R^{b}: bedeuten unabhängig voneinander jeweils Wasserstoff, (C₁-C₆)-Alkyl, Halogen-(C₁-C₆)-alkyl, (C₁-C₆)-Alkyloxy, (C₁-C₆)-Alkylthio oder Cyano,
- R¹: bedeutet (C₁-C₆)-Alkyl, Halogen-(C₁-C₆)-alkyl,
- R²: bedeutet (C₁-C₆)-Alkyl,
- R⁴: bedeutet Wasserstoff, (C₁-C₆)-Alkyl, Benzyl oder 4-Methoxybenzyl,
- n: bedeutet 0, 1 oder 2.

Bevorzugt sind Verbindungen (III), worin die Symbole und Indizes folgende Bedeutungen haben:
- L: bedeutet Chlor, Methoxy, Ethoxy oder Hydroxy,
- X: bedeutet Hydroxy, SH, Methylsulfanyl oder Halogen,
- Y: bedeutet Chlor oder Brom,
- R": bedeutet Wasserstoff, Methyl, Ethyl, Cyclopropyl, Trifluormethyl, Difluormethyl, Pentafluorethyl, Methylsulfanyl, Methylsulfinyl, Methylsulfonyl, Ethylsulfonyl oder Methoxy,
- R^{a}, R^{b}: bedeuten unabhängig voneinander jeweils Wasserstoff oder Methyl.

Verbindungen der Formel (IV) sind ebenfalls neu und eignen sich sehr gut als Intermediate zur

Herstellung einiger der erfindungsgemäßen Verbindungen der Formel (I). Die Verbindungen der Formel (IV) sind nicht per se Gegenstand der Erfindung.

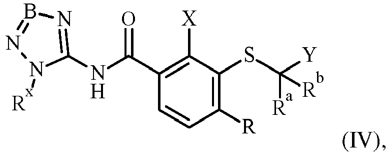

In Formel (IV) haben die Symbole und Indizes folgende Bedeutungen:
- B: bedeutet N oder CH,
- X: bedeutet Hydroxy, SR⁴, oder Halogen,
- Y: bedeutet Halogen,
- R: bedeutet Wasserstoff, Halogen, (C₁-C₆)-Alkyl, Halogen-(C₁-C₆)-alkyl, (C₃-C₆)-Cycloalkyl, R'O, R²(O)ₙS,
- R^{a}, R^{b}: bedeuten unabhängig voneinander jeweils Wasserstoff, (C₁-C₆)-Alkyl, Halogen-(C₁-C₆)-alkyl, (C₁-C₆)-Alkyloxy, (C₁-C₆)-Alkylthio oder Cyano,
- R^{x}: bedeutet (C₁-C₆)-Alkyl, (C₁-C₆)-Alkyl-O-(C₁-C₆)-alkyl, Phenyl,
- R¹: bedeutet (C₁-C₆)-Alkyl, Halogen-(C₁-C₆)-alkyl,
- R²: bedeutet (C₁-C₆)-Alkyl,
- R⁴: bedeutet Wasserstoff, (C₁-C₆)-Alkyl, Benzyl oder 4-Methoxybenzyl,
- n: bedeutet 0, 1 oder 2.
- Bevorzugt sind: Verbindungen (IV), worin die Symbole und Indizes folgende Bedeutungen haben:
- B: bedeutet N oder CH,
- X: bedeutet Hydroxy, SH oder Halogen,
- Y: bedeutet Chlor oder Brom,
- R: bedeutet Wasserstoff, Chlor, Brom, Iod, Methyl, Ethyl, Cyclopropyl, Trifluormethyl, Difluormethyl, Pentafluorethyl, Methylsulfanyl, Methylsulfinyl, Methylsulfonyl, Ethylsulfonyl, Methoxy,
- R^{a}, R^{b}: bedeuten unabhängig voneinander jeweils Wasserstoff oder Methyl,
- R^{x}: bedeutet Methyl, Ethyl, Propyl, Methoxymethyl, Methoxyethyl, 2-Methoxy-2-methyl-1-propyl.

Verbindungen der Formel (V) sind ebenfalls neu und eignen sich sehr gut als Intermediate zur Herstellung einiger der erfindungsgemäßen Verbindungen der Formel (I). Ein weiterer Gegenstand vorliegender Erfindung sind somit Verbindungen der Formel (V), 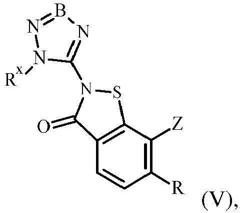 worin die Symbole und Indizes folgende Bedeutungen haben:
- B: bedeutet N oder CH,
- Z: bedeutet OR⁵, SR⁵
- R: bedeutet Wasserstoff, Halogen, (C₁-C₆)-Alkyl, Halogen-(C₁-C₆)-alkyl, (C₃-C₆)-Cycloalkyl, R'O, R²(O)ₙS,
- R^{x}: bedeutet (C₁-C₆)-Alkyl, (C₁-C₆)-Alkyl-O-(C₁-C₆)-alkyl oder Phenyl,
- R¹: bedeutet (C₁-C₆)-Alkyl, Halogen-(C₁-C₆)-alkyl,
- R²: bedeutet (C₁-C₆)-Alkyl,
- R⁵: bedeutet Wasserstoff, (C₁-C₆)-Alkyl, Allyl, Benzyl, 4-Methoxybemzyl,
- n: bedeutet 0, 1 oder 2.

Bevorzugt sind Verbindungen (V), worin die Symbole und Indizes folgende Bedeutungen haben:
- B: bedeutet N oder CH,
- Z: bedeutet OH, SH,
- R: bedeutet Wasserstoff, Chlor, Brom, Iod, Methyl, Ethyl, Cyclopropyl, Trifluormethyl, Difluormethyl, Pentafluorethyl, Methylsulfanyl, Methylsulfinyl, Methylsulfonyl, Ethylsulfonyl, Methoxy,
- R^{x}: bedeutet Methyl, Ethyl, Propyl, Methoxymethyl, Methoxyethyl, 2-Methoxy-2-methyl-1-propyl.

Die in nachfolgenden Tabellen 1 bis 4 aufgeführten erfindungsgemäßen Verbindungen der Formel (I) sowie die in Tabellen 5 bis 7 aufgeführten erfindungsgemäßen Verbindungen der Formel (II) sind ganz besonders bevorzugt.

**Tabelle 1: Verbindungen der allgemeinen Formel (I), worin B für CH und R^{x} für Methyl stehen und die anderen Substituenten und Indices die unten genannten Bedeutungen haben.**

| 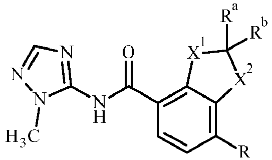 | | | | | |
|---|---|---|---|---|---|
| Nr. | X¹ | X2 | R^{a} | R^{b} | R |
| 1-1 | O | O | H | H | H |
| 1-2 | O | O | H | H | Me |
| 1-3 | O | O | H | H | CF₃ |
| 1-4 | O | O | H | H | CHF₂ |
| 1-5 | O | O | H | H | SO₂Me |
| 1-6 | O | O | F | F | H |
| 1-7 | O | O | F | F | Me |
| 1-8 | O | O | F | F | CF₃ |
| 1-9 | O | O | F | F | CHF₂ |
| 1-10 | O | O | F | F | SO₂Me |
| 1-11 | S | S | H | H | H |
| 1-12 | S | S | H | H | Me |
| 1-13 | S | S | H | H | CF₃ |
| 1-14 | S | S | H | H | CHF₂ |
| 1-15 | S | S | H | H | SO₂Me |
| 1-16 | S | S | F | F | CF₃ |
| 1-17 | S | S | F | F | SO₂Me |
| 1-18 | SO | SO | H | H | H |
| 1-19 | SO | SO | H | H | Me |
| 1-20 | SO | SO | H | H | CF₃ |
| 1-21 | SO | SO | H | H | CHF₂ |
| 1-22 | SO | SO | H | H | SO₂Me |
| 1-23 | SO₂ | SO₂ | H | H | H |
| 1-24 | SO₂ | SO₂ | H | H | Me |
| 1-25 | SO₂ | SO₂ | H | H | CF₃ |
| 1-26 | SO₂ | SO₂ | H | H | CHF₂ |
| 1-27 | SO₂ | SO₂ | H | H | SO₂Me |
| 1-28 | O | S | H | H | H |
| 1-29 | O | S | H | H | Cl |
| 1-30 | O | S | H | H | Br |
| 1-31 | O | S | H | H | I |
| 1-32 | O | S | H | H | Me |
| 1-33 | O | S | H | H | Et |
| 1-34 | O | S | H | H | c-Pr |
| 1-35 | O | S | H | H | CF₃ |
| 1-36 | O | S | H | H | CHF₂ |
| 1-37 | O | S | H | H | CF₂CF₃ |
| 1-38 | O | S | H | H | SMe |
| 1-39 | O | S | H | H | SOMe |
| 1-40 | O | S | H | H | SO₂Me |
| 1-41 | O | S | H | H | SO₂Et |
| 1-42 | O | S | H | H | OMe |
| 1-43 | O | SO | H | H | H |

| Nr. | X¹ | X² | R^{a} | R^{b} | R |
|---|---|---|---|---|---|
| 1-44 | O | SO | H | H | Cl |
| 1-45 | O | SO | H | H | Br |
| 1-46 | O | SO | H | H | I |
| 1-47 | O | SO | H | H | Me |
| 1-48 | O | SO | H | H | Et |
| 1-49 | O | SO | H | H | c-Pr |
| 1-50 | O | SO | H | H | CF₃ |
| 1-51 | O | SO | H | H | CHF₂ |
| 1-52 | O | SO | H | H | CF₂CF₃ |
| 1-53 | O | SO | H | H | SMe |
| 1-54 | O | SO | H | H | SOMe |
| 1-55 | O | SO | H | H | SO₂Me |
| 1-56 | O | SO | H | H | SO₂Et |
| 1-57 | O | SO | H | H | OMe |
| 1-58 | O | SO₂ | H | H | H |
| 1-59 | O | SO₂ | H | H | Cl |
| 1-60 | O | SO₂ | H | H | Br |
| 1-61 | O | SO₂ | H | H | I |
| 1-62 | O | SO₂ | H | H | Me |
| 1-63 | O | SO₂ | H | H | Et |
| 1-64 | O | SO₂ | H | H | c-Pr |
| 1-65 | O | SO₂ | H | H | CF₃ |
| 1-66 | O | SO₂ | H | H | CHF₂ |
| 1-67 | O | SO₂ | H | H | CF₂CF₃ |
| 1-68 | O | SO₂ | H | H | SMe |
| 1-69 | O | SO₂ | H | H | SOMe |
| 1-70 | O | SO₂ | H | H | SO₂Me |
| 1-71 | O | SO₂ | H | H | SO₂Et |
| 1-72 | O | SO₂ | H | H | OMe |
| 1-73 | O | S | Me | H | H |
| 1-74 | O | S | Me | H | Cl |
| 1-75 | O | S | Me | H | Me |
| 1-76 | O | S | Me | H | CF₃ |
| 1-77 | O | S | Me | H | CHF₂ |
| 1-78 | O | S | Me | H | SO₂Me |
| 1-79 | O | SO | Me | H | H |
| 1-80 | O | SO | Me | H | Cl |
| 1-81 | O | SO | Me | H | Me |
| 1-82 | O | SO | Me | H | CF₃ |
| 1-83 | O | SO | Me | H | CHF₂ |
| 1-84 | O | SO | Me | H | SO₂Me |
| 1-85 | O | SO₂ | Me | H | H |
| 1-86 | O | SO₂ | Me | H | Cl |
| 1-87 | O | SO₂ | Me | H | Me |
| 1-88 | O | SO₂ | Me | H | CF₃ |
| 1-89 | O | SO₂ | Me | H | CHF₂ |
| 1-90 | O | SO₂ | Me | H | SO₂Me |
| 1-91 | O | S | OMe | H | H |
| 1-92 | O | S | OMe | H | Cl |
| 1-93 | O | S | OMe | H | Me |
| 1-94 | O | S | OMe | H | CF₃ |
| 1-95 | O | S | OMe | H | CHF₂ |
| 1-96 | O | S | OMe | H | SO₂Me |
| 1-97 | O | SO | OMe | H | H |
| 1-98 | O | SO | OMe | H | Cl |
| 1-99 | O | SO | OMe | H | Me |
| 1-100 | O | SO | OMe | H | CF₃ |
| 1-101 | O | SO | OMe | H | CHF₂ |
| 1-102 | O | SO | OMe | H | SO₂Me |
| 1-103 | O | SO₂ | OMe | H | H |
| 1-104 | O | SO₂ | OMe | H | Cl |
| 1-105 | O | SO₂ | OMe | H | Me |
| 1-106 | O | SO₂ | OMe | H | CF₃ |
| 1-107 | O | SO₂ | OMe | H | CHF₂ |
| 1-108 | O | SO₂ | OMe | H | SO₂Me |
| 1-109 | O | S | OEt | H | H |
| 1-110 | O | S | OEt | H | Cl |
| 1-111 | O | S | OEt | H | Me |
| 1-112 | O | S | OEt | H | CF₃ |
| 1-113 | O | S | OEt | H | CHF₂ |
| 1-114 | O | S | OEt | H | SO₂Me |
| 1-115 | O | SO | OEt | H | H |
| 1-116 | O | SO | OEt | H | Cl |
| 1-117 | O | SO | OEt | H | Me |
| 1-118 | O | SO | OEt | H | CF₃ |
| 1-119 | O | SO | OEt | H | CHF₂ |
| 1-120 | O | SO | OEt | H | SO₂Me |
| 1-121 | O | SO₂ | OEt | H | H |
| 1-122 | O | SO₂ | OEt | H | Cl |
| 1-123 | O | SO₂ | OEt | H | Me |
| 1-124 | O | SO₂ | OEt | H | CF₃ |
| 1-125 | O | SO₂ | OEt | H | CHF₂ |
| 1-126 | O | SO₂ | OEt | H | SO₂Me |
| 1-127 | O | S | OH | H | H |
| 1-128 | O | S | OH | H | Cl |
| 1-129 | O | S | OH | H | Me |
| 1-130 | O | S | OH | H | CF₃ |
| 1-131 | O | S | OH | H | CHF₂ |
| 1-132 | O | S | OH | H | SO₂Me |
| 1-133 | O | SO | OH | H | H |
| 1-134 | O | SO | OH | H | Cl |
| 1-135 | O | SO | OH | H | Me |
| 1-136 | O | SO | OH | H | CF₃ |
| 1-137 | O | SO | OH | H | CHF₂ |
| 1-138 | O | SO | OH | H | SO₂Me |
| 1-139 | O | SO₂ | OH | H | H |
| 1-140 | O | SO₂ | OH | H | Cl |
| 1-141 | O | SO₂ | OH | H | Me |
| 1-142 | O | SO₂ | OH | H | CF₃ |
| 1-143 | O | SO₂ | OH | H | CHF₂ |
| 1-144 | O | SO₂ | OH | H | SO₂Me |
| 1-145 | O | S | SMe | H | H |
| 1-146 | O | S | SMe | H | Cl |
| 1-147 | O | S | SMe | H | Me |
| 1-148 | O | S | SMe | H | CF₃ |
| 1-149 | O | S | SMe | H | CHF₂ |
| 1-150 | O | S | SMe | H | SO₂Me |
| 1-151 | O | SO | SMe | H | H |
| 1-152 | O | SO | SMe | H | Cl |
| 1-153 | O | SO | SMe | H | Me |
| 1-154 | O | SO | SMe | H | CF₃ |
| 1-155 | O | SO | SMe | H | CHF₂ |
| 1-156 | O | SO | SMe | H | SO₂Me |
| 1-157 | O | SO₂ | SMe | H | H |
| 1-158 | O | SO₂ | SMe | H | Cl |
| 1-159 | O | SO₂ | SMe | H | Me |
| 1-160 | O | SO₂ | SMe | H | CF₃ |
| 1-161 | O | SO₂ | SMe | H | CHF₂ |
| 1-162 | O | SO₂ | SMe | H | SO₂Me |
| 1-163 | O | S | CN | H | H |
| 1-164 | O | S | CN | H | Cl |
| 1-165 | O | S | CN | H | Me |
| 1-166 | O | S | CN | H | CF₃ |
| 1-167 | O | S | CN | H | CHF₂ |
| 1-168 | O | S | CN | H | SO₂Me |
| 1-169 | O | SO | CN | H | H |
| 1-170 | O | SO | CN | H | Cl |
| 1-171 | O | SO | CN | H | Me |
| 1-172 | O | SO | CN | H | CF₃ |
| 1-173 | O | SO | CN | H | CHF₂ |
| 1-174 | O | SO | CN | H | SO₂Me |
| 1-175 | O | SO₂ | CN | H | H |
| 1-176 | O | SO₂ | CN | H | Cl |
| 1-177 | O | SO₂ | CN | H | Me |
| 1-178 | O | SO₂ | CN | H | CF₃ |
| 1-179 | O | SO₂ | CN | H | CHF₂ |
| 1-180 | O | SO₂ | CN | H | SO₂Me |
| 1-181 | O | S | C | O | H |
| 1-182 | O | S | C | O | Cl |
| 1-183 | O | S | C | O | Me |
| 1-184 | O | S | CO | | CF₃ |
| 1-185 | O | S | CO | | CHF₂ |
| 1-186 | O | S | CO | | SO₂Me |
| 1-187 | O | SO | CO | | H |
| 1-188 | O | SO | CO | | Cl |
| 1-189 | O | SO | CO | | Me |
| 1-190 | O | SO | CO | | CF₃ |
| 1-191 | O | SO | CO | | CHF₂ |
| 1-192 | O | SO | CO | | SO₂Me |
| 1-193 | O | SO₂ | CO | | H |
| 1-194 | O | SO₂ | CO | | Cl |
| 1-195 | O | SO₂ | CO | | Me |
| 1-196 | O | SO₂ | CO | | CF₃ |
| 1-197 | O | SO₂ | CO | | CHF₂ |
| 1-198 | O | SO₂ | CO | | SO₂Me |
| 1-199 | O | S | CS | | H |
| 1-200 | O | S | CS | | Cl |
| 1-201 | O | S | CS | | Me |
| 1-202 | O | S | CS | | CF₃ |
| 1-203 | O | S | CS | | CHF₂ |
| 1-204 | O | S | CS | | SO₂Me |
| 1-205 | O | SO | CS | | H |
| 1-206 | O | SO | CS | | Cl |
| 1-207 | O | SO | CS | | Me |
| 1-208 | O | SO | CS | | CF₃ |
| 1-209 | O | SO | CS | | CHF₂ |
| 1-210 | O | SO | CS | | SO₂Me |
| 1-211 | O | SO₂ | CS | | H |
| 1-212 | O | SO₂ | CS | | Cl |
| 1-213 | O | SO₂ | CS | | Me |
| 1-214 | O | SO₂ | CS | | CF₃ |
| 1-215 | O | SO₂ | CS | | CHF₂ |
| 1-216 | O | SO₂ | CS | | SO₂Me |
| 1-217 | S | O | H | H | H |
| 1-218 | S | O | H | H | Cl |
| 1-219 | S | O | H | H | Me |
| 1-220 | S | O | H | H | CF₃ |
| 1-221 | S | O | H | H | CHF₂ |
| 1-222 | S | O | H | H | SO₂Me |
| 1-223 | SO | O | H | H | H |
| 1-224 | SO | O | H | H | Cl |
| 1-225 | SO | O | H | H | Me |
| 1-226 | SO | O | H | H | CF₃ |
| 1-227 | SO | O | H | H | CHF₂ |
| 1-228 | SO | O | H | H | SO₂Me |
| 1-229 | SO₂ | O | H | H | H |
| 1-230 | SO₂ | O | H | H | Cl |
| 1-231 | SO₂ | O | H | H | Me |
| 1-232 | SO₂ | O | H | H | CF₃ |
| 1-233 | SO₂ | O | H | H | CHF₂ |
| 1-234 | SO₂ | O | H | H | SO₂Me |

**Tabelle 2: Verbindungen der allgemeinen Formel (I), worin B für N und R^{x} für Methyl stehen und die anderen Substituenten und Indices die unten genannten Bedeutungen haben.**

| 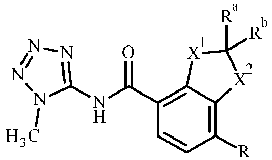 | | | | | |
|---|---|---|---|---|---|
| Nr. | X¹ | X² | R^{a} | R^{b} | R |
| 2-1 | O | O | H | H | H |
| 2-2 | O | O | H | H | Cl |
| 2-3 | O | O | H | H | Me |
| 2-4 | O | O | H | H | CF₃ |
| 2-5 | O | O | H | H | CHF₂ |
| 2-6 | O | O | H | H | SO₂Me |
| 2-7 | O | O | F | F | H |
| 2-8 | O | O | F | F | Cl |
| 2-9 | O | O | F | F | Me |
| 2-10 | O | O | F | F | c-Pr |
| 2-11 | O | O | F | F | CF₃ |
| 2-12 | O | O | F | F | CHF₂ |
| 2-13 | O | O | F | F | SMe |
| 2-14 | O | O | F | F | SOMe |
| 2-15 | O | O | F | F | SO₂Me |
| 2-16 | O | O | F | F | SO₂Et |
| 2-17 | O | O | F | F | OMe |
| 2-18 | S | S | H | H | H |
| 2-19 | S | S | H | H | Cl |
| 2-20 | S | S | H | H | Me |
| 2-21 | S | S | H | H | CF₃ |
| 2-22 | S | S | H | H | CHF₂ |
| 2-23 | S | S | H | H | SO₂Me |
| 2-24 | S | S | F | F | H |
| 2-25 | S | S | F | F | Cl |
| 2-26 | S | S | F | F | Me |
| 2-27 | S | S | F | F | c-Pr |
| 2-28 | S | S | F | F | CF₃ |
| 2-29 | S | S | F | F | CHF₂ |
| 2-30 | S | S | F | F | SOMe |
| 2-31 | S | S | F | F | SO₂Me |
| 2-32 | S | S | F | F | SO₂Et |
| 2-33 | S | S | F | F | OMe |
| 2-34 | SO | SO | H | H | H |
| 2-35 | SO | SO | H | H | Cl |
| 2-36 | SO | SO | H | H | Me |
| 2-37 | SO | SO | H | H | CF₃ |
| 2-38 | SO | SO | H | H | CHF₂ |
| 2-39 | SO | SO | H | H | SO₂Me |
| 2-40 | SO₂ | SO₂ | H | H | H |
| 2-41 | SO₂ | SO₂ | H | H | Cl |
| 2-42 | SO₂ | SO₂ | H | H | Me |
| 2-43 | SO₂ | SO₂ | H | H | CF₃ |
| 2-44 | SO₂ | SO₂ | H | H | CHF₂ |
| 2-45 | SO₂ | SO₂ | H | H | SO₂Me |
| 2-46 | O | S | H | H | H |
| 2-47 | O | S | H | H | Cl |
| 2-48 | O | S | H | H | Br |
| 2-49 | O | S | H | H | I |
| 2-50 | O | S | H | H | Me |
| 2-51 | O | S | H | H | Et |
| 2-52 | O | S | H | H | c-Pr |
| 2-53 | O | S | H | H | CF₃ |
| 2-54 | O | S | H | H | CHF₂ |
| 2-55 | O | S | H | H | CF₂CF₃ |
| 2-56 | O | S | H | H | SMe |
| 2-57 | O | S | H | H | SOMe |
| 2-58 | O | S | H | H | SO₂Me |
| 2-59 | O | S | H | H | SO₂Et |
| 2-60 | O | S | H | H | OMe |
| 2-61 | O | SO | H | H | H |
| 2-62 | O | SO | H | H | Cl |
| 2-63 | O | SO | H | H | Br |
| 2-64 | O | SO | H | H | I |
| 2-65 | O | SO | H | H | Me |
| 2-66 | O | SO | H | H | Et |
| 2-67 | O | SO | H | H | c-Pr |
| 2-68 | O | SO | H | H | CF₃ |
| 2-69 | O | SO | H | H | CHF₂ |
| 2-70 | O | SO | H | H | CF₂CF₃ |
| 2-71 | O | SO | H | H | SMe |
| 2-72 | O | SO | H | H | SOMe |
| 2-73 | O | SO | H | H | SO₂Me |
| 2-74 | O | SO | H | H | SO₂Et |
| 2-75 | O | SO | H | H | OMe |
| 2-76 | O | SO₂ | H | H | H |
| 2-77 | O | SO₂ | H | H | Cl |
| 2-78 | O | SO₂ | H | H | Br |
| 2-79 | O | SO₂ | H | H | I |
| 2-80 | O | SO₂ | H | H | Me |
| 2-81 | O | SO₂ | H | H | Et |
| 2-82 | O | SO₂ | H | H | c-Pr |
| 2-83 | O | SO₂ | H | H | CF₃ |
| 2-84 | O | SO₂ | H | H | CHF₂ |
| 2-85 | O | SO₂ | H | H | CF₂CF₃ |
| 2-86 | O | SO₂ | H | H | SMe |
| 2-87 | O | SO₂ | H | H | SOMe |
| 2-88 | O | SO₂ | H | H | SO₂Me |
| 2-89 | O | SO₂ | H | H | SO₂Et |
| 2-90 | O | SO₂ | H | H | OMe |
| 2-91 | O | S | Me | H | H |
| 2-92 | O | S | Me | H | Cl |
| 2-93 | O | S | Me | H | Me |
| 2-94 | O | S | Me | H | CF₃ |
| 2-95 | O | S | Me | H | CHF₂ |
| 2-96 | O | S | Me | H | SO₂Me |
| 2-97 | O | SO | Me | H | H |
| 2-98 | O | SO | Me | H | Cl |
| 2-99 | O | SO | Me | H | Me |
| 2-100 | O | SO | Me | H | CF₃ |
| 2-101 | O | SO | Me | H | CHF₂ |
| 2-102 | O | SO | Me | H | SO₂Me |
| 2-103 | O | SO₂ | Me | H | H |
| 2-104 | O | SO₂ | Me | H | Cl |
| 2-105 | O | SO₂ | Me | H | Me |
| 2-106 | O | SO₂ | Me | H | CF₃ |
| 2-107 | O | SO₂ | Me | H | CHF₂ |
| 2-108 | O | SO₂ | Me | H | SO₂Me |
| 2-109 | O | S | OMe | H | H |
| 2-110 | O | S | OMe | H | Cl |
| 2-111 | O | S | OMe | H | Me |
| 2-112 | O | S | OMe | H | CF₃ |
| 2-113 | O | S | OMe | H | CHF₂ |
| 2-114 | O | S | OMe | H | SO₂Me |
| 2-115 | O | SO | OMe | H | H |
| 2-116 | O | SO | OMe | H | Cl |
| 2-117 | O | SO | OMe | H | Me |
| 2-118 | O | SO | OMe | H | CF₃ |
| 2-119 | O | SO | OMe | H | CHF₂ |
| 2-120 | O | SO | OMe | H | SO₂Me |
| 2-121 | O | SO₂ | OMe | H | H |
| 2-122 | O | SO₂ | OMe | H | Cl |
| 2-123 | O | SO₂ | OMe | H | Me |
| 2-124 | O | SO₂ | OMe | H | CF₃ |
| 2-125 | O | SO₂ | OMe | H | CHF₂ |
| 2-126 | O | SO₂ | OMe | H | SO₂Me |
| 2-127 | O | S | OEt | H | H |
| 2-128 | O | S | OEt | H | Cl |
| 2-129 | O | S | OEt | H | Me |
| 2-130 | O | S | OEt | H | CF₃ |
| 2-131 | O | S | OEt | H | CHF₂ |
| 2-132 | O | S | OEt | H | SO₂Me |
| 2-133 | O | SO | OEt | H | H |
| 2-134 | O | SO | OEt | H | Cl |
| 2-135 | O | SO | OEt | H | Me |
| 2-136 | O | SO | OEt | H | CF₃ |
| 2-137 | O | SO | OEt | H | CHF₂ |
| 2-138 | O | SO | OEt | H | SO₂Me |
| 2-139 | O | SO₂ | OEt | H | H |
| 2-140 | O | SO₂ | OEt | H | Cl |
| 2-141 | O | SO₂ | OEt | H | Me |
| 2-142 | O | SO₂ | OEt | H | CF₃ |
| 2-143 | O | SO₂ | OEt | H | CHF₂ |
| 2-144 | O | SO₂ | OEt | H | SO₂Me |
| 2-145 | O | S | OH | H | H |
| 2-146 | O | S | OH | H | Cl |
| 2-147 | O | S | OH | H | Me |
| 2-148 | O | S | OH | H | CF₃ |
| 2-149 | O | S | OH | H | CHF₂ |
| 2-150 | O | S | OH | H | SO₂Me |
| 2-151 | O | SO | OH | H | H |
| 2-152 | O | SO | OH | H | Cl |
| 2-153 | O | SO | OH | H | Me |
| 2-154 | O | SO | OH | H | CF₃ |
| 2-155 | O | SO | OH | H | CHF₂ |
| 2-156 | O | SO | OH | H | SO₂Me |
| 2-157 | O | SO₂ | OH | H | H |
| 2-158 | O | SO₂ | OH | H | Cl |
| 2-159 | O | SO₂ | OH | H | Me |
| 2-160 | O | SO₂ | OH | H | CF₃ |
| 2-161 | O | SO₂ | OH | H | CHF₂ |
| 2-162 | O | SO₂ | OH | H | SO₂Me |
| 2-163 | O | S | SMe | H | H |
| 2-164 | O | S | SMe | H | Cl |
| 2-165 | O | S | SMe | H | Me |
| 2-166 | O | S | SMe | H | CF₃ |
| 2-167 | O | S | SMe | H | CHF₂ |
| 2-168 | O | S | SMe | H | SO₂Me |
| 2-169 | O | SO | SMe | H | H |
| 2-170 | O | SO | SMe | H | Cl |
| 2-171 | O | SO | SMe | H | Me |
| 2-172 | O | SO | SMe | H | CF₃ |
| 2-173 | O | SO | SMe | H | CHF₂ |
| 2-174 | O | SO | SMe | H | SO₂Me |
| 2-175 | O | SO₂ | SMe | H | H |
| 2-176 | O | SO₂ | SMe | H | Cl |
| 2-177 | O | SO₂ | SMe | H | Me |
| 2-178 | O | SO₂ | SMe | H | CF₃ |
| 2-179 | O | SO₂ | SMe | H | CHF₂ |
| 2-180 | O | SO₂ | SMe | H | SO₂Me |
| 2-181 | O | S | CN | H | H |
| 2-182 | O | S | CN | H | Cl |
| 2-183 | O | S | CN | H | Me |
| 2-184 | O | S | CN | H | CF₃ |
| 2-185 | O | S | CN | H | CHF₂ |
| 2-186 | O | S | CN | H | SO₂Me |
| 2-187 | O | SO | CN | H | H |
| 2-188 | O | SO | CN | H | Cl |
| 2-189 | O | SO | CN | H | Me |
| 2-190 | O | SO | CN | H | CF₃ |
| 2-191 | O | SO | CN | H | CHF₂ |
| 2-192 | O | SO | CN | H | SO₂Me |
| 2-193 | O | SO₂ | CN | H | H |
| 2-194 | O | SO₂ | CN | H | Cl |
| 2-195 | O | SO₂ | CN | H | Me |
| 2-196 | O | SO₂ | CN | H | CF₃ |
| 2-197 | O | SO₂ | CN | H | CHF₂ |
| 2-198 | O | SO₂ | CN | H | SO₂Me |
| 2-199 | O | S | CO | | H |
| 2-200 | O | S | CO | | Cl |
| 2-201 | O | S | CO | | Me |
| 2-202 | O | S | CO | | CF₃ |
| 2-203 | O | S | CO | | CHF₂ |
| 2-204 | O | S | CO | | SO₂Me |
| 2-205 | O | SO | CO | | H |
| 2-206 | O | SO | CO | | Cl |
| 2-207 | O | SO | CO | | Me |
| 2-208 | O | SO | CO | | CF₃ |
| 2-209 | O | SO | CO | | CHF₂ |
| 2-210 | O | SO | CO | | SO₂Me |
| 2-211 | O | SO₂ | CO | | H |
| 2-212 | O | SO₂ | CO | | Cl |
| 2-213 | O | SO₂ | CO | | Me |
| 2-214 | O | SO₂ | CO | | CF₃ |
| 2-215 | O | SO₂ | CO | | CHF₂ |
| 2-216 | O | SO₂ | CO | | SO₂Me |
| 2-217 | O | S | CS | | H |
| 2-218 | O | S | CS | | Cl |
| 2-219 | O | S | CS | | Me |
| 2-220 | O | S | CS | | CF₃ |
| 2-221 | O | S | CS | | CHF₂ |
| 2-222 | O | S | CS | | SO₂Me |
| 2-223 | O | SO | CS | | H |
| 2-224 | O | SO | CS | | Cl |
| 2-225 | O | SO | CS | | Me |
| 2-226 | O | SO | CS | | CF₃ |
| 2-227 | O | SO | CS | | CHF₂ |
| 2-228 | O | SO | CS | | SO₂Me |
| 2-229 | O | SO₂ | CS | | H |
| 2-230 | O | SO₂ | CS | | Cl |
| 2-231 | O | SO₂ | CS | | Me |
| 2-232 | O | SO₂ | CS | | CF₃ |
| 2-233 | O | SO₂ | CS | | CHF₂ |
| 2-234 | O | SO₂ | CS | | SO₂Me |
| 2-235 | S | O | H | H | H |
| 2-236 | S | O | H | H | Cl |
| 2-237 | S | O | H | H | Me |
| 2-238 | S | O | H | H | CF₃ |
| 2-239 | S | O | H | H | CHF₂ |
| 2-240 | S | O | H | H | SO₂Me |
| 2-241 | SO | O | H | H | H |
| 2-242 | SO | O | H | H | Cl |
| 2-243 | SO | O | H | H | Me |
| 2-244 | SO | O | H | H | CF₃ |
| 2-245 | SO | O | H | H | CHF₂ |
| 2-246 | SO | O | H | H | SO₂Me |
| 2-247 | SO₂ | O | H | H | H |
| 2-248 | SO₂ | O | H | H | Cl |
| 2-249 | SO₂ | O | H | H | Me |
| 2-250 | SO₂ | O | H | H | CF₃ |
| 2-251 | SO₂ | O | H | H | CHF₂ |
| 2-252 | SO₂ | O | H | H | SO₂Me |
| 2-253 | S | S | H | H | SMe |
| 2-254 | SO | S | H | H | Cl |
| 2-255 | S | SO | H | H | CF₃ |
| 2-256 | S | SO₂ | H | H | CF₃ |
| 2-257 | S | S | OMe | H | CF₃ |

**Tabelle 3: Verbindungen der allgemeinen Formel (I), worin B für N und R^{x} für Ethyl stehen und die anderen Substituenten und Indices die unten genannten Bedeutungen haben.**

| 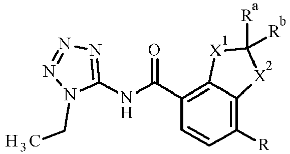 | | | | | |
|---|---|---|---|---|---|
| Nr. | X¹ | X² | R^{a} | R^{b} | R |
| 3-1 | O | O | H | H | H |
| 3-2 | O | O | H | H | Cl |
| 3-3 | O | O | H | H | Me |
| 3-4 | O | O | H | H | CF₃ |
| 3-5 | O | O | H | H | CHF₂ |
| 3-6 | O | O | H | H | SO₂Me |
| 3-7 | O | O | F | F | H |
| 3-8 | O | O | F | F | Cl |
| 3-9 | O | O | F | F | Me |
| 3-10 | O | O | F | F | c-Pr |
| 3-11 | O | O | F | F | CF₃ |
| 3-12 | O | O | F | F | CHF₂ |
| 3-13 | O | O | F | F | SOMe |
| 3-14 | O | O | F | F | SO₂Me |
| 3-15 | O | O | F | F | SO₂Et |
| 3-16 | O | O | F | F | OMe |
| 3-17 | S | S | H | H | H |
| 3-18 | S | S | H | H | Cl |
| 3-19 | S | S | H | H | Me |
| 3-20 | S | S | H | H | CF₃ |
| 3-21 | S | S | H | H | CHF₂ |
| 3-22 | S | S | H | H | SO₂Me |
| 3-23 | S | S | F | F | H |
| 3-24 | S | S | F | F | Cl |
| 3-25 | S | S | F | F | Me |
| 3-26 | S | S | F | F | c-Pr |
| 3-27 | S | S | F | F | CF₃ |
| 3-28 | S | S | F | F | CHF₂ |
| 3-29 | S | S | F | F | SOMe |
| 3-30 | S | S | F | F | SO₂Me |
| 3-31 | S | S | F | F | SO₂Et |
| 3-32 | S | S | F | F | OMe |
| 3-33 | SO | SO | H | H | H |
| 3-34 | SO | SO | H | H | Cl |
| 3-35 | SO | SO | H | H | Me |
| 3-36 | SO | SO | H | H | CF₃ |
| 3-37 | SO | SO | H | H | CHF₂ |
| 3-38 | SO | SO | H | H | SO₂Me |
| 3-39 | SO₂ | SO₂ | H | H | H |
| 3-40 | SO₂ | SO₂ | H | H | Cl |
| 3-41 | SO₂ | SO₂ | H | H | Me |
| 3-42 | SO₂ | SO₂ | H | H | CF₃ |
| 3-43 | SO₂ | SO₂ | H | H | CHF₂ |
| 3-44 | SO₂ | SO₂ | H | H | SO₂Me |
| 3-45 | O | S | H | H | H |
| 3-46 | O | S | H | H | Cl |
| 3-47 | O | S | H | H | Br |
| 3-48 | O | S | H | H | I |
| 3-49 | O | S | H | H | Me |
| 3-50 | O | S | H | H | Et |
| 3-51 | O | S | H | H | c-Pr |
| 3-52 | O | S | H | H | CF₃ |
| 3-53 | O | S | H | H | CHF₂ |
| 3-54 | O | S | H | H | CF₂CF₃ |
| 3-55 | O | S | H | H | SMe |
| 3-56 | O | S | H | H | SOMe |
| 3-57 | O | S | H | H | SO₂Me |
| 3-58 | O | S | H | H | SO₂Et |
| 3-59 | O | S | H | H | OMe |
| 3-60 | O | SO | H | H | H |
| 3-61 | O | SO | H | H | Cl |
| 3-62 | O | SO | H | H | Br |
| 3-63 | O | SO | H | H | I |
| 3-64 | O | SO | H | H | Me |
| 3-65 | O | SO | H | H | Et |
| 3-66 | O | SO | H | H | c-Pr |
| 3-67 | O | SO | H | H | CF₃ |
| 3-68 | O | SO | H | H | CHF₂ |
| 3-69 | O | SO | H | H | CF₂CF₃ |
| 3-70 | O | SO | H | H | SMe |
| 3-71 | O | SO | H | H | SOMe |
| 3-72 | O | SO | H | H | SO₂Me |
| 3-73 | O | SO | H | H | SO₂Et |
| 3-74 | O | SO | H | H | OMe |
| 3-75 | O | SO₂ | H | H | H |
| 3-76 | O | SO₂ | H | H | Cl |
| 3-77 | O | SO₂ | H | H | Br |
| 3-78 | O | SO₂ | H | H | I |
| 3-79 | O | SO₂ | H | H | Me |
| 3-80 | O | SO₂ | H | H | Et |
| 3-81 | O | SO₂ | H | H | c-Pr |
| 3-82 | O | SO₂ | H | H | CF₃ |
| 3-83 | O | SO₂ | H | H | CHF₂ |
| 3-84 | O | SO₂ | H | H | CF₂CF₃ |
| 3-85 | O | SO₂ | H | H | SMe |
| 3-86 | O | SO₂ | H | H | SOMe |
| 3-87 | O | SO₂ | H | H | SO₂Me |
| 3-88 | O | SO₂ | H | H | SO₂Et |
| 3-89 | O | SO₂ | H | H | OMe |
| 3-90 | O | S | Me | H | H |
| 3-91 | O | S | Me | H | Cl |
| 3-92 | O | S | Me | H | Me |
| 3-93 | O | S | Me | H | CF₃ |
| 3-94 | O | S | Me | H | CHF₂ |
| 3-95 | O | S | Me | H | SO₂Me |
| 3-96 | O | SO | Me | H | H |
| 3-97 | O | SO | Me | H | Cl |
| 3-98 | O | SO | Me | H | Me |
| 3-99 | O | SO | Me | H | CF₃ |
| 3-100 | O | SO | Me | H | CHF₂ |
| 3-101 | O | SO | Me | H | SO₂Me |
| 3-102 | O | SO₂ | Me | H | H |
| 3-103 | O | SO₂ | Me | H | Cl |
| 3-104 | O | SO₂ | Me | H | Me |
| 3-105 | O | SO₂ | Me | H | CF₃ |
| 3-106 | O | SO₂ | Me | H | CHF₂ |
| 3-107 | O | SO₂ | Me | H | SO₂Me |
| 3-108 | O | S | OMe | H | H |
| 3-109 | O | S | OMe | H | Cl |
| 3-110 | O | S | OMe | H | Me |
| 3-111 | O | S | OMe | H | CF₃ |
| 3-112 | O | S | OMe | H | CHF₂ |
| 3-113 | O | S | OMe | H | SO₂Me |
| 3-114 | O | SO | OMe | H | H |
| 3-115 | O | SO | OMe | H | Cl |
| 3-116 | O | SO | OMe | H | Me |
| 3-117 | O | SO | OMe | H | CF₃ |
| 3-118 | O | SO | OMe | H | CHF₂ |
| 3-119 | O | SO | OMe | H | SO₂Me |
| 3-120 | O | SO₂ | OMe | H | H |
| 3-121 | O | SO₂ | OMe | H | Cl |
| 3-122 | O | SO₂ | OMe | H | Me |
| 3-123 | O | SO₂ | OMe | H | CF₃ |
| 3-124 | O | SO₂ | OMe | H | CHF₂ |
| 3-125 | O | SO₂ | OMe | H | SO₂Me |
| 3-126 | O | S | OEt | H | H |
| 3-127 | O | S | OEt | H | Cl |
| 3-128 | O | S | OEt | H | Me |
| 3-129 | O | S | OEt | H | CF₃ |
| 3-130 | O | S | OEt | H | CHF₂ |
| 3-131 | O | S | OEt | H | SO₂Me |
| 3-132 | O | SO | OEt | H | H |
| 3-133 | O | SO | OEt | H | Cl |
| 3-134 | O | SO | OEt | H | Me |
| 3-135 | O | SO | OEt | H | CF₃ |
| 3-136 | O | SO | OEt | H | CHF₂ |
| 3-137 | O | SO | OEt | H | SO₂Me |
| 3-138 | O | SO₂ | OEt | H | H |
| 3-139 | O | SO₂ | OEt | H | Cl |
| 3-140 | O | SO₂ | OEt | H | Me |
| 3-141 | O | SO₂ | OEt | H | CF₃ |
| 3-142 | O | SO₂ | OEt | H | CHF₂ |
| 3-143 | O | SO₂ | OEt | H | SO₂Me |
| 3-144 | O | S | OH | H | H |
| 3-145 | O | S | OH | H | Cl |
| 3-146 | O | S | OH | H | Me |
| 3-147 | O | S | OH | H | CF₃ |
| 3-148 | O | S | OH | H | CHF₂ |
| 3-149 | O | S | OH | H | SO₂Me |
| 3-150 | O | SO | OH | H | H |
| 3-151 | O | SO | OH | H | Cl |
| 3-152 | O | SO | OH | H | Me |
| 3-153 | O | SO | OH | H | CF₃ |
| 3-154 | O | SO | OH | H | CHF₂ |
| 3-155 | O | SO | OH | H | SO₂Me |
| 3-156 | O | SO₂ | OH | H | H |
| 3-157 | O | SO₂ | OH | H | Cl |
| 3-158 | O | SO₂ | OH | H | Me |
| 3-159 | O | SO₂ | OH | H | CF₃ |
| 3-160 | O | SO₂ | OH | H | CHF₂ |
| 3-161 | O | SO₂ | OH | H | SO₂Me |
| 3-162 | O | S | SMe | H | H |
| 3-163 | O | S | SMe | H | Cl |
| 3-164 | O | S | SMe | H | Me |
| 3-165 | O | S | SMe | H | CF₃ |
| 3-166 | O | S | SMe | H | CHF₂ |
| 3-167 | O | S | SMe | H | SO₂Me |
| 3-168 | O | SO | SMe | H | H |
| 3-169 | O | SO | SMe | H | Cl |
| 3-170 | O | SO | SMe | H | Me |
| 3-171 | O | SO | SMe | H | CF₃ |
| 3-172 | O | SO | SMe | H | CHF₂ |
| 3-173 | O | SO | SMe | H | SO₂Me |
| 3-174 | O | SO₂ | SMe | H | H |
| 3-175 | O | SO₂ | SMe | H | Cl |
| 3-176 | O | SO₂ | SMe | H | Me |
| 3-177 | O | SO₂ | SMe | H | CF₃ |
| 3-178 | O | SO₂ | SMe | H | CHF₂ |
| 3-179 | O | SO₂ | SMe | H | SO₂Me |
| 3-180 | O | S | CN | H | H |
| 3-181 | O | S | CN | H | Cl |
| 3-182 | O | S | CN | H | Me |
| 3-183 | O | S | CN | H | CF₃ |
| 3-184 | O | S | CN | H | CHF₂ |
| 3-185 | O | S | CN | H | SO₂Me |
| 3-186 | O | SO | CN | H | H |
| 3-187 | O | SO | CN | H | Cl |
| 3-188 | O | SO | CN | H | Me |
| 3-189 | O | SO | CN | H | CF₃ |
| 3-190 | O | SO | CN | H | CHF₂ |
| 3-191 | O | SO | CN | H | SO₂Me |
| 3-192 | O | SO₂ | CN | H | H |
| 3-193 | O | SO₂ | CN | H | Cl |
| 3-194 | O | SO₂ | CN | H | Me |
| 3-195 | O | SO₂ | CN | H | CF₃ |
| 3-196 | O | SO₂ | CN | H | CHF₂ |
| 3-197 | O | SO₂ | CN | H | SO₂Me |
| 3-198 | O | S | CO | | H |
| 3-199 | O | S | CO | | Cl |
| 3-200 | O | S | CO | | Me |
| 3-201 | O | S | CO | | CF₃ |
| 3-202 | O | S | CO | | CHF₂ |
| 3-203 | O | S | CO | | SO₂Me |
| 3-204 | O | SO | CO | | H |
| 3-205 | O | SO | CO | | Cl |
| 3-206 | O | SO | CO | | Me |
| 3-207 | O | SO | CO | | CF₃ |
| 3-208 | O | SO | CO | | CHF₂ |
| 3-209 | O | SO | CO | | SO₂Me |
| 3-210 | O | SO₂ | CO | | H |
| 3-211 | O | SO₂ | CO | | Cl |
| 3-212 | O | SO₂ | CO | | Me |
| 3-213 | O | SO₂ | CO | | CF₃ |
| 3-214 | O | SO₂ | CO | | CHF₂ |
| 3-215 | O | SO₂ | CO | | SO₂Me |
| 3-216 | O | S | CS | | H |
| 3-217 | O | S | CS | | Cl |
| 3-218 | O | S | CS | | Me |
| 3-219 | O | S | CS | | CF₃ |
| 3-220 | O | S | CS | | CHF₂ |
| 3-221 | O | S | CS | | SO₂Me |
| 3-222 | O | SO | CS | | H |
| 3-223 | O | SO | CS | | Cl |
| 3-224 | O | SO | CS | | Me |
| 3-225 | O | SO | CS | | CF₃ |
| 3-226 | O | SO | CS | | CHF₂ |
| 3-227 | O | SO | CS | | SO₂Me |
| 3-228 | O | SO₂ | CS | | H |
| 3-229 | O | SO₂ | CS | | Cl |
| 3-230 | O | SO₂ | CS | | Me |
| 3-231 | O | SO₂ | CS | | CF₃ |
| 3-232 | O | SO₂ | CS | | CHF₂ |
| 3-233 | O | SO₂ | CS | | SO₂Me |
| 3-234 | S | O | H | H | H |
| 3-235 | S | O | H | H | Cl |
| 3-236 | S | O | H | H | Me |
| 3-237 | S | O | H | H | CF₃ |
| 3-238 | S | O | H | H | CHF₂ |
| 3-239 | S | O | H | H | SO₂Me |
| 3-240 | SO | O | H | H | H |
| 3-241 | SO | O | H | H | Cl |
| 3-242 | SO | O | H | H | Me |
| 3-243 | SO | O | H | H | CF₃ |
| 3-244 | SO | O | H | H | CHF₂ |
| 3-245 | SO | O | H | H | SO₂Me |
| 3-246 | SO₂ | O | H | H | H |
| 3-247 | SO₂ | O | H | H | Cl |
| 3-248 | SO₂ | O | H | H | Me |
| 3-249 | SO₂ | O | H | H | CF₃ |
| 3-250 | SO₂ | O | H | H | CHF₂ |
| 3-251 | SO₂ | O | H | H | SO₂Me |

**Tabelle 4: Verbindungen der allgemeinen Formel (I), worin B für CH und R^{x} für Propyl stehen und die anderen Substituenten und Indices die unten genannten Bedeutungen haben.**

| 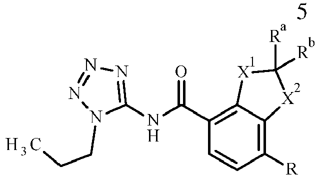 | | | | | |
|---|---|---|---|---|---|
| Nr. | X¹ | X² | R^{a} | R^{b} | R |
| 4-1 | O | O | H | H | H |
| 4-2 | O | O | H | H | Me |
| 4-3 | O | O | H | H | CF₃ |
| 4-4 | O | O | H | H | CHF₂ |
| 4-5 | O | O | H | H | SO₂Me |
| 4-6 | O | O | F | F | H |
| 4-7 | O | O | F | F | Me |
| 4-8 | O | O | F | F | CF₃ |
| 4-9 | O | O | F | F | CHF₂ |
| 4-10 | O | O | F | F | SO₂Me |
| 4-11 | S | S | H | H | H |
| 4-12 | S | S | H | H | Me |
| 4-13 | S | S | H | H | CF₃ |
| 4-14 | S | S | H | H | CHF₂ |
| 4-15 | S | S | H | H | SO₂Me |
| 4-16 | S | S | F | F | CF₃ |
| 4-17 | S | S | F | F | SO₂Me |
| 4-18 | SO | SO | H | H | H |
| 4-19 | SO | SO | H | H | Me |
| 4-20 | SO | SO | H | H | CF₃ |
| 4-21 | SO | SO | H | H | CHF₂ |
| 4-22 | SO | SO | H | H | SO₂Me |
| 4-23 | SO₂ | SO₂ | H | H | H |
| 4-24 | SO₂ | SO₂ | H | H | Me |
| 4-25 | SO₂ | SO₂ | H | H | CF₃ |
| 4-26 | SO₂ | SO₂ | H | H | CHF₂ |
| 4-27 | SO₂ | SO₂ | H | H | SO₂Me |
| 4-28 | O | S | H | H | H |
| 4-29 | O | S | H | H | Cl |
| 4-30 | O | S | H | H | Br |
| 4-31 | O | S | H | H | I |
| 4-32 | O | S | H | H | Me |
| 4-33 | O | S | H | H | Et |
| 4-34 | O | S | H | H | c-Pr |
| 4-35 | O | S | H | H | CF₃ |
| 4-36 | O | S | H | H | CHF₂ |
| 4-37 | O | S | H | H | CF₂CF₃ |
| 4-38 | O | S | H | H | SMe |
| 4-39 | O | S | H | H | SOMe |
| 4-40 | O | S | H | H | SO₂Me |
| 4-41 | O | S | H | H | SO₂Et |
| 4-42 | O | S | H | H | OMe |
| 4-43 | O | SO | H | H | H |
| 4-44 | O | SO | H | H | Cl |
| 4-45 | O | SO | H | H | Br |
| 4-46 | O | SO | H | H | I |
| 4-47 | O | SO | H | H | Me |
| 4-48 | O | SO | H | H | Et |
| 4-49 | O | SO | H | H | c-Pr |
| 4-50 | O | SO | H | H | CF₃ |
| 4-51 | O | SO | H | H | CHF₂ |
| 4-52 | O | SO | H | H | CF₂CF₃ |
| 4-53 | O | SO | H | H | SMe |
| 4-54 | O | SO | H | H | SOMe |
| 4-55 | O | SO | H | H | SO₂Me |
| 4-56 | O | SO | H | H | SO₂Et |
| 4-57 | O | SO | H | H | OMe |
| 4-58 | O | SO₂ | H | H | H |
| 4-59 | O | SO₂ | H | H | Cl |
| 4-60 | O | SO₂ | H | H | Br |
| 4-61 | O | SO₂ | H | H | I |
| 4-62 | O | SO₂ | H | H | Me |
| 4-63 | O | SO₂ | H | H | Et |
| 4-64 | O | SO₂ | H | H | c-Pr |
| 4-65 | O | SO₂ | H | H | CF₃ |
| 4-66 | O | SO₂ | H | H | CHF₂ |
| 4-67 | O | SO₂ | H | H | CF₂CF₃ |
| 4-68 | O | SO₂ | H | H | SMe |
| 4-69 | O | SO₂ | H | H | SOMe |
| 4-70 | O | SO₂ | H | H | SO₂Me |
| 4-71 | O | SO₂ | H | H | SO₂Et |
| 4-72 | O | SO₂ | H | H | OMe |
| 4-73 | O | S | Me | H | H |
| 4-74 | O | S | Me | H | Cl |
| 4-75 | O | S | Me | H | Me |
| 4-76 | O | S | Me | H | CF₃ |
| 4-77 | O | S | Me | H | CHF₂ |
| 4-78 | O | S | Me | H | SO₂Me |
| 4-79 | O | SO | Me | H | H |
| 4-80 | O | SO | Me | H | Cl |
| 4-81 | O | SO | Me | H | Me |
| 4-82 | O | SO | Me | H | CF₃ |
| 4-83 | O | SO | Me | H | CHF₂ |
| 4-84 | O | SO | Me | H | SO₂Me |
| 4-85 | O | SO₂ | Me | H | H |
| 4-86 | O | SO₂ | Me | H | Cl |
| 4-87 | O | SO₂ | Me | H | Me |
| 4-88 | O | SO₂ | Me | H | CF₃ |
| 4-89 | O | SO₂ | Me | H | CHF₂ |
| 4-90 | O | SO₂ | Me | H | SO₂Me |
| 4-91 | O | S | OMe | H | H |
| 4-92 | O | S | OMe | H | Cl |
| 4-93 | O | S | OMe | H | Me |
| 4-94 | O | S | OMe | H | CF₃ |
| 4-95 | O | S | OMe | H | CHF₂ |
| 4-96 | O | S | OMe | H | SO₂Me |
| 4-97 | O | SO | OMe | H | H |
| 4-98 | O | SO | OMe | H | Cl |
| 4-99 | O | SO | OMe | H | Me |
| 4-100 | O | SO | OMe | H | CF₃ |
| 4-101 | O | SO | OMe | H | CHF₂ |
| 4-102 | O | SO | OMe | H | SO₂Me |
| 4-103 | O | SO₂ | OMe | H | H |
| 4-104 | O | SO₂ | OMe | H | Cl |
| 4-105 | O | SO₂ | OMe | H | Me |
| 4-106 | O | SO₂ | OMe | H | CF₃ |
| 4-107 | O | SO₂ | OMe | H | CHF₂ |
| 4-108 | O | SO₂ | OMe | H | SO₂Me |
| 4-109 | O | S | OEt | H | H |
| 4-110 | O | S | OEt | H | Cl |
| 4-111 | O | S | OEt | H | Me |
| 4-112 | O | S | OEt | H | CF₃ |
| 4-113 | O | S | OEt | H | CHF₂ |
| 4-114 | O | S | OEt | H | SO₂Me |
| 4-115 | O | SO | OEt | H | H |
| 4-116 | O | SO | OEt | H | Cl |
| 4-117 | O | SO | OEt | H | Me |
| 4-118 | O | SO | OEt | H | CF₃ |
| 4-119 | O | SO | OEt | H | CHF₂ |
| 4-120 | O | SO | OEt | H | SO₂Me |
| 4-121 | O | SO₂ | OEt | H | H |
| 4-122 | O | SO₂ | OEt | H | Cl |
| 4-123 | O | SO₂ | OEt | H | Me |
| 4-124 | O | SO₂ | OEt | H | CF₃ |
| 4-125 | O | SO₂ | OEt | H | CHF₂ |
| 4-126 | O | SO₂ | OEt | H | SO₂Me |
| 4-127 | O | S | OH | H | H |
| 4-128 | O | S | OH | H | Cl |
| 4-129 | O | S | OH | H | Me |
| 4-130 | O | S | OH | H | CF₃ |
| 4-131 | O | S | OH | H | CHF₂ |
| 4-132 | O | S | OH | H | SO₂Me |
| 4-133 | O | SO | OH | H | H |
| 4-134 | O | SO | OH | H | Cl |
| 4-135 | O | SO | OH | H | Me |
| 4-136 | O | SO | OH | H | CF₃ |
| 4-137 | O | SO | OH | H | CHF₂ |
| 4-138 | O | SO | OH | H | SO₂Me |
| 4-139 | O | SO₂ | OH | H | H |
| 4-140 | O | SO₂ | OH | H | Cl |
| 4-141 | O | SO₂ | OH | H | Me |
| 4-142 | O | SO₂ | OH | H | CF₃ |
| 4-143 | O | SO₂ | OH | H | CHF₂ |
| 4-144 | O | SO₂ | OH | H | SO₂Me |
| 4-145 | O | S | SMe | H | H |
| 4-146 | O | S | SMe | H | Cl |
| 4-147 | O | S | SMe | H | Me |
| 4-148 | O | S | SMe | H | CF₃ |
| 4-149 | O | S | SMe | H | CHF₂ |
| 4-150 | O | S | SMe | H | SO₂Me |
| 4-151 | O | SO | SMe | H | H |
| 4-152 | O | SO | SMe | H | Cl |
| 4-153 | O | SO | SMe | H | Me |
| 4-154 | O | SO | SMe | H | CF₃ |
| 4-155 | O | SO | SMe | H | CHF₂ |
| 4-156 | O | SO | SMe | H | SO₂Me |
| 4-157 | O | SO₂ | SMe | H | H |
| 4-158 | O | SO₂ | SMe | H | Cl |
| 4-159 | O | SO₂ | SMe | H | Me |
| 4-160 | O | SO₂ | SMe | H | CF₃ |
| 4-161 | O | SO₂ | SMe | H | CHF₂ |
| 4-162 | O | SO₂ | SMe | H | SO₂Me |
| 4-163 | O | S | CN | H | H |
| 4-164 | O | S | CN | H | Cl |
| 4-165 | O | S | CN | H | Me |
| 4-166 | O | S | CN | H | CF₃ |
| 4-167 | O | S | CN | H | CHF₂ |
| 4-168 | O | S | CN | H | SO₂Me |
| 4-169 | O | SO | CN | H | H |
| 4-170 | O | SO | CN | H | Cl |
| 4-171 | O | SO | CN | H | Me |
| 4-172 | O | SO | CN | H | CF₃ |
| 4-173 | O | SO | CN | H | CHF₂ |
| 4-174 | O | SO | CN | H | SO₂Me |
| 4-175 | O | SO₂ | CN | H | H |
| 4-176 | O | SO₂ | CN | H | Cl |
| 4-177 | O | SO₂ | CN | H | Me |
| 4-178 | O | SO₂ | CN | H | CF₃ |
| 4-179 | O | SO₂ | CN | H | CHF₂ |
| 4-180 | O | SO₂ | CN | H | SO₂Me |
| 4-181 | O | S | C | O | H |
| 4-182 | O | S | CO | | Cl |
| 4-183 | O | S | CO | | Me |
| 4-184 | O | S | CO | | CF₃ |
| 4-185 | O | S | CO | | CHF₂ |
| 4-186 | O | S | CO | | SO₂Me |
| 4-187 | O | SO | CO | | H |
| 4-188 | O | SO | CO | | Cl |
| 4-189 | O | SO | CO | | Me |
| 4-190 | O | SO | CO | | CF₃ |
| 4-191 | O | SO | CO | | CHF₂ |
| 4-192 | O | SO | CO | | SO₂Me |
| 4-193 | O | SO₂ | CO | | H |
| 4-194 | O | SO₂ | CO | | Cl |
| 4-195 | O | SO₂ | CO | | Me |
| 4-196 | O | SO₂ | CO | | CF₃ |
| 4-197 | O | SO₂ | CO | | CHF₂ |
| 4-198 | O | SO₂ | CO | | SO₂Me |
| 4-199 | O | S | CS | | H |
| 4-200 | O | S | CS | | Cl |
| 4-201 | O | S | CS | | Me |
| 4-202 | O | S | CS | | CF₃ |
| 4-203 | O | S | CS | | CHF₂ |
| 4-204 | O | S | CS | | SO₂Me |
| 4-205 | O | SO | CS | | H |
| 4-206 | O | SO | CS | | Cl |
| 4-207 | O | SO | CS | | Me |
| 4-208 | O | SO | CS | | CF₃ |
| 4-209 | O | SO | CS | | CHF₂ |
| 4-210 | O | SO | CS | | SO₂Me |
| 4-211 | O | SO₂ | CS | | H |
| 4-212 | O | SO₂ | CS | | Cl |
| 4-213 | O | SO₂ | CS | | Me |
| 4-214 | O | SO₂ | CS | | CF₃ |
| 4-215 | O | SO₂ | CS | | CHF₂ |
| 4-216 | O | SO₂ | CS | | SO₂Me |
| 4-217 | S | O | H | H | H |
| 4-218 | S | O | H | H | Cl |
| 4-219 | S | O | H | H | Me |
| 4-220 | S | O | H | H | CF₃ |
| 4-221 | S | O | H | H | CHF₂ |
| 4-222 | S | O | H | H | SO₂Me |
| 4-223 | SO | O | H | H | H |
| 4-224 | SO | O | H | H | Cl |
| 4-225 | SO | O | H | H | Me |
| 4-226 | SO | O | H | H | CF₃ |
| 4-227 | SO | O | H | H | CHF₂ |
| 4-228 | SO | O | H | H | SO₂Me |
| 4-229 | SO₂ | O | H | H | H |
| 4-230 | SO₂ | O | H | H | Cl |
| 4-231 | SO₂ | O | H | H | Me |
| 4-232 | SO₂ | O | H | H | CF₃ |
| 4-233 | SO₂ | O | H | H | CHF₂ |
| 4-234 | SO₂ | O | H | H | SO₂Me |

**Tabelle 5: Verbindungen der allgemeinen Formel (II), worin L für Methoxy steht und die anderen Substituenten und Indices die unten genannten Bedeutungen haben.**

| 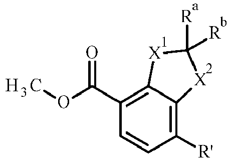 | | | | | |
|---|---|---|---|---|---|
| Nr. | X¹ | X² | R^{a} | R^{b} | R' |
| 5-1 | O | S | H | H | CF₃ |
| 5-2 | O | S | H | H | CHF₂ |
| 5-3 | O | S | H | H | CF₂CF₃ |
| 5-4 | O | SO | H | H | CF₃ |
| 5-5 | O | SO | H | H | CHF₂ |
| 5-6 | O | SO | H | H | CF₂CF₃ |
| 5-7 | O | SO₂ | H | H | CF₃ |
| 5-8 | O | SO₂ | H | H | CHF₂ |
| 5-9 | O | SO₂ | H | H | CF₂CF₃ |
| 5-10 | O | S | Me | H | CF₃ |
| 5-11 | O | S | Me | H | CHF₂ |
| 5-12 | O | SO | Me | H | CF₃ |
| 5-13 | O | SO | Me | H | CHF₂ |
| 5-14 | O | SO₂ | Me | H | CF₃ |
| 5-15 | O | SO₂ | Me | H | CHF₂ |
| 5-16 | O | S | OMe | H | CF₃ |
| 5-17 | O | S | OMe | H | CHF₂ |
| 5-18 | O | SO | OMe | H | CF₃ |
| 5-19 | O | SO | OMe | H | CHF₂ |
| 5-20 | O | SO₂ | OMe | H | CF₃ |
| 5-21 | O | SO₂ | OMe | H | CHF₂ |
| 5-22 | O | S | OEt | H | CF₃ |
| 5-23 | O | S | OEt | H | CHF₂ |
| 5-24 | O | SO | OEt | H | CF₃ |
| 5-25 | O | SO | OEt | H | CHF₂ |
| 5-26 | O | SO₂ | OEt | H | CF₃ |
| 5-27 | O | SO₂ | OEt | H | CHF₂ |
| 5-28 | O | S | OH | H | CF₃ |
| 5-29 | O | S | OH | H | CHF₂ |
| 5-30 | O | SO | OH | H | CF₃ |
| 5-31 | O | SO | OH | H | CHF₂ |
| 5-32 | O | SO₂ | OH | H | CF₃ |
| 5-33 | O | SO₂ | OH | H | CHF₂ |
| 5-34 | O | S | SMe | H | CF₃ |
| 5-35 | O | S | SMe | H | CHF₂ |
| 5-36 | O | SO | SMe | H | CF₃ |
| 5-37 | O | SO | SMe | H | CHF₂ |
| 5-38 | O | SO₂ | SMe | H | CF₃ |
| 5-39 | O | SO₂ | SMe | H | CHF₂ |
| 5-40 | O | S | CN | H | CF₃ |
| 5-41 | O | S | CN | H | CHF₂ |
| 5-42 | O | SO | CN | H | CF₃ |
| 5-43 | O | SO | CN | H | CHF₂ |
| 5-44 | O | SO₂ | CN | H | CF₃ |
| 5-45 | O | SO₂ | CN | H | CHF₂ |
| 5-46 | O | S | CO | | CF₃ |
| 5-47 | O | S | CO | | CHF₂ |
| 5-48 | O | SO | CO | | CF₃ |
| 5-49 | O | SO | CO | | CHF₂ |
| 5-50 | O | SO₂ | CO | | CF₃ |
| 5-51 | O | SO₂ | CO | | CHF₂ |
| 5-52 | O | S | CS | | CF₃ |
| 5-53 | O | S | CS | | CHF₂ |
| 5-54 | O | SO | CS | | CF₃ |
| 5-55 | O | SO | CS | | CHF₂ |
| 5-56 | O | SO₂ | CS | | CF₃ |
| 5-57 | O | SO₂ | CS | | CHF₂ |
| 5-58 | S | O | H | H | CF₃ |
| 5-59 | S | O | H | H | CHF₂ |
| 5-60 | SO | O | H | H | CF₃ |
| 5-61 | SO | O | H | H | CHF₂ |
| 5-62 | SO₂ | O | H | H | CF₃ |
| 5-63 | SO₂ | O | H | H | CHF₂ |
| 5-64 | 0 | S | H | H | SO₂Me |

**Tabelle 6: Verbindungen der allgemeinen Formel (II), wobei L für Hydroxy steht und die anderen Substituenten und Indices die unten genannten Bedeutungen haben.**

| 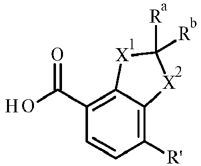 | | | | | |
|---|---|---|---|---|---|
| Nr. | X¹ | X² | R^{a} | R^{b} | R' |
| 6-1 | O | S | H | H | CF₃ |
| 6-2 | O | S | H | H | CHF₂ |
| 6-3 | O | S | H | H | CF₂CF₃ |
| 6-4 | O | SO | H | H | CF₃ |
| 6-5 | O | SO | H | H | CHF₂ |
| 6-6 | O | SO | H | H | CF₂CF₃ |
| 6-7 | O | SO₂ | H | H | CF₃ |
| 6-8 | O | SO₂ | H | H | CHF₂ |
| 6-9 | O | SO₂ | H | H | CF₂CF₃ |
| 6-10 | O | S | Me | H | CF₃ |
| 6-11 | O | S | Me | H | CHF₂ |
| 6-12 | O | SO | Me | H | CF₃ |
| 6-13 | O | SO | Me | H | CHF₂ |
| 6-14 | O | SO₂ | Me | H | CF₃ |
| 6-15 | O | SO₂ | Me | H | CHF₂ |
| 6-16 | O | S | OMe | H | CF₃ |
| 6-17 | O | S | OMe | H | CHF₂ |
| 6-18 | O | SO | OMe | H | CF₃ |
| 6-19 | O | SO | OMe | H | CHF₂ |
| 6-20 | O | SO₂ | OMe | H | CF₃ |
| 6-21 | O | SO₂ | OMe | H | CHF₂ |
| 6-22 | O | S | OEt | H | CF₃ |
| 6-23 | O | S | OEt | H | CHF₂ |
| 6-24 | O | SO | OEt | H | CF₃ |
| 6-25 | O | SO | OEt | H | CHF₂ |
| 6-26 | O | SO₂ | OEt | H | CF₃ |
| 6-27 | O | SO₂ | OEt | H | CHF₂ |
| 6-28 | O | S | OH | H | CF₃ |
| 6-29 | O | S | OH | H | CHF₂ |
| 6-30 | O | SO | OH | H | CF₃ |
| 6-31 | O | SO | OH | H | CHF₂ |
| 6-32 | O | SO₂ | OH | H | CF₃ |
| 6-33 | O | SO₂ | OH | H | CHF₂ |
| 6-34 | O | S | SMe | H | CF₃ |
| 6-35 | O | S | SMe | H | CHF₂ |
| 6-36 | O | SO | SMe | H | CF₃ |
| 6-37 | O | SO | SMe | H | CHF₂ |
| 6-38 | O | SO₂ | SMe | H | CF₃ |
| 6-39 | O | SO₂ | SMe | H | CHF₂ |
| 6-40 | O | S | CN | H | CF₃ |
| 6-41 | O | S | CN | H | CHF₂ |
| 6-42 | O | SO | CN | H | CF₃ |
| 6-43 | O | SO | CN | H | CHF₂ |
| 6-44 | O | SO₂ | CN | H | CF₃ |
| 6-45 | O | SO₂ | CN | H | CHF₂ |
| 6-46 | O | S | CO | | CF₃ |
| 6-47 | O | S | CO | | CHF₂ |
| 6-48 | O | SO | CO | | CF₃ |
| 6-49 | O | SO | CO | | CHF₂ |
| 6-50 | O | SO₂ | CO | | CF₃ |
| 6-51 | O | SO₂ | CO | | CHF₂ |
| 6-52 | O | S | CS | | CF₃ |
| 6-53 | O | S | CS | | CHF₂ |
| 6-54 | O | SO | CS | | CF₃ |
| 6-55 | O | SO | CS | | CHF₂ |
| 6-56 | O | SO₂ | CS | | CF₃ |
| 6-57 | O | SO₂ | CS | | CHF₂ |
| 6-58 | S | O | H | H | CF₃ |
| 6-59 | S | O | H | H | CHF₂ |
| 6-60 | SO | O | H | H | CF₃ |
| 6-61 | SO | O | H | H | CHF₂ |
| 6-62 | SO₂ | O | H | H | CF₃ |
| 6-63 | SO₂ | O | H | H | CHF₂ |
| 6-64 | O | S | H | H | SO₂Me |

**Tabelle 7: Verbindungen der allgemeinen Formel (II), wobei L für Chlor steht und die anderen Substituenten und Indices die unten genannten Bedeutungen haben.**

| 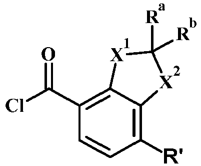 | | | | | |
|---|---|---|---|---|---|
| Nr. | X¹ | X² | R^{a} | R^{b} | R' |
| 7-1 | O | S | H | H | CF₃ |
| 7-2 | O | S | H | H | CHF₂ |
| 7-3 | O | S | H | H | CF₂CF₃ |
| 7-4 | O | SO | H | H | CF₃ |
| 7-5 | O | SO | H | H | CHF₂ |
| 7-6 | O | SO | H | H | CF₂CF₃ |
| 7-7 | O | SO₂ | H | H | CF₃ |
| 7-8 | O | SO₂ | H | H | CHF₂ |
| 7-9 | O | SO₂ | H | H | CF₂CF₃ |
| 7-10 | O | S | Me | H | CF₃ |
| 7-11 | O | S | Me | H | CHF₂ |
| 7-12 | O | SO | Me | H | CF₃ |
| 7-13 | O | SO | Me | H | CHF₂ |
| 7-14 | O | SO₂ | Me | H | CF₃ |
| 7-15 | O | SO₂ | Me | H | CHF₂ |
| 7-16 | O | S | OMe | H | CF₃ |
| 7-17 | O | S | OMe | H | CHF₂ |
| 7-18 | O | SO | OMe | H | CF₃ |
| 7-19 | O | SO | OMe | H | CHF₂ |
| 7-20 | O | SO₂ | OMe | H | CF₃ |
| 7-21 | O | SO₂ | OMe | H | CHF₂ |
| 7-22 | O | S | OEt | H | CF₃ |
| 7-23 | O | S | OEt | H | CHF₂ |
| 7-24 | O | SO | OEt | H | CF₃ |
| 7-25 | O | SO | OEt | H | CHF₂ |
| 7-26 | O | SO₂ | OEt | H | CF₃ |
| 7-27 | O | SO₂ | OEt | H | CHF₂ |
| 7-28 | O | S | OH | H | CF₃ |
| 7-29 | O | S | OH | H | CHF₂ |
| 7-30 | O | SO | OH | H | CF₃ |
| 7-31 | O | SO | OH | H | CHF₂ |
| 7-32 | O | SO₂ | OH | H | CF₃ |
| 7-33 | O | SO₂ | OH | H | CHF₂ |
| 7-34 | O | S | SMe | H | CF₃ |
| 7-35 | O | S | SMe | H | CHF₂ |
| 7-36 | O | SO | SMe | H | CF₃ |
| 7-37 | O | SO | SMe | H | CHF₂ |
| 7-38 | O | SO₂ | SMe | H | CF₃ |
| 7-39 | O | SO₂ | SMe | H | CHF₂ |
| 7-40 | O | S | CN | H | CF₃ |
| 7-41 | O | S | CN | H | CHF₂ |
| 7-42 | O | SO | CN | H | CF₃ |
| 7-43 | O | SO | CN | H | CHF₂ |
| 7-44 | O | SO₂ | CN | H | CF₃ |
| 7-45 | O | SO₂ | CN | H | CHF₂ |
| 7-46 | O | S | CO | | CF₃ |
| 7-47 | O | S | CO | | CHF₂ |
| 7-48 | O | SO | CO | | CF₃ |
| 7-49 | O | SO | CO | | CHF₂ |
| 7-50 | O | SO₂ | CO | | CF₃ |
| 7-51 | O | SO₂ | CO | | CHF₂ |
| 7-52 | O | S | CS | | CF₃ |
| 7-53 | O | S | CS | | CHF₂ |
| 7-54 | O | SO | CS | | CF₃ |
| 7-55 | O | SO | CS | | CHF₂ |
| 7-56 | O | SO₂ | CS | | CF₃ |
| 7-57 | O | SO₂ | CS | | CHF₂ |
| 7-58 | S | O | H | H | CF₃ |
| 7-59 | S | O | H | H | CHF₂ |
| 7-60 | SO | O | H | H | CF₃ |
| 7-61 | SO | O | H | H | CHF₂ |
| 7-62 | SO₂ | O | H | H | CF₃ |
| 7-63 | SO₂ | O | H | H | CHF₂ |

Erfindungsgemäße Verbindungen der Formel (I) können beispielsweise nach den in WO2012/028579 A1 angegebenen Methoden hergestellt werden. Die dazu benötigten Verbindungen der Formel (II') lassen sich durch die in Schemata 1 bis 3 beschriebenen Syntheserouten herstellen. 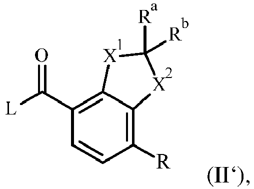 worin die Symbole und Indizes folgende Bedeutungen haben:
- L: bedeutet Halogen oder R³O,
- R³: bedeutet Wasserstoff oder (C₁-C₆)-Alkyl,
- X¹, X²: bedeuten unabhängig voneinander O oder S(O)ₙ,,
- R: bedeutet Wasserstoff, Halogen, (C₁-C₆)-Alkyl, Halogen-(C₁-C₆)-alkyl, (C₃-C₆)-Cycloalkyl, R'O, R²(O)ₙS,
- R^{a}, R^{b}: bedeuten unabhängig voneinander jeweils Wasserstoff, Fluor, Chlor, Hydroxy, (C₁-C₆)-Alkyl, Halogen-(C₁₋C₆)-alkyl, (C₁-C₆)-Alkyloxy, (C₁-C₆)-Alkylthio, Cyano, oder
- R^{a} und R^{b}: bilden gemeinsam mit dem Kohlenstoffatom, an dem sie gebunden sind eine Carbonyl- oder eine Thiocarbonylgruppe,
- n: bedeutet 0, 1 oder 2.

Die Substituenten L, R, Y, R^{a} und R^{b} haben in den in nachfolgenden Schemata 1 und 2 dargestellten Formeln die für Verbindungen der Formel (II) genannten Bedeutungen.

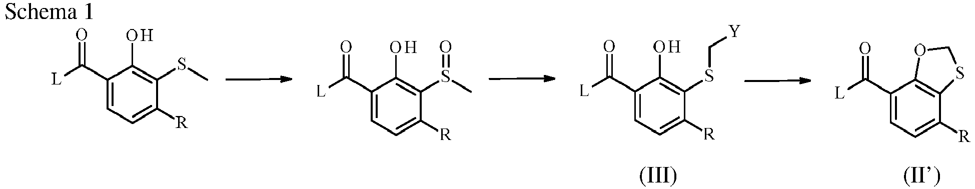

Verbindungen der Formel (II'), worin X¹ Sauerstoff und X² Schwefel bedeutet, können beispielsweise gemäß der in Schema 1 angebenen Reaktionsfolge - ausgehend von substituierten 3-Thioalkyl - Salicylsäurederivaten - durch Schwefeloxidation, Pummerer-Umlagerung und Ringbildung hergestellt werden. Die substituierten 3-Thioalkyl Salicylsäurederivaten sind grundsätzlich bekannt bzw. können nach den in US2015/322003 angegebenen Methoden hergestellt werden.

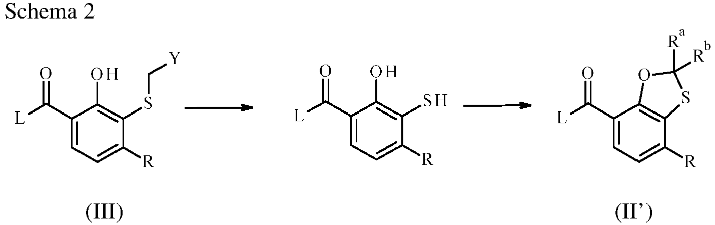

Verbindungen der Formel (III), worin X¹ Sauerstoff und X² Schwefel bedeutet, können beispielsweise auch gemäß der in Schema 2 angebenen Reaktionsfolge - ausgehend vom Pummerer-Umlagerungsprodukt in Schema 1 - durch Hydrolyse zum Thiol und anschließender Ringbildung hergestellt werden.

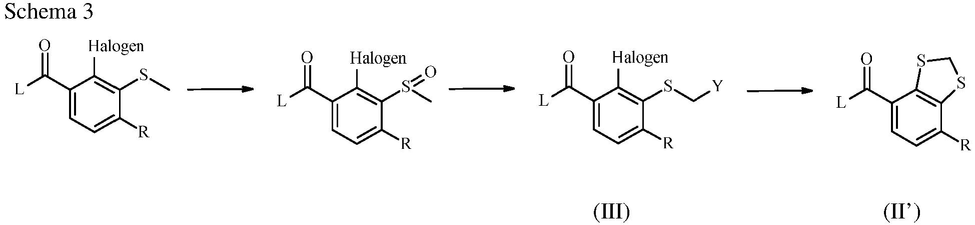

Verbindungen der Formel (III), worin X¹ und X² Schwefel bedeuten, können beispielsweise gemäß der in Schema 3 angebenen Reaktionsfolge - ausgehend von substituierten 3-Thioalkyl -2-halogenbenzoesäurederivaten - durch Schwefeloxidation, Pummerer-Umlagerung und Ringbildung durch doppelte Substitution mit Sulfid hergestellt werden. Die substituierten 3-Thioalkyl-benzoesäurederivate sind grundsätzlich bekannt bzw. können z.B. nach den in US2011/45980 oder US2008/305956 angegebenen Methoden hergestellt werden.

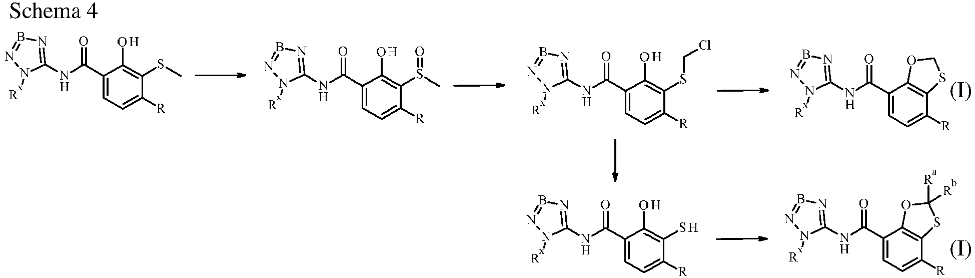

Verbindungen der Formel (I), worin X¹ Sauerstoff und X² Schwefel bedeutet, können beispielsweise auch unter Einbeziehung der in Schema 1 und 2 beschriebenen Reaktionsfolgen, angewandt auf die entspechenden Triazol-, beziehungsweise Tetrazolamide, bemäß Schema 4 hergestellt werden.

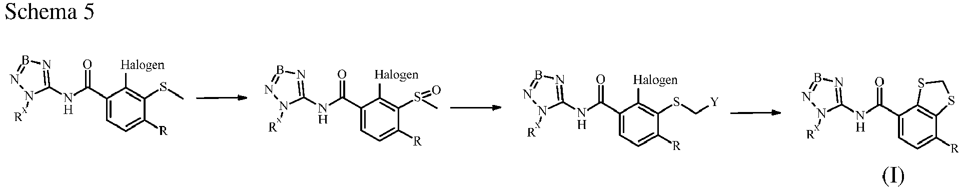

Verbindungen der Formel (I), worin X¹ und X² Schwefel bedeuten, können beispielsweise auch unter Einbeziehung der in Schema 3 beschriebenen Reaktionsfolgen, angewandt auf die entspechenden Triazol-, beziehungsweise Tetrazolamide, bemäß Schema 5 hergestellt werden.

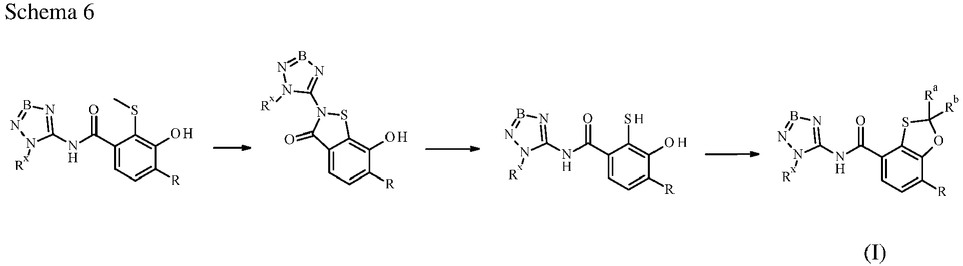

Verbindungen der Formel (I), worin X¹ Schwefel und X² Sauerstoff bedeutet, können beispielsweise gemäß der in Schema 6 angebenen Reaktionsfolge - ausgehend von 2-Thiomethyl, 3-Hydroxy-Benzamidderivaten - durch Benzisothiazolonbildung, reduktiver Ringöffnung und anschließender Ringbildung hergestellt werden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Die erfindungsgemäßen Verbindungen der Formel (I) (und/oder deren Salze), im Folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Die erfindungsgemäßen Verbindungen können in Nutzkulturen Selektivitäten aufweisen und können auch als nichtselektive Herbizide eingesetzt werden.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten in der Agrarindustrie verwendeten Wirkstoff, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften liegen in einer Toleranz oder Resistenz gegen abiotische Stressoren z.B. Hitze, Kälte, Trockenheit, Salz und ultraviolette Strahlung.

Bevorzugt ist die Anwendung der erfindungsgemäßen Verbindungen der Formel (I) oder deren Salze in wirtschaftlich bedeutenden transgenen Kulturen von Nutz-und Zierpflanzen. Die Verbindungen der Formel (I) können als Herbizide in Nutzpflanzenkulturen eingesetzt werden, welche gegenüber den phytotoxischen Wirkungen der Herbizide resistent sind bzw. gentechnisch resistent gemacht wurden.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen gentechnische Veränderungen von Kulturpflanzen zwecks Modifikation der in den Pflanzen synthetisierten Stärke (z.B. WO 92/011376 A, WO 92/014827 A, WO 91/019806 A), transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum^{™} GAT^{™} (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg, oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen. So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4-D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung OptimumTM GATTM (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxid-addukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen Wirkstoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II oder Protoporphyrinogen-Oxidase beruhen, einsetzbar, wie sie z.B. aus Weed Research 26 (1986) 441-445 oder "The Pesticide Manual", 16th edition, The British Crop Protection Council und the Royal Soc. of Chemistry, 2006 und dort zitierter Literatur beschrieben sind. Nachfolgend werden beispielhaft bekannte Herbizide oder Pflanzenwachstumsregulatoren genannt, die mit den erfindungsgemäßen Verbindungen kombiniert werden können, wobei diese Wirkstoffe entweder mit ihrem "common name" in der englischsprachigen Variante gemäß International Organization for Standardization (ISO) oder mit dem chemischen Namen bzw. mit der Codenummer bezeichnet sind. Dabei sind stets sämtliche Anwendungsformen wie beispielsweise Säuren, Salze, Ester sowie auch alle isomeren Formen wie Stereoisomere und optische Isomere umfaßt, auch wenn diese nicht explizit erwähnt sind.

Beispiele für solche herbiziden Mischungspartner sind:
Acetochlor, acifluorfen, acifluorfen-sodium, aclonifen, alachlor, allidochlor, alloxydim, alloxydimsodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlorpotassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenacsodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyranil, cyclopyrimorate, cyclosulfamuron, cycloxydim, cyhalofop, cyhalofop-butyl, cyprazine, 2,4-D, 2,4-D-butotyl, -butyl, -dimethylammonium, -diolamin, -ethyl, 2-ethylhexyl, -isobutyl, -isooctyl, - isopropylammonium, -potassium, -triisopropanolammonium und -trolamine, 2,4-DB, 2,4-DB-butyl, - dimethylammonium, isooctyl, -potassium und -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyr-sodium, dimefuron, dimepiperate, dimethachlor, dimethametryn, dimethenamid, dimethenamid-P, dimetrasulfuron, dinitramine, dinoterb, diphenamid, diquat, diquatdibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, - dimethylammonium und -methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, - potassium, -sodium und -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuronmethyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(Dimethoxyphosphoryl)-ethyl-(2,4-dichlorphenoxy)acetat, imazamethabenz, Imazamethabenz-methyl, imazamox, imazamox-ammonium, imazapic, imazapic-ammonium, imazapyr, imazapyr-isopropylammonium, imazaquin, imazaquinammonium, imazethapyr, imazethapyr-immonium, imazosulfuron, indanofan, indaziflam, iodosulfuron, iodosulfuron-methyl-sodium, ioxynil, ioxynil-octanoate, -potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl)sulfonyl)-5,5-dimethyl-4,5-dihydro-1,2-oxazol, ketospiradox, lactofen, lenacil, linuron, MCPA, MCPA-butotyl, -dimethylammonium, -2-ethylhexyl, - isopropylammonium, -potassium und -sodium, MCPB, MCPB-methyl, -ethyl und -sodium, mecoprop, mecoprop-sodium, und -butotyl, mecoprop-P, mecoprop-P-butotyl, -dimethylammonium, -2-ethylhexyl und -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, neburon, nicosulfuron, nonanoic acid (Pelargonsäure), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuronethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron, , SYN-523, SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trifluoressigsäure), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazonemethyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, sowie die folgenden Verbindungen:

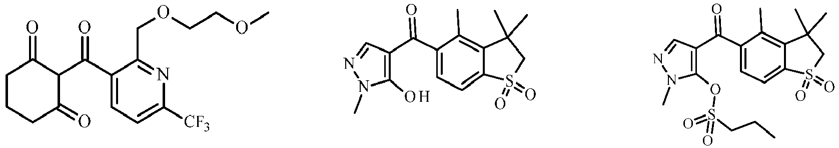

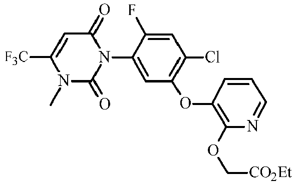

Beispiele für Pflanzenwachstumsregulatoren als mögliche Mischungspartner sind:
Acibenzolar, acibenzolar-S-methyl, 5-Aminolävulinsäure, ancymidol, 6-benzylaminopurine, Brassinolid, Catechin, chlormequat chloride, cloprop, cyclanilide, 3-(Cycloprop-1-enyl)propionsäure, daminozide, dazomet, n-decanol, dikegulac, dikegulac-sodium, endothal, endothaldipotassium, -disodium, und mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, Jasmonsäure, Jasmonsäuremethylester, maleic hydrazide, mepiquat chloride, 1-methylcyclopropene, 2-(1-naphthyl)acetamide, 1-naphthylacetic acid, 2- naphthyloxyacetic acid, nitrophenolate-mixture, 4-Oxo-4[(2-phenylethyl)amino]buttersäure, paclobutrazol, N-phenylphthalamic acid, prohexadione, prohexadione-calcium, prohydrojasmone, Salicylsäure, Strigolacton, tecnazene, thidiazuron, triacontanol, trinexapac, trinexapac-ethyl, tsitodef, uniconazole, uniconazole-P.

Safener, die in Kombination mit den erfindungsgemäßen Verbindungen der Formel (I) und ggf. in Kombinationen mit weiteren Wirkstoffen wie z.B. Insektiziden, Akariziden, Herbiziden, Fungiziden wie oben aufgelistet, eingesetzt werden können, sind vorzugsweise ausgewählt aus der Gruppe bestehend aus:
S1) Verbindungen der Formel (S1), 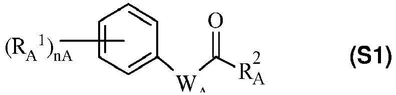 wobei die Symbole und Indizes folgende Bedeutungen haben:
   n_{A} ist eine natürliche Zahl von 0 bis 5, vorzugsweise 0 bis 3;
   R_{A}¹ ist Halogen, (C₁-C₄)Alkyl, (C₁-C₄)Alkoxy, Nitro oder (C₁-C₄)Haloalkyl;
   W_{A} ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴), 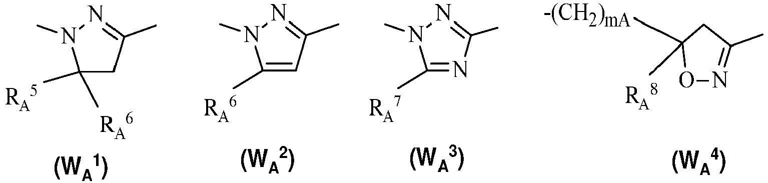
   m_{A} ist 0 oder 1;
   R_{A}² ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   R_{A}³ ist Wasserstoff oder ein unsubstituierter oder substituierter aliphatischer Kohlenwasserstoffrest, vorzugsweise mit insgesamt 1 bis 18 C-Atomen;
   R_{A}⁴ ist Wasserstoff, (C₁-C₆)Alkyl, (C₁-C₆)Alkoxy oder substituiertes oder unsubstituiertes Phenyl;
   R_{A}⁵ ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   R_{A}⁶, R_{A}⁷, R_{A}⁸ sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
      a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
      b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
      c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
      d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäure-ethylester (S1-7), und verwandte Verbindungen wie sie in EP-A-174 562 und EP-A-346 620 beschrieben sind;
      e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-3-carbonsäure (S1-10) oder 5,5-Diphenyl-2-isoxazolin-3-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 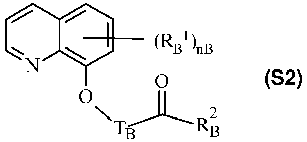 wobei die Symbole und Indizes folgende Bedeutungen haben:
   R_{B}¹ ist Halogen, (C₁-C₄)Alkyl, (C₁-C₄)Alkoxy, Nitro oder (C₁-C₄)Haloalkyl;
   n_{B} ist eine natürliche Zahl von 0 bis 5, vorzugsweise 0 bis 3;
   R_{B}² ist OR_{B}³, SR_{B}³ oder NR_{B}³R_{B}⁴ oder ein gesättigter
   oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   R_{B}³ ist Wasserstoff oder ein unsubstituierter oder substituierter aliphatischer Kohlenwasserstoffrest, vorzugsweise mit insgesamt 1 bis 18 C-Atomen;
   R_{B}⁴ ist Wasserstoff, (C₁-C₆)Alkyl, (C₁-C₆)Alkoxy oder substituiertes oder unsubstituiertes Phenyl;
   T_{B} ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
      a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium-Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
      b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Verbindungen der Formel (S3) 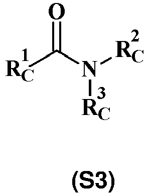 wobei die Symbole und Indizes folgende Bedeutungen haben:
   R_{C}¹ ist (C₁-C₄)Alkyl, (C₁-C₄)Haloalkyl, (C₂-C₄)Alkenyl, (C₂-C₄)Haloalkenyl, (C₃-C₇)Cycloalkyl,
   vorzugsweise Dichlormethyl;
   R_{C}², R_{C}³ sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
      Wirkstoffe vom Typ der Dichloracetamide, die häufig als Vorauflaufsafener (bodenwirksame Safener) angewendet werden, wie z. B.
      "Dichlormid" (N,N-Diallyl-2,2-dichloracetamid) (S3-1),
      "R-29148" (3-Dichloracetyl-2,2,5-trimethyl-1,3-oxazolidin) der Firma Stauffer (S3-2),
      "R-28725" (3-Dichloracetyl-2,2,-dimethyl-1,3-oxazolidin) der Firma Stauffer (S3-3),
      "Benoxacor" (4-Dichloracetyl-3,4-dihydro-3-methyl-2H-1,4-benzoxazin) (S3-4),
      "PPG-1292" (N-Allyl-N-[(1,3-dioxolan-2-yl)-methyl]-dichloracetamid) der Firma PPG Industries (S3-5),
      "DKA-24" (N-Allyl-N-[(allylaminocarbonyl)methyl]-dichloracetamid) der Firma Sagro-Chem (S3-6),
      "AD-67" oder "MON 4660" (3-Dichloracetyl-1-oxa-3-aza-spiro[4,5]decan) der Firma Nitrokemia bzw.
      Monsanto (S3-7),
      "TI-35" (1-Dichloracetyl-azepan) der Firma TRI-Chemical RT (S3-8),
      "Diclonon" (Dicyclonon) oder "BAS145138" oder "LAB145138" (S3-9)
      ((RS)-1-Dichloracetyl-3,3,8a-trimethylperhydropyrrolo[1,2-a]pyrimidin-6-on) der Firma BASF,
      "Furilazol" oder "MON 13900" ((RS)-3-Dichloracetyl-5-(2-furyl)-2,2-dimethyloxazolidin) (S3-10);
      sowie dessen (R)-Isomer (S3-11).
S4) N-Acylsulfonamide der Formel (S4) und ihre Salze, 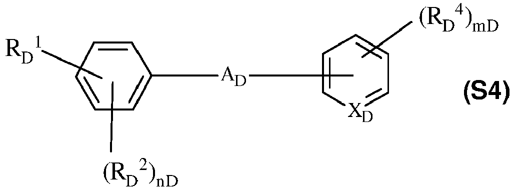 worin die Symbole und Indizes folgende Bedeutungen haben:
   A_{D} ist SO₂-NR_{D}³-CO oder CO-NR_{D}³-SO₂
   X_{D} ist CH oder N;
   R_{D}¹ ist CO-NR_{D}⁵R_{D}⁶ oder NHCO-R_{D}⁷;
   R_{D}² ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   R_{D}³ ist Wasserstoff, (C₁-C₄)Alkyl, (C₂-C₄)Alkenyl oder (C₂-C₄)Alkinyl;
   R_{D}⁴ ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   R_{D}⁵ ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
   R_{D}⁶ ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
   R_{D}⁵ und R_{D}⁶ gemeinsam mit dem dem sie tragenden Stickstoffatom einen Pyrrolidinyl- oder Piperidinyl-Rest bilden;
   R_{D}⁷ ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   n_{D} ist 0, 1 oder 2;
   m_{D} ist 1 oder 2;
   v_{D} ist 0, 1, 2 oder 3;
   davon bevorzugt sind Verbindungen vom Typ der N-Acylsulfonamide, z.B. der nachfolgenden Formel (S4^{a}), die z. B. bekannt sind aus WO-A-97/45016 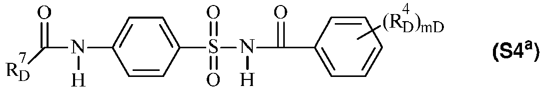
   worin
   R_{D}⁷ (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   R_{D}⁴ Halogen, (C₁-C₄)Alkyl, (C₁-C₄)Alkoxy, CF₃;
   m_{D} 1 oder 2;
   v_{D} ist 0, 1, 2 oder 3 bedeutet;
   sowie
   Acylsulfamoylbenzoesäureamide, z.B. der nachfolgenden Formel (S4^{b}), die z.B. bekannt sind aus WO-A-99/16744, 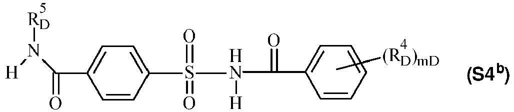
   z.B. solche worin
   R_{D}⁵ = Cyclopropyl und (R_{D}⁴) = 2-OMe ist ("Cyprosulfamide", S4-1),
   R_{D}⁵ = Cyclopropyl und (R_{D}⁴) = 5-Cl-2-OMe ist (S4-2),
   R_{D}⁵ = Ethyl und (R_{D}⁴) = 2-OMe ist (S4-3),
   R_{D}⁵ = Isopropyl und (R_{D}⁴) = 5-Cl-2-OMe ist (S4-4) und
   R_{D}⁵ = Isopropyl und (R_{D}⁴) = 2-OMe ist (S4-5).
   sowie
   Verbindungen vom Typ der N-Acylsulfamoylphenylharnstoffe der Formel (S4^{c}), die z.B. bekannt sind aus der EP-A-365484, 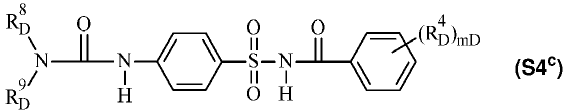
   worin
   R_{D}⁸ und R_{D}⁹ unabhängig voneinander Wasserstoff, (C₁-C₈)Alkyl, (C₃-C₈)Cycloalkyl, (C₃-C₆)Alkenyl, (C₃-C₆)Alkinyl,
   R_{D}⁴ Halogen, (C₁-C₄)Alkyl, (C₁-C₄)Alkoxy, CF₃
   m_{D} 1 oder 2 bedeutet;
   beispielsweise
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff,
   sowie
   N-Phenylsulfonylterephthalamide der Formel (S4^{d}), die z.B. bekannt sind aus CN 101838227, 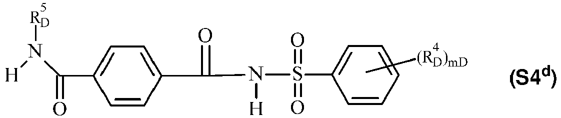
   z.B. solche worin
   R_{D}⁴ Halogen, (C₁-C₄)Alkyl, (C₁-C₄)Alkoxy, CF₃;
   m_{D} 1 oder 2;
   R_{D}⁵ Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl bedeutet.
S5) Wirkstoffe aus der Klasse der Hydroxyaromaten und der aromatisch-aliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7),wie sie in der WO-A-1998/38856 beschrieben sind 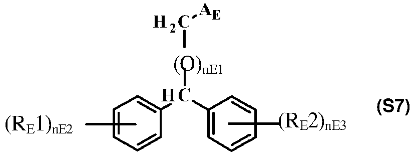 worin die Symbole und Indizes folgende Bedeutungen haben:
   R_{E}¹, R_{E}² sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   A_{E} ist COOR_{E}³ oder COSR_{E}⁴
   R_{E}³, R_{E}⁴ sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   n_{E}¹ ist 0 oder 1
   n_{E}², n_{E}³ sind unabhängig voneinander 0, 1 oder 2,
   vorzugsweise:
      Diphenylmethoxyessigsäure,
      Diphenylmethoxyessigsäureethylester,
      Diphenylmethoxyessigsäuremethylester (CAS-Reg.Nr. 41858-19-9) (S7-1).
S8) Verbindungen der Formel (S8),wie sie in der WO-A-98/27049 beschrieben sind 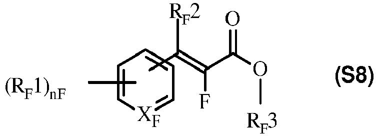 worin
   X_{F} CH oder N,
   n_{F} für den Fall, dass X_{F}=N ist, eine ganze Zahl von 0 bis 4 und
      für den Fall, dass X_{F}=CH ist, eine ganze Zahl von 0 bis 5 ,
   R_{F}¹ Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   R_{F}² Wasserstoff oder (C₁-C₄)Alkyl
   R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; bedeuten, oder deren Salze,
   vorzugsweise Verbindungen worin
   X_{F} CH,
   n_{F} eine ganze Zahl von 0 bis 2 ,
   R_{F}¹ Halogen, (C₁-C₄)Alkyl, (C₁-C₄)Haloalkyl, (C₁-C₄)Alkoxy, (C₁-C₄)Haloalkoxy,
   R_{F}² Wasserstoff oder (C₁-C₄)Alkyl,
   R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten,
   oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b})
   wie sie in der WO-A-2007/023719 und WO-A-2007/023764 beschrieben sind 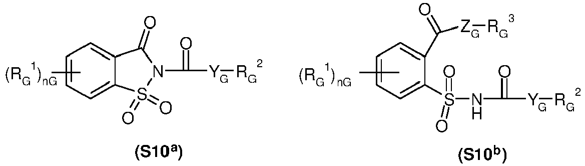 worin
   R_{G}¹ Halogen, (C₁-C₄)Alkyl, Methoxy, Nitro, Cyano, CF₃; OCF₃
   Y_{G}, Z_{G} unabhängig voneinander O oder S,
   n_{G} eine ganze Zahl von 0 bis 4,
   R_{G}² (C₁-C₁₆)Alkyl, (C₂-C₆)Alkenyl, (C₃-C₆)Cycloalkyl, Aryl; Benzyl, Halogenbenzyl,
   R_{G}³ Wasserstoff oder (C₁-C₆)Alkyl bedeutet.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG 838" (CAS-Reg.Nr. 133993-74-5) (2-propenyl 1-oxa-4-azaspiro[4.5]decan-4-carbodithioat) (S13-6) der Firma Nitrokemia,
   "Disulfoton" (O,O-Diethyl S-2-ethylthioethyl phosphordithioat) (S13-7),
   "Dietholate" (O,O-Diethyl-O-phenylphosphorothioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY 93" (S-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC 940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere
   wie sie in der WO-A-2008/131861 und WO-A-2008/131860 beschrieben sind, worin 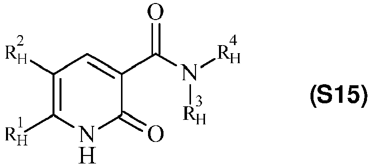
   R_{H}¹ einen (C₁-C₆)Haloalkylrest bedeutet und
   R_{H}² Wasserstoff oder Halogen bedeutet und
   R_{H}³, R_{H}⁴ unabhängig voneinander Wasserstoff, (C₁-C₁₆)Alkyl, (C₂-C₁₆)Alkenyl oder (C₂-C₁₆)Alkinyl,
   wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
   wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-_{C4})Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   bedeutet oder
   R_{H}³ (C₁-C₄)-Alkoxy, (C₂-C₄)Alkenyloxy, (C₂-C₆)Alkinyloxy oder (C₂-C₄)Haloalkoxy bedeutet und
   R_{H}⁴ Wasserstoff oder (C₁-C₄)-Alkyl bedeutet oder
   R_{H}³ und R_{H}⁴ zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Besonders bevorzugte Safener sind Mefenpyr-diethyl, Cyprosulfamid, Isoxadifen-ethyl, Cloquintocetmexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolether-sulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepoly-glykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfett-säureester oder Polyoxethylensorbitanester wie z.B. Polyoxyethylensorbitan-fettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoff-konzentration z.B. etwa 10 bis 90% Gew.-%, der Rest zu 100 Gew.-% besteht aus üblichen Formulierungsbestandteilen. Bei emulgierbaren Konzentraten kann die Wirkstoffkonzentration etwa 1 bis 90%, vorzugsweise 5 bis 80% Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80%, vorzugsweise 2 bis 50 Gew.-% Wirkstoff. Bei wasser-dispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80% Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Trägerstoff bedeutet eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels. Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden. Im Allgemeinen enthalten die erfindungsgemäßen Mittel und Formulierungen zwischen 0,05 und 99 Gew.-%, 0,01 und 98 Gew.-%, vorzugsweise zwischen 0,1 und 95 Gew.-%, besonders bevorzugt zwischen 0,5 und 90% % Wirkstoff, ganz besonders bevorzugt zwischen 10 und 70 Gewichtsprozent. Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl dispergierbare Pulver, Öl mischbare fließfähige Konzentrate, Öl mischbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgut-behandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen. Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus oder Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis. Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430, US 5,876,739, US 2003/0176428 A1, WO 2002/080675 A1, WO 2002/028186 A2.

Die erfindungsgemäßen Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser. Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende Hauptanbaupflanzen erwähnt: Mais, Sojabohne, Baumwolle, Brassica Ölsaaten wie Brassica napus (z.B. Canola), Brassica rapa, B. juncea (z.B. (Acker-)Senf) und Brassica carinata, Reis, Weizen Zuckerrübe, Zurckerrohr, Hafer, Roggen, Gerste, Hirse, Triticale, Flachs, Wein und verschiedene Früchte und Gemüse von verschiedenen botanischen Taxa wie z.B. Rosaceae sp. (beispielsweise Kernfrüchte wie Apfel und Birne, aber auch Steinfrüchte wie Aprikosen, Kirschen, Mandeln und Pfirsiche und Beerenfrüchte wie Erdbeeren), Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp. (beispielsweise Bananenbäume und -plantagen), Rubiaceae sp. (beispielsweise Kaffee), Theaceae sp., Sterculiceae sp., Rutaceae sp. (beispielsweise Zitronen, Organen und Grapefruit); Solanaceae sp. (beispielsweise Tomaten, Kartoffeln, Pfeffer, Auberginen), Liliaceae sp., Compositae sp. (beispielsweise Salat, Artischocke and Chicoree - einschließlich Wurzelchicoree, Endivie oder gemeinen Chicoree), Umbelliferae sp. (beispielsweise Karrotte, Petersilie, Stangensellerie und Knollensellerie), Cucurbitaceae sp. (beispielsweise Gurke - einschließlich Gewürzgurke, Kürbis, Wassermelone, Flaschenkürbis und Melonen), Alliaceae sp. (beispielsweise Lauch und Zwiebel), Cruciferae sp. (beispielsweise Weißkohl, Rotkohl, Brokkoli, Blumenkohl, Rosenkohl, Pak Choi, Kohlrabi, Radieschen, Meerrettich, Kresse und Chinakohl), Leguminosae sp. (beispielsweise Erdnüsse, Erbsen, und Bohnen - wie z.B. Stangenbohne und Ackerbohne), Chenopodiaceae sp. (beispielsweise Mangold, Futterrübe, Spinat, Rote Rübe), Malvaceae (beispielsweise Okra), Asparagaceae (beispielsweise Spargel); Nutzpflanzen und Zierpflanzen in Garten und Wald; sowie jeweils genetisch modifizierte Arten dieser Pflanzen.

Wie oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann bei der Behandlung von genetisch modifizierten Organismen (GMOs), z.B. Pflanzen (wie z.B. Kulturpflanzen und Bäume) oder Samen, verwendet werden. Bei genetisch modifizierten Pflanzen (oder transgenen Pflanzen) handelt es sich um Pflanzen, bei denen ein heterologes Gen stabil in das Genom integriert wurde. Der Ausdruck "heterologes Gen" bedeutet im Wesentlichen ein Gen, das außerhalb der Pflanze oder der Pflanzenzelle bereitgestellt oder assembliert wird und das, wenn es in das Zellkerngenom, das Chloroplastengenom oder das Mitochondriengenom eingeführt wird, der transformierten Pflanze neue oder verbesserte agronomische oder sonstige Merkmale verleiht, und zwar dadurch, dass es ein Protein oder Polypeptid von Interesse exprimiert oder dass es ein anderes Gen, das in der Pflanze vorliegt, bzw. andere Gene, die in der Pflanze vorliegen, herunterreguliert oder abschaltet (z.B. mittels Antisense-Technologie, Cosuppressionstechnologie, RNA-Interferenz-Technologie (RNAi-Technologie) oder MikroRNA-Technologie (miRNA-Technologie)). Ein heterologes Gen, das in das Genom integriert worden ist, wird auch als Transgen bezeichnet. Ein Transgen, das in das Pflanzengenom integriert worden ist, wird Transformations-Event oder transgenes Event genannt.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind z.B. die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser-oder Bodensalzgehalt, erhöhte Blühleistung, Ernteerleichterung, Reifebeschleunigung, höhere Erträge, größere Früchte, größere Pflanzenhöhe, intensivere grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte Mikroorganismen. Dies kann gegebenenfalls einer der Gründe für die erhöhte Wirksamkeit der erfindungsgemäßen Kombinationen sein, z.B. gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten Mikroorganismen inokkuliert werden, einen beträchtlichen Resistenzgrad gegen diese Mikroorganismen aufweisen. Im vorliegenden Fall versteht man unter unerwünschten Mikroorganismen phytopathogene Pilze, Bakterien und Viren. Die erfindungsgemäßen Substanzen lassen sich daher zum Schutz von Pflanzen gegen Angriff durch die oben erwähnten Pathogene innerhalb eines gewissen Zeitraums nach der Behandlung einsetzen. Der Zeitraum, über den eine Schutzwirkung erzielt wird, erstreckt sich im Allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Beispiele für nematoden- oder insektenresistente Pflanzen sind z.B. in den US-Patentanmeldungen 11/765,491, 11/765,494, 10/926,819, 10/782,020, 12/032,479, 10/783,417, 10/782,096, 11/657,964, 12/192,904, 11/396,808, 12/166,253, 12/166,239, 12/166,124, 12/166,209, 11/762,886, 12/364,335, 11/763,947, 12/252,453, 12/209,354, 12/491,396, 12/497,221, 12/644,632, 12/646,004, 12/701,058, 12/718,059, 12/721,595, 12/638,591 und in WO 11/002992, WO 11/014749, WO 11/103247, WO 11/103248, WO 12/135436, WO 12/135501, WO 2013134523, WO 2013134535, WO 2014036238, WO 2014126986A1 WO 2014138339, WO 2014003769, WO 2015021367, WO 2015021354, WO 2015077525, WO 2015038262, WO 2015041769, WO 2015088937A beschrieben.

Beispiele von Pflanzenresistenzen gegen andere Pathogenarten werden z.B. in WO 13/050410, WO 2013127988, WO 2013135726 WO 2015036378, WO 2015036469, WO 2015177206 beschrieben.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können z.B. Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und nicht-Stress-Bedingungen) beeinflusst werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Aufplatzen von Schoten sowie Standfestigkeit. Zu den weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im Allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine durch Inzucht erzeugte pollensterile Elternlinie (den weiblichen Kreuzungspartner) mit einer anderen durch Inzucht erzeugten pollenfertilen Elternlinie (dem männlichen Kreuzungspartner) kreuzt. Das Hybridsaatgut wird typischerweise von den pollensterilen Pflanzen geerntet und an Erzeuger verkauft. Pollensterile Pflanzen können manchmal (z. B. beim Mais) durch Entfahnen, d. h. mechanisches Entfernen der männlichen Geschlechtsorgane (bzw. der männlichen Blüten), produziert werden; es ist jedoch üblicher, dass die Pollensterilität auf genetischen Determinanten im Pflanzengenom beruht, insbesondere dann, wenn es sich bei dem gewünschten Saatgut, das man von den Hybridpflanzen ernten will, um die Samen handelt, wird üblicherweise sichergestellt, dass die Pollenfertilität in Hybridpflanzen wiederhergestellt wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden z.B. für *Brassica*-Arten beschrieben (WO 92/05251, WO 95/09910, WO 98/27806, WO 05/002324, WO 06/021972 und US 6,229,072). Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei z.B. eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden (z.B. WO 91/02069). Weitere Pflanzen, welche männliche Sterilitätsgene und fruchtbarkeitswiederherstellende Gene beinhalten, sowie Systeme für die Hybridsaatgutherstellung werden z.B. in WO 2014170387 und WO 2014195152 beschrieben.

Pflanzen oder Pflanzensorten (die mit Methoden der Pflanzenbiotechnologie, wie der Gentechnik, erhalten werden), die erfindungsgemäß behandelt werden können, sind auch herbizidtolerante Pflanzen, d.h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidresistente Pflanzen sind z.B. Glyphosate-tolerante Pflanzen, d.h. Pflanzen, die gegenüber dem Herbizid Glyphosate oder dessen Salzen tolerant gemacht worden sind. Pflanzen können auf verschiedene Weisen Glyphosate-tolerant gemacht werden. So können z.B. Glyphosate-tolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium* (Science 1983, 221, 370-371), das CP4-Gen des Bakteriums *Agrobacterium sp.* (Curr. Topics Plant Physiol. 1992, 7, 139-145), die Gene, die für eine EPSPS aus der Petunie (Science 1986, 233, 478-481), für eine EPSPS aus der Tomate (J. Biol. Chem. 1988, 263, 4280-4289) oder für eine EPSPS aus Eleusine (WO 01/66704) kodieren. Es kann sich auch um eine mutierte EPSPS handeln, wie sie z.B. in EP 0837944, WO 00/66746, WO 00/66747 oder WO 02/26995, WO 2011/000498 beschrieben ist. Glyphosate-tolerante Pflanzen können auch dadurch erhalten werden, dass man ein Gen exprimiert, das für ein Glyphosate-Oxidoreduktase-Enzym, wie es in US 5,776,760 und US 5,463,175 beschrieben ist, kodiert. Glyphosate-tolerante Pflanzen können auch dadurch erhalten werden, dass man ein Gen exprimiert, das für ein Glyphosate-acetyltransferase-Enzym, wie es in z.B. WO 02/036782, WO 03/092360, WO 05/012515 und WO 07/024782 beschrieben ist, kodiert. Glyphosate-tolerante Pflanzen können auch dadurch erhalten werden, dass man Pflanzen, die natürlich vorkommende Mutationen der oben erwähnten Gene, wie sie z.B. in WO 01/024615 oder WO 03/013226 beschrieben sind, enthalten, selektiert. Pflanzen, die Glyphosate-Toleranz vermittelnde EPSPS-Gene exprimieren, sind z.B. in den US-Patentanmeldungen 11/517,991, 10/739,610, 12/139,408, 12/352,532, 11/312,866, 11/315,678, 12/421,292, 11/400,598, 11/651,752, 11/681,285, 11/605,824, 12/468,205, 11/760,570, 11/762,526, 11/769,327, 11/769,255, 11/943801 oder 12/362,774 beschrieben. Pflanzen, die andere eine Glyphosate-Toleranz vermittelnde Gene enthalten, wie Decarboxylase-Gene, sind z.B. in den US-Patentanmeldungen 11/588,811, 11/185,342, 12/364,724, 11/185,560 oder 12/423,926 beschrieben.

Sonstige herbizidresistente Pflanzen sind z.B. Pflanzen, die gegenüber Herbiziden, die das Enzym Glutaminsynthase hemmen, wie Bialaphos, Phosphinothricin oder Glufosinate, tolerant gemacht worden sind. Solche Pflanzen können dadurch erhalten werden, dass man ein Enzym exprimiert, das das Herbizid entgiftet, oder eine Mutante des Enzyms Glutaminsynthase verwendet, das gegenüber Hemmung resistent ist, z.B. beschrieben in der US-Patentanmeldung 11/760,602. Solch ein wirksames entgiftendes Enzym ist z.B. ein Enzym, das für eine Phosphinothricin-acetyltransferase kodiert (wie z.B. das bar- oder pat-Protein aus Streptomyces-Arten). Pflanzen, die eine exogene Phosphinothricin-acetyltransferase exprimieren, sind z.B. in den US-Patenten 5,561,236; 5,648,477; 5,646,024; 5,273,894; 5,637,489; 5,276,268; 5,739,082; 5,908,810 und 7,112,665 beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei HPPD handelt es sich um ein Enzym, das die Reaktion, in der para-Hydroxyphenylpyruvat (HPP) zu Homogentisat umgesetzt wird, katalysiert. Pflanzen, die gegenüber HPPD-Hemmern tolerant sind, können mit einem Gen, das für ein natürlich vorkommendes resistentes HPPD-Enzym kodiert, oder einem Gen, das für ein mutiertes oder chimäres HPPD-Enzym gemäß WO 96/38567, WO 99/24585, WO 99/24586, WO 09/144079, WO 02/046387 oder US 6,768,044, WO 11/076877, WO 11/076882, WO 11/076885, WO 11/076889, WO 11/076892, WO 13/026740, WO 13/092552, WO 13/092551, WO 12/092555, WO 2014043435, WO 2015138394, WO 2015135881 kodiert, transformiert werden.

Eine Toleranz gegenüber HPPD-Hemmern kann auch dadurch erzielt werden, dass man Pflanzen mit Genen transformiert, die für solche Enzyme kodieren, die die Bildung von Homogentisat trotz Hemmung des nativen HPPD-Enzyms durch den HPPD-Hemmer ermöglichen. Solche Pflanzen und Gene sind in WO 99/34008 und WO 02/36787 beschrieben. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Enzym mit Prephenatdehydrogenase-Aktivität (PDH-Aktivität) kodiert, wie dies in WO 04/024928 beschrieben ist. Außerdem können Pflanzen dadurch toleranter gegenüber HPPD-Hemmer-Herbiziden gemacht werden, dass man ihrem Genom ein Gen hinzufügt, welches ein Enzym kodiert, das zur Metabolisierung oder zum Abbau von HPPD-Hemmern, wie den in WO 07/103567 und WO 08/150473 gezeigten CYP450-Enzymen, befähigt ist.

Noch weitere herbizidresistente Pflanzen sind Pflanzen, die gegenüber Acetolactatsynthase (ALS)-Hemmern tolerant gemacht worden sind. Zu bekannten ALS-Hemmern zählen z.B. Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen, wie dies z.B. bei Tranel und Wright (Weed Science 2002, 50, 700-712) jedoch auch in den US-Patenten 5,605,011, 5,378,824, 5,141,870 und 5,013,659, beschrieben ist. Die Herstellung von Sulfonylharnstoff-toleranten Pflanzen und Imidazolinon-toleranten Pflanzen ist in den US-Patenten 5,605,011; 5,013,659; 5,141,870; 5,767,361; 5,731,180; 5,304,732; 4,761,373; 5,331,107; 5,928,937; und 5,378,824; sowie in WO 96/33270 beschrieben. Weitere Imidazolinon-tolerante Pflanzen sind auch in z.B. WO 04/040012, WO 04/106529, WO 05/020673, WO 05/093093, WO 06/007373, WO 06/015376, WO 06/024351 und WO 06/060634 beschrieben. Weitere Sulfonylharnstoff- und Imidazolinon-tolerante Pflanzen sind auch in z.B. WO 07/024782, WO 11/076345, WO 12/058223, WO 12/150335, WO 2013127766, WO 2014090760, WO 2015004242, WO 2015024957 WO 2015082413 und in der US-Patentanmeldung 61/288958 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden, wie dies z.B. für die Sojabohne in US 5,084,082, für Reis in WO 97/41218, für die Zuckerrübe in US 5,773,702 und WO 99/057965, für Salat in dem US 5,198,599 oder für die Sonnenblume in WO 01/065922 beschrieben ist.

Pflanzen, die gegen 2,4-D oder Dicamba tolerant sind werden z.B. in US6153401 beschrieben.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für Folgendes kodiert:
1) ein insektizides Kristallprotein aus *Bacillus thuringiensis* oder einen insektiziden Teil davon, wie die insektiziden Kristallproteine, die von Crickmore et al(Microbiology and Molecular Biology Reviews 1998, 62, 807-813), von Crickmore et al. (2005) in der Bacillus thuringiensis-Toxinnomenklatur aktualisiert, online bei: http://www.lifesci.sussex.ac.uk/Home/Neil_Crickmore/Bt/), zusammengestellt wurden, oder insektizide Teile davon, z.B. Proteine der Cry-Proteinklassen Cry1Ab, Cry1Ac, Cry1B, Cry1C, Cry1D, Cry1F, Cry2Ab, Cry3Aa oder Cry3Bb oder insektizide Teile davon (z.B. EP-A 1999141 und WO 07/107302), oder derartige von synthetischen Genen kodierte Proteine, wie z.B. in WO 2013134523, WO 2013134535 und der US-Patentanmeldung 12/249,016 beschrieben; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cry34 und Cry35 (Nat. Biotechnol. 2001, 19, 668-72; Applied Environm. Microbiol. 2006, 71, 1765-1774) besteht, oder das binäre Toxin, das aus dem Cry1A- oder Cry1F-Protein und dem Cry2Aa- oder Cry2Ab- oder Cry2Ae-Protein besteht (US-Patentanmeldung 12/214,022 und EP-A 2 300 618) besteht; oder
3) ein insektizides Hybridprotein, das Teile von unterschiedlichen insektiziden Kristallproteinen aus *Bacillus thuringiensis* umfaßt, wie z.B. ein Hybrid aus den Proteinen von 1) oben oder ein Hybrid aus den Proteinen von 2) oben, z. B. das Protein Cry1A.105, das von dem Mais-Event MON89034 produziert wird (WO 07/027777); oder
4) ein Protein gemäß einem der Punkte 1) bis 3) oben, wobei einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der betroffenen Zielinsektenarten zu erweitern und/oder wegen Veränderungen, die in die Kodier-DNA während der Klonierung oder Transformation induziert wurden, wie das Protein Cry3Bb1 in Mais-Events MON863 oder MON88017 oder das Protein Cry3A im Mais-Event MIR604; oder
5) ein insektizides sezerniertes Protein aus *Bacillus thuringiensis* oder *Bacillus cereus* oder einen insektiziden Teil davon, wie die vegetativ wirkenden insektiziden Proteine (vegetative insecticidal proteins, VIP), die unter http://www.lifesci.sussex.ac.uk/home/Neil Crickmore/Bt/vip.html, angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht (WO 94/21795) oder
7) ein insektizides Hybridprotein, das Teile von verschiedenen sezernierten Proteinen von *Bacillus thuringiensis* oder *Bacillus cereus* umfaßt, wie ein Hybrid der Proteine von 1) oben oder ein Hybrid der Proteine von 2) oben; oder
8) ein Protein gemäß einem der Punkte 5) bis 7) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der betroffenen Zielinsektenart zu erweitern und/oder wegen Veränderungen, die in die Kodier-DNA während der Klonierung oder Transformation eingeführt wurden (wobei die Kodierung für ein insektizides Protein erhalten bleibt), wie das Protein VIP3Aa im Baumwoll-Event COT 102; oder
9) ein sezerniertes Protein aus *Bacillus thuringiensis* oder *Bacillus cereus,* das in Gegenwart eines Kristallproteins aus *Bacillus thuringiensis* insektizid wirkt, wie das binäre Toxin, das aus VIP3 und Cry1A oder Cry1F besteht (US-Patentanmeldungen 61/126083 und 61/195019), oder das binäre Toxin, das aus dem Protein VIP3 und den Proteinen Cry2Aa oder Cry2Ab oder Cry2Ae besteht (US-Patentanmeldung 12/214,022 und EP-A 2 300 618); oder
10) ein Protein gemäß 9) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen, und/oder um das Spektrum der betroffenen Zielinsektenart zu erweitern, und/oder wegen Veränderungen, die in die Kodier-DNA während der Klonierung oder Transformation eingeführt wurden (wobei die Codierung für ein insektizides Protein erhalten bleibt).

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 10 kodieren. In einer Ausführungsform enthält eine insektenresistente Pflanze mehr als ein Transgen, das für ein Protein nach einer der oben genannten Klassen 1 bis 10 kodiert, um das Spektrum der betroffenen Zielinsektenarten zu erweitern, wenn verschiedene Proteine, die auf verschiedene Zielinsektenarten abzielen, verwendet werden, oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Eine "insektenresistente transgene Pflanze" beinhaltet im vorliegenden Zusammenhang weiterhin jegliche Pflanze, die mindestens ein Transgen enthält, welches eine Sequenz umfasst, die bei Expression eine doppelsträngige RNA produziert, welche bei Aufnahme durch ein Pflanzenschädlingsinsekt das Wachstum dieses Schädlingsinsekts hemmt, wie dies z.B. in WO 07/080126, WO 06/129204, WO 07/074405, WO 07/080127 und WO 07/035650 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
1) Pflanzen, die ein Transgen enthalten, das die Expression und/oder Aktivität des Gens für die Poly(ADP-ribose)polymerase (PARP) in den Pflanzenzellen oder Pflanzen zu reduzieren vermag, wie dies in WO 00/04173, WO 06/045633, EP-A 1 807 519 oder EP-A 2 018 431 beschrieben ist.
2) Pflanzen, die ein stresstoleranzförderndes Transgen enthalten, das die Expression und/oder Aktivität der für PARG kodierenden Gene der Pflanzen oder Pflanzenzellen zu reduzieren vermag, wie dies z.B. in WO 04/090140 beschrieben ist.
3) Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase, wie dies z. B. in der EP-A 1 794 306, WO 06/133827, WO 07/107326, EP-A 1 999 263 oder WO 07/107326 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie z.B.:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet. Diese transgenen Pflanzen, die eine modifizierte Stärke synthetisieren, sind z.B. in EP-A 0 571 427, WO 95/04826, EP-A 0 719 338, WO 96/15248, WO 96/19581, WO 96/27674, WO 97/11188, WO 97/26362, WO 97/32985, WO 97/42328, WO 97/44472, WO 97/45545, WO 98/27212, WO 98/40503, WO 99/58688, WO 99/58690, WO 99/58654, WO 00/08184, WO 00/08185, WO 00/08175, WO 00/28052, WO 00/77229, WO 01/12782, WO 01/12826, WO 02/101059, WO 03/071860, WO 04/056999, WO 05/030942, WO 05/030941, WO 05/095632, WO 05/095617, WO 05/095619, WO 2005/095618, WO 05/123927, WO 06/018319, WO 06/103107, WO 06/108702, WO 07/009823, WO 00/22140, WO 06/063862, WO 06/072603, WO 02/034923, WO 08/017518, WO 08/080630, WO 08/080631, WO 08/090008, WO 01/14569, WO 02/79410, WO 03/33540, WO 04/078983, WO 01/19975, WO 95/26407, WO 96/34968, WO 98/20145, WO 99/12950, WO 99/66050, WO 99/53072, US 6,734,341, WO 00/11192, WO 98/22604, WO 98/32326, WO 01/98509, WO 01/98509, WO 05/002359, US 5,824,790, US 6,013,861, WO 94/04693, WO 94/09144, WO 94/11520, WO 95/35026, WO 97/20936, WO 10/012796, WO 10/003701, WO13/053729, WO13/053730 offenbart.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind (wie z.B. WO 2015044209). Beispiele sind Pflanzen, die Polyfructose, insbesondere des Inulin- und Levantyps, produzieren, wie dies EP-A 0 663 956, WO 96/01904, WO 96/21023, WO 98/39460 und WO 99/24593 beschrieben ist, Pflanzen, die alpha-1,4-Glucane produzieren, wie dies in WO 95/31553, US 2002031826, US 6,284,479, US 5,712,107, WO 97/47806, WO 97/47807, WO 97/47808 und WO 00/14249 beschrieben ist, Pflanzen, die alpha-1,6-verzweigte alpha-1,4-Glucane produzieren, wie dies in WO 00/73422 beschrieben ist, und Pflanzen, die Alternan produzieren, wie dies in WO 00/47727, WO 00/73422, US 5,908,975 und EP-A 0 728 213 beschrieben ist.
3) Transgene Pflanzen, die Hyaluronan produzieren, wie dies z.B. in WO 06/032538, WO 07/039314, WO 07/039315, WO 07/039316, JP-A 2006-304779 und WO 05/012529 beschrieben ist.
4) Transgene Pflanzen oder Hybridpflanzen, wie Zwiebeln mit Merkmalen wie `hoher Gehalt an löslichen Feststoffen', 'milde' (low pungency, LP) und/oder 'Langzeitlagerung' (long storage, LS), wie dies in den US-Patentanmeldungen 12/020,360 beschrieben ist.
5) Transgene Pflanzen, die einen steigenden Ertrag anzeigen, wie z.B. offenbart in WO 11/095528, WO 2014161908, WO 2015032428 oder WO 2015117265.
6) Pflanzen, einschließlich transgener Pflanzen, die Früchte oder Gemüse mit verbesserten Eigenschaften bereitstellen, wie in WO 2013156204, WO 2013120781, WO 2014090968, WO 2014049002, WO 2014079896, WO 2014118150, WO 2015040098A1 beschrieben.

Pflanzen oder Pflanzensorten (die nach Methoden der pflanzlichen Biotechnologie, wie der Gentechnik, erhalten werden können), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten, wie dies in WO 98/00549 beschrieben ist;
b) Pflanzen wie Baumwollpflanzen, die eine veränderte Form von rsw2- oder rsw3-homologen Nukleinsäuren enthalten, wie dies in WO 04/053219 beschrieben ist;
c) Pflanzen wie Baumwollpflanzen mit einer erhöhten Expression der Saccharosephosphatsynthase, wie dies in WO 01/17333 beschrieben ist;
d) Pflanzen wie Baumwollpflanzen mit einer erhöhten Expression der Saccharosesynthase, wie dies in WO 02/45485 beschrieben ist;
e) Pflanzen wie Baumwollpflanzen, bei denen der Zeitpunkt der Durchlaßsteuerung der Plasmodesmen an der Basis der Faserzelle verändert ist, z. B. durch Herunterregulieren der faserselektiven β-1,3-Glucanase, wie dies in WO 05/017157 oder WO 09/143995 beschrieben ist;
f) Pflanzen wie Baumwollpflanzen mit Fasern mit veränderter Reaktivität, z.B. durch Expression des N-Acetylglucosamintransferasegens, darunter auch nodC, und von Chitinsynthasegenen, wie dies in WO 06/136351, WO 2011/089021, WO 2011/089021, WO 2012/074868 und WO 2015140191 beschrieben ist.

Pflanzen oder Pflanzensorten (die nach Methoden der pflanzlichen Biotechnologie, wie der Gentechnik, erhalten werden können), die ebenfalls erfindungsgemäß behandelt werden können, sind Pflanzen wie Raps oder verwandte *Brassica*-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produzieren, wie dies z.B. in US 5,969,169, US 5,840,946, US 6,323,392, US 6,063, 947 oder WO 2014006158, WO 2014006159, WO 2014006162 beschrieben ist;
b) Pflanzen wie Rapspflanzen, die Öl mit einem niedrigen Linolensäuregehalt produzieren, wie dies in US 6,270,828, US 6,169,190 oder US 5,965,755, WO 2011/060946 beschrieben ist;
c) Pflanzen wie Rapspflanzen, die Öl mit einem niedrigen gesättigten Fettsäuregehalt produzieren, wie dies z.B. in US 5,434,283 oder US-Patentanmeldung 12/668303 oder WO 2014006158, WO 2014006159 beschrieben ist.

Pflanzen oder Pflanzensorten (die nach Methoden der pflanzlichen Biotechnologie, wie der Gentechnik, erhalten werden können), die ebenfalls erfindungsgemäß behandelt werden können, sind Pflanzen wie Raps oder verwandte *Brassica*-Pflanzen mit veränderten Samenstreuungseigenschaften. Solche Pflanzen können durch genetische Transformation oder durch Selektion von Pflanzen, die eine Mutation enthalten, die solche veränderten Samenstreuungseigenschaften verleihen, erhalten werden; dazu zählen Rapspflanzen mit verzögerter oder verringerter Samenstreuung, wie dies in der US-Patentanmeldung 61/135,230, WO 09/068313 und WO 10/006732 beschrieben ist.

Pflanzen oder Pflanzensorten (die nach Methoden der pflanzlichen Biotechnologie, wie der Gentechnik, erhalten werden können), die ebenfalls erfindungsgemäß behandelt werden können, sind Pflanzen wie Tabakpflanzen mit veränderten posttranslationalen Proteinmodifikationsmustern, wie dies z.B. in WO 10/121818 und WO 10/145846 beschrieben ist.

Transgene Nutzpflanzen, die gemäß der Erfindung behandelt werden können, sind vorzugsweise Pflanzen, die Transformationsereignisse (transformation events) oder eine Kombination von Transformationsereignissen enthalten, und die zum Beispiel in den Datenbanken für diverse nationale oder regionale Registrierbehörden aufgelistet sind, einschließlich Event 531/PV-GHBK04 (Baumwolle, Insektenkontrolle, beschrieben in WO 2002/040677); Event 1143-14A (Baumwolle, Insektenkontrolle, nicht hinterlegt, beschrieben in WO 06/128569); Event 1143-51B (Baumwolle, Insektenkontrolle, nicht hinterlegt, beschrieben in WO 06/128570); Event 1445 (Baumwolle, Herbizidtoleranz, nicht hinterlegt, beschrieben in US-A 2002-120964 oder WO 02/034946); Event 17053 (Reis, Herbizidtoleranz, hinterlegt als PTA-9843, beschrieben in WO 10/117737); Event 17314 (Reis, Herbizidtoleranz, hinterlegt als PTA-9844, beschrieben in WO 10/117735); Event 281-24-236 (Baumwolle, Insektenkontrolle - Herbizidtoleranz, hinterlegt als PTA-6233, beschrieben in WO 05/103266 oder US-A 2005-216969); Event 3006-210-23 (Baumwolle, Insektenkontrolle - Herbizidtoleranz, hinterlegt als PTA-6233, beschrieben in US-A 2007-143876 oder WO 05/103266); Event 3272 [Mais, Qualitätsmerkmal (quality trait), hinterlegt als PTA-9972, beschrieben in WO 06098952 oder US-A 2006-230473]; Event 40416 (Mais, Insektenkontrolle - Herbizidtoleranz, hinterlegt als ATCC PTA-11508, beschrieben in WO 11/075593); Event 43A47 (Mais, Insektenkontrolle - Herbizidtoleranz, hinterlegt als ATCC PTA-11509, beschrieben in WO 11/075595); Event 5307 (Mais, Insektenkontrolle, hinterlegt als ATCC PTA-9561, beschrieben in WO 10/077816); Event ASR-368 [Straussgras (bent grass), Herbizidtoleranz, hinterlegt als ATCC PTA-4816, beschrieben in US-A 2006-162007 oder WO 04/053062]; Event B16 (Mais, Herbizidtoleranz, nicht hinterlegt, beschrieben in US-A 2003-126634); Event BPS-CV127-9 (Sojabohne, Herbizidtoleranz, hinterlegt als NCIMB Nr. 41603, beschrieben in WO 10/080829); Event BLR1 (Rapspflanzen, Rekultivierung der männlichen Sterilität, hinterlegt als NCIMB 41193, beschrieben in WO 2005/074671), Event CE43-67B (Baumwolle, Insektenkontrolle, hinterlegt als DSM ACC2724, beschrieben in US-A 2009-217423 oder WO 06/128573); Event CE44-69D (Baumwolle, Insektenkontrolle, nicht hinterlegt, beschrieben in US-A 2010-0024077); Event CE44-69D (Baumwolle, Insektenkontrolle, nicht hinterlegt, beschrieben in WO 06/128571); Event CE46-02A (Baumwolle, Insektenkontrolle, nicht hinterlegt, beschrieben in WO 06/128572); Event COT102 (Baumwolle, Insektenkontrolle, nicht hinterlegt, beschrieben in US-A 2006-130175 oder WO 04/039986); Event COT202 (Baumwolle, Insektenkontrolle, nicht hinterlegt, beschrieben in US-A 2007-067868 oder WO 05/054479); Event COT203 (Baumwolle, Insektenkontrolle, nicht hinterlegt, beschrieben in WO 05/054480); Event DAS21606-3/1606 (Sojabohne, Herbizidtoleranz, hinterlegt als PTA-11028, beschrieben in WO 012/033794), Event DAS40278 (Mais, Herbizidtoleranz, hinterlegt als ATCC PTA-10244, beschrieben in WO 11/022469); Event DAS-44406-6/pDAB8264.44.06.1 (Sojabohne, Herbizidtoleranz, hinterlegt als PTA-11336, beschrieben in WO 2012/075426), Event DAS-14536-7/pDAB8291.45.36.2 (Sojabohne, Herbizidtoleranz, hinterlegt als PTA-11335, beschrieben in WO 2012/075429), Event DAS-59122-7 (Mais, Insektenkontrolle - Herbizidtoleranz, hinterlegt als ATCC PTA 11384 , beschrieben in US-A 2006-070139); Event DAS-59132 (Mais, Insektenkontrolle - Herbizidtoleranz, nicht hinterlegt, beschrieben in WO 09/100188); Event DAS68416 (Sojabohne, Herbizidtoleranz, hinterlegt als ATCC PTA-10442, beschrieben in WO 11/066384 oder WO 11/066360); Event DP-098140-6 (Mais, Herbizidtoleranz, hinterlegt als ATCC PTA-8296, beschrieben in US-A 2009-137395 oder WO 08/112019); Event DP-305423-1 (Sojabohne, Qualitätsmerkmal, nicht hinterlegt, beschrieben in US-A 2008-312082 oder WO 08/054747); Event DP-32138-1 (Mais, Hybridsystem, hinterlegt als ATCC PTA-9158, beschrieben in US-A 2009-0210970 oder WO 09/103049); Event DP-356043-5 (Sojabohne, Herbizidtoleranz, hinterlegt als ATCC PTA-8287, beschrieben in US-A 2010-0184079 oder WO 08/002872); Event EE-1 (Aubergine, Insektenkontrolle, nicht hinterlegt, beschrieben in WO 07/091277); Event FI117 (Mais, Herbizidtoleranz, hinterlegt als ATCC 209031, beschrieben in US-A 2006-059581 oder WO 98/044140); Event FG72 (Sojabohne, Herbizidtoleranz, hinterlegt als PTA-11041, beschrieben in WO 2011/063413), Event GA21 (Mais, Herbizidtoleranz, hinterlegt als ATCC 209033, beschrieben in US-A 2005-086719 oder WO 98/044140); Event GG25 (Mais, Herbizidtoleranz, hinterlegt als ATCC 209032, beschrieben in US-A 2005-188434 oder WO 98/044140); Event GHB119 (Baumwolle, Insektenkontrolle - Herbizidtoleranz, hinterlegt als ATCC PTA-8398, beschrieben in WO 08/151780); Event GHB614 (Baumwolle, Herbizidtoleranz, hinterlegt als ATCC PTA-6878, beschrieben in US-A 2010-050282 oder WO 07/017186); Event GJ11 (Mais, Herbizidtoleranz, hinterlegt als ATCC 209030, beschrieben in US-A 2005-188434 oder WO 98/044140); Event GM RZ13 (Zuckerrübe, Virusresistenz, hinterlegt als NCIMB-41601, beschrieben in WO 10/076212); Event H7-1 (Zuckerrübe, Herbizidtoleranz, hinterlegt als NCIMB 41158 oder NCIMB 41159, beschrieben in US-A 2004-172669 oder WO 04/074492); Event JOPLIN1 (Weizen, Pilzresistenz, nicht hinterlegt, beschrieben in US-A 2008-064032); Event LL27 (Sojabohne, Herbizidtoleranz, hinterlegt als NCIMB41658, beschrieben in WO 06/108674 oder US-A 2008-320616); Event LL55 (Sojabohne, Herbizidtoleranz, hinterlegt als NCIMB 41660, beschrieben in WO 06/108675 oder US-A 2008-196127); Event LLcotton25 (Baumwolle, Herbizidtoleranz, hinterlegt als ATCC PTA-3343, beschrieben in WO 03/013224 oder US-A 2003-097687); Event LLRICE06 (Reis, Herbizidtoleranz, hinterlegt als ATCC-23352, beschrieben in US 6,468,747oder WO 00/026345); Event LLRice62 (Reis, Herbizidtoleranz, hinterlegt als ATCC-203352, beschrieben in WO 00/026345); Event LLRICE601 (Reis, Herbizidtoleranz, hinterlegt als ATCC PTA-2600, beschrieben in US-A 2008-2289060 oder WO 00/026356); Event LY038 (Mais, Qualitätsmerkmal, hinterlegt als ATCC PTA-5623, beschrieben in US-A 2007-028322 oder WO 05/061720); Event MIR162 (Mais, Insektenkontrolle, hinterlegt als PTA-8166, beschrieben in US-A 2009-300784 oder WO 07/142840); Event MIR604 (Mais, Insektenkontrolle, nicht hinterlegt, beschrieben in US-A 2008-167456 oder WO 05/103301); Event MON15985 (Baumwolle, Insektenkontrolle, hinterlegt als ATCC PTA-2516, beschrieben in US-A 2004-250317 oder WO 02/100163); Event MON810 (Mais, Insektenkontrolle, nicht hinterlegt, beschrieben in US-A 2002-102582); Event MON863 (Mais, Insektenkontrolle, hinterlegt als ATCC PTA-2605, beschrieben in WO 04/011601 oder US-A 2006-095986); Event MON87427 (Mais, Bestäubungskontrolle, hinterlegt als ATCC PTA-7899, beschrieben in WO 11/062904); Event MON87460 (Mais, Stresstoleranz, hinterlegt als ATCC PTA-8910, beschrieben in WO 09/111263 oder US-A 2011-0138504); Event MON87701 (Sojabohne, Insektenkontrolle, hinterlegt als ATCC PTA-8194, beschrieben in US-A 2009-130071 oder WO 09/064652); Event MON87705 (Sojabohne, Qualitätsmerkmal - Herbizidtoleranz, hinterlegt als ATCC PTA-9241, beschrieben in US-A 2010-0080887 oder WO 10/037016); Event MON87708 (Sojabohne, Herbizidtoleranz, hinterlegt als ATCC PTA-9670, beschrieben in WO 11/034704); Event MON87712 (Sojabohne, Ertrag, hinterlegt als ATCC PTA-10296, beschrieben in WO 12/051199); Event MON87754 (Sojabohne, Qualitätsmerkmal, hinterlegt als ATCC PTA-9385, beschrieben in WO 10/024976); Event MON87769 (Sojabohne, Qualitätsmerkmal, hinterlegt als ATCC PTA-8911, beschrieben in US-A 2011-0067141 oder WO 09/102873); Event MON88017 (Mais, Insektenkontrolle - Herbizidtoleranz, hinterlegt als ATCC PTA-5582, beschrieben in US-A 2008-028482 oder WO 05/059103); Event MON88913 (Baumwolle, Herbizidtoleranz, hinterlegt als ATCC PTA-4854, beschrieben in WO 04/072235 oder US-A 2006-059590); Event MON88302 (Rapspflanze, Herbizidtoleranz, hinterlegt als PTA-10955, beschrieben in WO 2011/153186); Event MON88701 (Baumwolle, Herbizidtoleranz, hinterlegt als PTA-11754, beschrieben in WO 2012/134808); Event MON89034 (Mais, Insektenkontrolle, hinterlegt als ATCC PTA-7455, beschrieben in WO 07/140256 oder US-A 2008-260932); Event MON89788 (Sojabohne, Herbizidtoleranz, hinterlegt als ATCC PTA-6708, beschrieben in US-A 2006-282915 oder WO 06/130436); Event MS11 (Raps, Bestäubungskontrolle - Herbizidtoleranz, hinterlegt als ATCC PTA-850 oder PTA-2485, beschrieben in WO 01/031042); Event MS8 (Raps, Bestäubungskontrolle - Herbizidtoleranz, hinterlegt als ATCC PTA-730, beschrieben in WO 01/041558 oder US-A 2003-188347); Event NK603 (Mais, Herbizidtoleranz, hinterlegt als ATCC PTA-2478, beschrieben in US-A 2007-292854); Event PE-7 (Reis, Insektenkontrolle, nicht hinterlegt, beschrieben in WO 08/114282); Event RF3 (Raps, Bestäubungskontrolle - Herbizidtoleranz, hinterlegt als ATCC PTA-730, beschrieben in WO 01/041558 oder US-A 2003-188347); Event RT73 (Raps, Herbizidtoleranz, nicht hinterlegt, beschrieben in WO 02/036831 oder US-A 2008-070260); Event SYHT0H2/SYN-00H2-5 (Sojabohne, Herbizidtoleranz, hinterlegt als PTA-11226, beschrieben in WO 2012/082548); Event T227-1 (Zuckerrübe, Herbizidtoleranz, nicht hinterlegt, beschrieben in WO 02/44407 oder US-A 2009-265817); Event T25 (Mais, Herbizidtoleranz, nicht hinterlegt, beschrieben in US-A 2001-29014 oder WO 01/051654); Event T304-40 (Baumwolle, Insektenkontrolle - Herbizidtoleranz, hinterlegt als ATCC PTA-8171, beschrieben in US-A 2010-077501 oder WO 08/122406); Event T342-142 (Baumwolle, Insektenkontrolle, nicht hinterlegt, beschrieben in WO 06/128568); Event TC1507 (Mais, Insektenkontrolle - Herbizidtoleranz, nicht hinterlegt, beschrieben in US-A 2005-039226 oder WO 04/099447); Event VIP1034 (Mais, Insektenkontrolle - Herbizidtoleranz, hinterlegt als ATCC PTA-3925, beschrieben in WO 03/052073); Event 32316 (Mais, Insektenkontrolle - Herbizidtoleranz, hinterlegt als PTA-11507, beschrieben in WO 11/084632); Event 4114 (Mais, Insektenkontrolle - Herbizidtoleranz, hinterlegt als PTA-11506, beschrieben in WO 11/084621); Event EE-GM3 / FG72 (Sojabohne, Herbizidtoleranz, ATCC Zugriffsnummer PTA-11041, WO 2011/063413A2); Event DAS-68416-4 (Sojabohne, Herbizidtoleranz, ATCC Zugriffsnummer PTA-10442, WO2 011/066360A1); Event DAS-68416-4 (Sojabohne, Herbizidtoleranz, ATCC Zugriffsnummer PTA-10442, WO 2011/066384A1); Event DP-040416-8 (Mais, Insektenkontrolle, ATCC Zugriffsnummer PTA-11508, WO 2011/075593A1); Event DP-043A47-3 (Mais, Insektenkontrolle, ATCC Zugriffsnummer PTA-11509, WO 2011/075595A1), Event DP-004114-3 (Mais, Insektenkontrolle, ATCC Zugriffsnummer PTA-11506, WO 2011/084621A1); Event DP-032316-8 (Mais, Insektenkontrolle, ATCC Zugriffsnummer PTA-11507, WO 2011/084632A1); Event MON-88302-9 (Rapspflanze, Herbizidtoleranz, ATCC Zugriffsnummer PTA-10955, WO 2011/153186A1); Event DAS-21606-3 (Sojabohne, Herbizidtoleranz, ATCC Zugriffsnummer PTA-11028, WO 2012/033794A2); Event MON-87712-4 (Sojapflanze, Qualitätsmerkmal, ATCC Zugriffsnummer PTA-10296, WO 2012/051199A2); Event DAS-44406-6 (Sojabohne, stacked Herbizidtoleranz, ATCC Zugriffsnummer PTA-11336, WO 2012/075426A1); Event DAS-14536-7 (Sojabohne, stacked Herbizidtoleranz, ATCC Zugriffsnummer PTA-11335, WO 2012/075429A1); Event SYN-000H2-5 (Sojabohne, stacked Herbizidtoleranz, ATCC Zugriffsnummer PTA-11226, WO 2012/082548A2); Event DP-061061-7 (Rapspflanze, Herbizidtoleranz, nicht hinterlegt, WO 2012071039A1); Event DP-073496-4 (Rapspflanze, Herbizidtoleranz, nicht hinterlegt, US2012131692); Event 8264.44.06.1 (Sojabohne, stacked Herbizidtoleranz, ATCC Zugriffsnummer PTA-11336, WO 2012075426A2); Event 8291.45.36.2 (Sojabohne, stacked Herbizidtoleranz, ATCC Zugriffsnummer PTA-11335, WO 2012075429A2); Event SYHT0H2 (Sojabohne, ATCC Zugriffsnummer PTA-11226, WO 2012/082548A2); Event MON88701 (Baumwolle, ATCC Zugriffsnummer PTA-11754, WO 2012/134808A1); Event KK179-2 (Alfalfa, ATCC Zugriffsnummer PTA-11833, WO2013003558A1); Event pDAB8264.42.32.1 (Sojabohne, stacked Herbizidtoleranz, ATCC Zugriffsnummer PTA-11993, WO 2013010094A1); Event MZDT09Y (Mais, ATCC Zugriffsnummer PTA-13025, WO 2013012775A1); Event KK179-2 (Alfalfa, ATCC Zugriffsnummer PTA-11833, WO2013003558A1); Event pDAB8264.42.32.1 (Sojabohne, stacked Herbizidtoleranz, ATCC Zugriffsnummer PTA-1 1993, WO2013010094A1); Event MZDT09Y (Mais, ATCC Zugriffsnummer PTA- 13025, WO2013012775A1); Event VCO-01981-5 (Mais, Herbizidtoleranz, NCIMB Zugriffsnummer 41842, WO2013014241A1); Event DAS-81419-2 X DAS-68416-4 (Sojabohne, stacked Insektenresistenz und Herbizidtoleranz, ATCC Zugriffsnummer PTA-10442, WO2013016516A1); Event DAS-81419-2 (Sojabohne, stacked Insektenresistenz und Herbizidtoleranz, ATCC Zugriffsnummer PTA-12006, WO2013016527A1); Event HCEM485 (Mais, Herbizidtoleranz, ATCC Zugriffsnummer PTA-12014, WO2013025400A1); Event pDAB4468.18.07.1 (Baumwolle, Herbizidtoleranz, ATCC Zugriffsnummer PTA-12456, WO2013112525A2); Event pDAB4468.19.10.3 (Baumwolle, Herbizidtoleranz, ATCC Zugriffsnummer PTA-12457, WO2013112527A1).

Die nachstehenden Beispiele erläutern die Erfindung näher.

### Chemische Beispiele

### Beispiel 2-21: Synthese von 7-(Trifluormethyl)-1,3-benzodithiolan-N-(1-methyltetrazol-5-yl)-4-carbonsäureamid

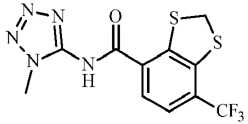

Zu 350 mg (1.3 mmol) 7-(Trifluormethyl)-1,3-benzodithiolan-4-carbonsäure, 0.52 ml (6.5 mmol) N-Methylimidazol und 195 mg (1.95 mmol) 5-Amino-1-methyltetrazol in 5 ml Acetonitril werden 0.12 ml (1.7 mmol) Thionylchlorid zugetropft. Nach 2 Tagen (d) Rühren bei Raumtemperatur (RT) wird mit 5 ml Wasser und 3 ml 2N Salzsäure versetzt. Die erhaltenen Kristalle werden abgesaugt, mit Wasser gewaschen und getrocknet. Ausbeute 175 mg.

Intermediat 2-21A: Synthese von 2-Fluor-3-methylthio-4-(trifluormethyl)-benzoesäuremethylester Zu 7.62 g (30 mmol) 2-Fluor-3-methylthio-4-(trifluormethyl)-benzoesäure (Synthese gemäß US2011/45980) und 4.9 g (35 mmol) Kaliumcarbonat in 30 ml DMF werden bei 0°C 3.2 ml (33 mmol) Dimethylsulfat zugegeben und 3 h bei RT gerührt. Anschließend wird auf Wasser gegossen, mit Esssigester extrahiert und die organische Phase mit 2N Salzsäure, Wasser, ges. Natriumbicarbonatlösung und ges. Kochsalzlösung gewaschen. Die organische Phase wird getrocknet, eingeengt und das erhaltene Rohprodukt ohne Reinigung weiter umgesetzt. (NMR, CDCl₃: 7.94 (t, 1H), 7.55 (dd, 1H), 3.97 (s, 3H), 2.48 (s, 3H))

Intermediat 2-21B: Synthese von 2-Fluor-3-methylsulfinyl-4-(trifluormethyl)-benzoesäuremethylester Zu 8.02 g (30mmol) 2-Fluor-3-methylthio-4-(trifluormethyl)-benzoesäuremethylester in 200 ml Dichlormethan werden bei 0°C 6.90 g (30 mmol) m-Chlorperbenzoesäure (ca. 77%ig) gegeben. Nach 30 min wird mit Natriumbisulfitlösung gequencht. Die organische Phase wird mit ges. Natriumbicarbonatlösung gewaschen, getrocknet und eingeengt. Das erhaltene Rohprodukt wird ohne Reinigung weiter umgesetzt. (NMR, CDCl₃: 8.18 (t, 1H), 7.63 (dd, 1H), 4.00 (s, 3H), 3.13 (s, 3H))

### Intermediat 2-21C: Synthese von 2-Fluor-3-chlormethylsulfanyl-4-(trifluormethyl)-benzoesäuremethylester

597 mg (2.1 mmol) 2-Fluor-3-methylsulfinyl-4-(trifluormethyl)-benzoesäuremethylester in 6 ml Thionychlorid werden 3 h unter Rückfluss erhitzt. Anschließend wird die Lösung eingeengt und das erhaltene Rohprodukt ohne Reinigung weiter umgesetzt. (NMR, CDCl₃: 8.08 (t, 1H), 7.64 (dd, 1H), 4.93 (s, 2H), 3.99 (s, 3H))

### Intermediat 2-21D: Synthese von 7-(Trifluormethyl)-1,3-benzodithiolan-4-carbonsäuremethylester

Zu erhaltenem Indermediat 2-21C (ca. 2.1 mmol) 2-Fluor-3-chlormethylsulfanyl-4-(trifluormethyl)-benzoesäuremethylester in 5 ml Dimethylformamid werden bei 0°C 195 mg (2.5 mmol) Natriumsulfid (wasserfrei) gegeben und die Mischung anschließend 5 h bei 80°C gerührt. Anschließend wird auf 2N Salzsäure gegossen, mit Esssigester extrahiert und die organische Phase mit Wasser, ges. Natriumbicarbonatlösung und ges. Kochsalzlösung gewaschen. Die organische Phase wird getrocknet, eingeengt und das erhaltene Rohprodukt säulenchromatographisch (Hepten/Essigester) gereinigt. Ausbeute 385 mg (65% d.Th.). (NMR, CDCl₃: 7.78 (dd, 1H), 7.35 (d, 1H), 4.50 (s, 2H) 3.96 (s, 3H))

### Intermediat 2-21E: Synthese von 7-(Trifluormethyl)-1,3-benzodithiolan-4-carbonsäure

Eine Mischung aus 385 mg (1.3 mmol) 7-(Trifluormethyl)-1,3-benzodithiolan-4-carbonsäuremethylester in 3ml Methanol und 3 ml 2N Natronlauge wird 2 h bei 50°C gerührt. Anschließend wird mit 2N Salzsäure sauer gestellt, mit Dichlormethan extrahiert und die organische Phase getrocknet, eingeengt und das erhaltene Rohprodukt ohne Reinigung weiter umgesetzt. (NMR, CDCl₃: 7.84 (dd, 1H), 7.38 (d, 1H), 4.52 (s, 2H))

### Beispiel 2-53: Synthese von 4-(Trifluormethyl)-1,3-benzoxathiolan-N-(1-methyltetrazol-5-yl)-7-carbonsäureamid

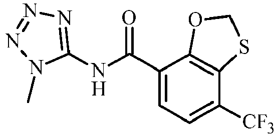

Zu 1.5 g (6 mmol) 4-(Trifluormethyl)-1,3-benzoxathiolan-7-carbonsäure und 788 mg (7.8 mmol) 5-Amino-1-methyltetrazol in eienm Gemisch von 7.5 ml Acetonitril und 5 ml Pyridin werden 0.76ml (8.7 mmol) Oxaylchlorid zugetropft. Nach 3 d bei RT wird mit Wasser und 2N Salzsäure versetzt und mit Essigester verdünnt. Die organische Phase wird getrocknet, eingeengt und der erhaltene Rückstand mit Isopropanol gewaschen. Die Kristalle werden in Methanol aufgelöst und mit Wasser wieder ausgefällt, mit Wasser gewaschen und getrocknet. Ausbeute 1.15 g.

### Intermediat 2-53A: Synthese von 2-Hydroxy-3-methylsulfinyl-4-(trifluormethyl)-benzoesäure-ethylester

Zu 6.45 g (22.5 mmol) 2-Hydroxy-3-methylthio-4-(trifluormethyl)-benzoesäureethylester in 15 ml Acetonitril werden 20 mg Schwefelsäure und 2.4 ml (27 mmol) Wasserstoffperoxid (35%ig) gegeben und anschließend 20 h auf 60°C erhitzt. Nach Zugabe von Natriumbisulfit-Lösung wird die Mischung eingeengt, der Rückstand abgesaugt und mit Wasser und wenig Essigester gewaschen. Die organische Phase wird mit ges. Kochsalzlösung gewaschen, getrocknet, eingeengt und das erhaltene Rohprodukt ohne Reinigung weiter umgesetzt. Ausbeute 6.64 g. (NMR, DMSO-D₆: 11.80 (brs, 1H), 8.05 (d, 1H), 7.45 (d, 1H), 4.39 (q, 2H), 3.15 (s, 3H), 1.34 (t, 3H))

### Intermediat 2-53B: Synthese von 2-Hydroxy-3-chlormethylsulfanyl-4-(trifluormethyl)-benzoesäureethylester

Zu 5.6 g (18.2 mmol) 2-Hydroxy-3-methylsulfinyl-4-(trifluormethyl)-benzoesäureethylester werden unter Wasserkühlung 6 ml Thionychlorid zugetropft (Gasentwicklung!) und anschließend noch 15 min nachgerührt. Die Mischung wird eingeengt und ohne Reinigung weiter umgesetzt. (NMR, DMSO-D₆: 11.68 (brs, 1H), 8.07 (dd, 1H), 7.45 (d, 1H), 5.32 (s, 2H), 4.45 (q, 2H), 1.38 (t, 3H))

### Intermediat 6-1: Synthese von 4-(Trifluormethyl)-1,3-benzoxathiolan-7-carbonsäure

5 g (18.2 mmol) 2-Hydroxy-3-chlormethylsulfanyl-4-(trifluormethyl)-benzoesäureethylester, gelöst in 5 ml Acetonitril, wird unter Eisbadkühlung zu 170 g 20%iger Natronlauge gegeben. Nach Stehen über Nacht wird mit konz. Salzsäure auf pH 1 angesäuert, der Niederschlag abgesaugt und mit Wasser gewaschen. Durch anschließendes auflösen in 5%iger Natronlauge, absaugen von ungelösten Nebenkomponenten und ausfällen durch ansäuern mit konz. Salzsäure wird nach trocknen 3.9 g Produkt erhalten.

### Beispiel 2-68: Synthese von 4-(Trifluormethyl)-1,3-benzoxathiolan-3-oxid-N-(1-methyltetrazol-5-yl)-7-carbonsäureamid

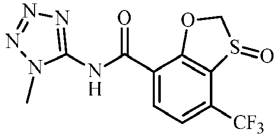

Zu 400 mg (1.2 mmol) 4-(Trifluormethyl)-1,3-benzoxathiolan-N-(1-methyltetrazol-5-yl)-7-carbonsäureamid in 10 ml Acetonitril werden 10 mg Schwefelsäure und 0.37 ml (3.6 mmol) Wasserstoffperoxid (30%ig) gegeben und anschließend 13 h auf 50°C erhitzt. Nach Zugabe von Natriumbisulfit-Lösung wird die Mischung eingeengt, der Rückstand abgesaugt und mit Wasser und wenig Essigester gewaschen. Ausbeute 190 mg.

### Beispiel 2-94: Synthese von 2-Methyl-4-(trifluormethyl)-1,3-benzoxathiolan-N-(1-methyltetrazol-5-yl)-7-carbonsäureamid

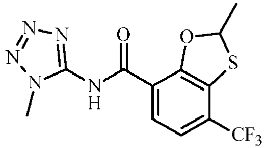

Zu 200 mg (0.6 mmol) 2-Methyl-4-(trifluormethyl)-1,3-benzoxathiolan-7-carbonsäure und 83 mg (0.8 mmol) 5-Amino-1-methyltetrazol in eienm Gemisch von 1 ml Acetonitril und 0.5 ml Pyridin werden 0.08 ml (0.88 mmol) Oxaylchlorid zugetropft. Nach Stehen über Nacht bei RT wird mit Wasser und Essigester verdünnt. Die organische Phase wird mit ges. Natriumbicarbonatlösung und ges. Kochsalzlösung gewaschen, getrocknet, eingeengt und das erhaltene Rohprodukt säulenchromatographisch (Hepten/Essigester) gereinigt. Ausbeute 50 mg.

### Intermediat 2-94A: Synthese von 2-Hydroxy-3-mercapto-4-(trifluormethyl)-benzoesäureethylester

5 g (15.9 mmol) 2-Hydroxy-3-chlormethylsulfanyl-4-(trifluormethyl)-benzoesäureethylester (Intermediat 2-53B) in 20 ml Ethanol und 20 ml konz. Salzsäure werden 8 h unter Rückfluss erhitzt. Anschließend wird das Ethanol abgezogen, mit Esssigester verdünnt, die organische Phase getrocknet und partiell eingeengt. Nach Zugabe von Heptan werden Kristalle erhalten. Ausbeute 3.97 g. (NMR, DMSO-D₆: 11.5 (brs), 7.73 (d, 1H), 7.31 (d, 1H), 4.43 (q, 2H), 1.37 (t, 3H))

### Intermediat 6-10: Synthese von 2-Methyl-4-(trifluormethyl)-1,3-benzoxathiolan-7-carbonsäure

Eine Mischung von 400 mg (1.05 mmol) 2-Hydroxy-3-mercapto-4-(trifluormethyl)-benzoesäureethylester, 0.75 ml (3.15 mmol) Tributylamin und 296 mg (1.58 mmol) 1,1-Dibromethan in 4 ml Acetonitril werden bei 65°C gerührt. Nachdem kein Edukt mehr detektierbar ist, wird die Mischung eingeengt und in 5 ml Ethanol gelöst. Nach Zugabe von 1 ml Natronlauge (50%ig) wird 1 h bei Raumtemperatur gerührt. Anschließend wird mit Wasser verdünnt, das Ethanol abgezogen und der Rückstand angesäuert. Der ausgefallene Feststoff wird abgesaugt und mit Wasser gewaschen und getrocknet. Ausbeute 235 mg.

### Beispiel 2-112: Synthese von 2-Methoxy-4-(trifluormethyl)-1,3-benzoxathiolan-N-(1-methyltetrazol-5-yl)-7-carbonsäureamid

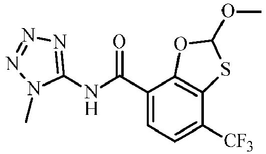

Zu 200 mg (0.56 mmol) 4-(Trifluormethyl)-1,3-benzoxathiolan-3-on-N-(1-methyltetrazol-5-yl)-7-carbonsäureamid in 2.5 ml Acetonitril werden 0.2 ml (2.8 mmol) Thionylchlorid zugegeben und die Mischung 1 h bei RT gerührt. Anschließend wird mit 5 ml Methaol verdünnt. Nach 10 min wird mit Wasser vedünnt und Essigester extrahiert und die organische Phase mit ges. Kochsazlösung gewaschen. Nach einengen und waschen des Rückstands mit wenig Essigester erhält man 105 mg farlosen Feststoff.

### Beispiel 2-238: Synthese von 7-(Trifluormethyl)-1,3-benzoxathiolan-N-(1-methyltetrazol-5-yl)-4-carbonsäureamid

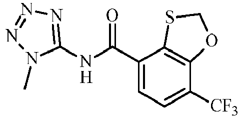

95 mg (0.27 mmol) 3-Hydroxy-2-methylsulfinyl-4-(trifluoromethyl)-N-(1-methyltetrazol-5-yl)-benzamid werden 30 min in 1 ml Thionylchlorid gerührt. Anschließend wird überschüssiges Thionylchlorid am Rotationsverdampfer entfernt, der Rückstand in 1 ml DMF aufgenommem und bei 0°C mit 60 mg (0.82 mmol) Natriummethanthiolat versetzt. Nach 1 h werden 0.04 ml (0.54 mmol) Dibrommethan zugegeben und 2 h bei RT und 1 h bei 80°C gerührt. Nach Abkühlen wird mit ges. Ammoniumchloridlösung versetzt und die Mischung mit Essigester extrahiert. Die organische Phase wird mit ges. Kochsalzlösung gewaschen, getrocknet, eingeengt und das erhaltene Rohprodukt säulenchromatographisch (Heptan/Esssigester) gereinigt. Ausbeute 14 mg.

### Intermediat 2-238A: Synthese von 3-Fluor-2-methylthio-4-(trifluormethyl)-N-(1-methyltetrazol-5-yl)-benzamid

Zu 381 mg (1.5 mmol) 3-Fluor-2-methylthio-4-(trifluormethyl)-benzoesäure (Synthese: EP2105437), 0.24 ml (3 mmol) N-Methylimidazol und 182 mg (1.8 mmol) 5-Amino-1-methyltetrazol in 5 ml Pyridin werden bei 0°C 0.15 ml (1.95 mmol) Thionylchlorid zugetropft. Nach 2 d bei RT wird mit Wasser und 2N Salzsäure versetzt. Die erhaltenen Kristalle werden abgesaugt, mit Wasser gewaschen und getrocknet. Ausbeute 430 mg. (NMR, DMSO-D₆: 11.86 (brs, 1H), 7.94 (dd, 1H), 7.71 (d, 1H), 4.04 (s, 3H), 3.14 (s, 3H))

### Intermediat 2-238B: Synthese von 3-Fluor-2-methylsulfinyl-4-(trifluormethyl)-N-(1-methyltetrazol-5-yl)-benzamid

Eine Mischung von 1.01 g (3 mmol) 3-Fluor-2-methylthio-4-(trifluormethyl)-N-(1-methyltetrazol-5-yl)-benzamid und 0.17 ml Wasserstoffperoxid (50%ig) in 10 ml Essigsäure wird 13 h bei 40°C gerührt. Anschließend wird mit 140 ml Wasser verdünnt und die erhaltenen Kristalle abgesaugt und getrocknet. Ausbeute 645 mg. (NMR, DMSO-D₆: 12.02 (brs, 1H), 8.18 (dd, 1H), 7.87 (d, 1H), 4.04 (s, 3H), 2.52 (s, 3H))

### Intermediat 2-238C: Synthese von 3-(4-Methoxybenzyloxy)-2-methylsulfinyl-4-(trifluormethyl)-N-(1-methyltetrazol-5-yl)-benzamid

Zu einer Mischung von 600 mg (1.7 mmol) 3-Fluor-2-methylsulfinyl-4-(trifluormethyl)-N-(1-methyltetrazol-5-yl)-benzamid und 0.32 ml (2.6 mmol) Anisalkohol in 10 ml THF werden bei 0°C 430 mg (3.75 mmol) Kalium-t-Butanolat gegeben. Nach 16 h bei Raumtemperatut wir mit 2N Salzäure versetzt, die Mischung mit Dichlormethan extrahiert, die organische Phase getrocknet und eingeengt.

Das erhaltene Rohprodukt wird ohne Reinigung weiter umgesetzt. Ausbeute 970 mg. (NMR, CDCl₃: 11.42 (brs, 1H), 7.87 (d, 1H), 7.66 (d, 1H), 7.29 (m, 2H), 6.95 (m, 2H), 5.06 (d, 1H), 5.00 (d, 1H), 4.19 (s, 3H), 3.85 (s, 3H), 2.98 (s, 3H))

### Intermediat 2-238D: Synthese von 3-Hydroxy-2-methylsulfinyl-4-(trifluormethyl)-N-(1-methyltetrazol-5-yl)-benzamid

Zu einer Mischung von 475 mg (1.01 mmol) 3-(4-Methoxybenzyloxy)-2-methylsulfinyl-4-(trifluormethyl)-N-(1-methyltetrazol-5-yl)-benzamid und 0.33 ml (3.03 mmol) Anisol in 10 ml Dichlormethan werden 0.23 ml (3.03 mmol) Trifluoressigsäure gegeben und 6 d bei Raumtemperatur gerührt. Anschließend wird mit Wasser versetzt und mit Essigester verdünnt. Die organische Phase wird mit ges. Natriumbicarbonatlösung gewaschen, die erhaltene wässrige Phase mit 2N Salzsäure angesäuert und die erhaltenen Kristalle abgesaugt, mit Wasser gewaschen und getrocknet. Ausbeute 115 mg. (NMR, DMSO-D₆: 12.98 (brs, 1H), 12.07 (brs, 1H), 8.00 (d, 1H), 7.66 (d, 1H), 3.97 (s, 3H), 3.23 (s, 3H))

Die NMR-Daten offenbarter Beispiele werden entweder in klassischer Form (δ-Werte, Anzahl der H-Atome, Multiplettaufspaltung) oder als sogenannte NMR-Peak-Listen aufgeführt. Beim NMR-Peak-Listenverfahren werden die NMR-Daten ausgewählter Beispiele in Form von NMR-Peaklisten notiert, wobei zu jedem Signalpeak erst der δ-Wert in ppm und dann die Signalintensität durch ein Leerzeichen getrennt aufgeführt wird. Die δ-Wert-Signalintensitäts - Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren wird Tetramethylsilan benutzt und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu ¹H-NMR-Peaklisten können der Research Disclosure Database Number 564025 entnommen werden.

### Analytische Daten

| Nr. | NMR |
|---|---|
| 1-35 | ¹H-NMR(400.0 MHz, DMSO-d₆): δ = 10.73 (br s, 1H); 7.90 (s, 1H); 7.58 (d, 1H); 7.39 (d, 1H); 6.02 (s, 2H); 3.70 (s, 3H) |
| 1-50 | ¹H-NMR(400.0 MHz, DMSO-d₆): δ = 8.23 (d, 1H); 7.93 (s, 1H); 7.76 (d, 1H); 5.90 (d, 1H); 5.38 (d, 1H); 3.76 (s, 3H) |
| 1-65 | ¹H-NMR(400.0 MHz, DMSO-d₆): δ = 11.28 (br s, 1H); 8.25 (d, 1H); 7.92 (s, 1H); 7.82 (d, 1H); 5.63 (s, 2H); 3.75 (s, 3H) |
| 2-4 | ¹H-NMR(400.0 MHz, DMSO-d₆): δ = 7.46 (d, 1H); 7.28 (d, 1H); 6.37 (s, 2H); 3.94 (s, 3H) |
| 2-7 | ¹H-NMR(400.0 MHz, DMSO-d₆): δ = 11.68 (br s, 1H); 7.73 (m, 2H); 7.41 (t, 1H); 3.96 (s, 3H) |
| 2-13 | ¹H-NMR(400.0 MHz, DMSO-d₆): δ = 11.60 (br s, 1H); 7.76 (d, 1H); 7.33 (d, 1H); 3.95 (s, 3H); 2.67 (s, 3H) |
| 2-15 | ¹H-NMR(400.0 MHz, DMSO-d₆): δ = 11.91 (br s, 1H); 7.92 (d, 1H); 7.77 (d, 1H); 3.97 (s, 3H); 3.43 (s, 3H) |
| 2-19 | ¹H-NMR(400.0 MHz, DMSO-d₆): δ = 11.85 (br s, 1H); 7.79 (d, 1H); 7.40 (d, 1H); 4.64 (s, 2H); 3.92 (s, 3H) |
| 2-20 | ¹H-NMR(400.0 MHz, DMSO-d₆): δ = 11.56 (br s, 1H); 7.73 (d, 1H); 7.13 (d, 1H); 4.55 (s, 2H); 3.91 (s, 3H); 2.29 (s, 3H) |
| 2-21 | ¹H-NMR(400.0 MHz, CDCl₃): δ = 11.82 (br s, 1H); 8.05 (d, 1H); 7.55 (d, 1H); 4.49 (s, 2H); 4.09 (s, 3H) |
| 2-22 | ¹H-NMR(400.0 MHz, DMSO-d₆): δ = 11.82 (br s, 1H); 7.49 (d, 1H); 7.51 (d, 1H); 7.08 (t, 1H); 4.65 (s, 2H); 3.94 (s, 3H) |
| 2-41 | ¹H-NMR(400.0 MHz, DMSO-d₆): δ = 12.31 (br s, 1H); 8.40 (d, 1H); 8.34 (d, 1H); 6.00 (s, 2H); 4.00 (s, 3H) |
| 2-43 | ¹H-NMR(400.0 MHz, DMSO-d₆): δ = 12.45 (br s, 1H); 8.65 (d, 1H); 8.58 (d, 1H); 6.10 (s, 2H); 4.02 (s, 3H) |
| 2-50 | ¹H-NMR(400.0 MHz, DMSO-d₆): δ = 10.71 (br s, 1H); 7.43 (d, 1H); 6.94 (d, 1H); 5.95 (s, 2H); 3.91 (s, 3H); 2.27 (s, 3H) |
| 2-53 | ¹H-NMR(400.0 MHz, DMSO-d₆): δ = 11.20 (br s, 1H); 7.62 (d, 1H); 7.42 (d, 1H); 6.04 (s, 2H); 3.94 (s, 3H) |
| 2-54 | ¹H-NMR(400.0 MHz, DMSO-d₆): δ = 11.08 (br s, 1H); 7.56 (d, 1H); 7.26 (d, 1H); 7.11 (t, 1H); 5.99 (s, 2H); 3.94 (s, 3H) |
| 2-55 | ¹H-NMR(400.0 MHz, DMSO-d₆): δ = 11.25 (br s, 1H); 7.64 (d, 1H); 7.35 (d, 1H); 6.01 (s, 2H); 3.95 (s, 3H) |
| 2-58 | ¹H-NMR(400.0 MHz, DMSO-d₆): δ = 11.23 (br s, 1H); 7.64 (d, 1H); 7.53 (d, 1H); 5.98 (s, 2H); 3.94 (s, 3H); 3.27 (s, 3H) |
| 2-65 | ¹H-NMR(400.0 MHz, DMSO-d₆): δ = 11.07 (br s, 1H); 8.01 (d, 1H); 7.25 (d, 1H); 5.79 (d, 1H); 5.29 (d, 1H); 3.96 (s, 3H); 2.68 (s, 3H) |
| 2-68 | ¹H-NMR(400.0 MHz, DMSO-d₆): δ = 11.58 (br s, 1H); 8.26 (d, 1H); 7.78 (d, 1H); 5.91 (d, 1H); 5.39 (d, 1H); 3.99 (s, 3H) |
| 2-69 | ¹H-NMR(400.0 MHz, DMSO-d₆): δ = 11.44 (br s, 1H); 8.20 (d, 1H); 7.58 (d, 1H); 7.46 (t, 1H); 5.86 (d, 1H); 5.33 (d, 1H); 3.98 (s, 3H) |
| 2-70 | ¹H-NMR(400.0 MHz, DMSO-d₆): δ = 11.62 (br s, 1H); 8.29 (d, 1H); 7.73 (d, 1H); 5.91 (d, 1H); 5.38 (d, 1H); 3.99 (s, 3H) |
| 2-73 | ¹H-NMR(400.0 MHz, DMSO-d₆): δ = 11.60 (br s, 1H); 8.29 (d, 1H); 7.86 (d, 1H); 5.85 (d, 1H); 5.38 (d, 1H); 3.99 (s, 3H); 3.39 (s, 3H) |
| 2-80 | ¹H-NMR(400.0 MHz, DMSO-d₆): δ = 11.19 (br s, 1H); 8.01 (d, 1H); 7.26 (d, 1H); 5.47 (s, 2H); 3.94 (s, 3H); 2.58 (s, 3H) |
| 2-83 | ¹H-NMR(400.0 MHz, DMSO-d₆): δ = 11.60 (br s, 1H); 8.29 (d, 1H); 7.85 (d, 1H); 5.63 (s, 2H); 3.98 (s, 3H) |
| 2-84 | ¹H-NMR(400.0 MHz, DMSO-d₆): δ = 11.50 (br s, 1H); 8.22 (d, 1H); 7.64 (d, 1H); 7.37 (t, 1H); 5.56 (s, 2H); 3.97 (s, 3H) |
| 2-85 | ¹H-NMR(400.0 MHz, DMSO-d₆): δ = 11.63 (br s, 1H); 8.31 (d, 1H); 7.79 (d, 1H); 5.63 (s, 2H); 3.98 (s, 3H) |
| 2-88 | ¹H-NMR(400.0 MHz, DMSO-d₆): δ = 11.62 (br s, 1H); 8.32 (d, 1H); 7.86 (d, 1H); 5.60 (s, 2H); 3.97 (s, 3H); 3.42 (s, 3H) |
| 2-94 | ¹H-NMR(400.0 MHz, DMSO-d₆): δ = 11.16 (br s, 1H); 7.63 (d, 1H); 7.41 (d, 1H); 6.57 (q, 1H); 3.95 (s, 3H); 1.80 (d, 3H) |
| 2-106 | ¹H-NMR(400.0 MHz, DMSO-d₆): δ = 8.24 (br d, 1H); 7.69 (d, 1H); 5.62 (br s, 1H); 3.81 (br s, 3H); 1.65 (br s, 3H) |
| 2-112 | ¹H-NMR(400.0 MHz, DMSO-d₆): δ = 11.42 (br s, 1H); 7.72 (d, 1H); 7.55 (d, 1H); 7.53 (s, 1H); 3.97 (s, 3H); 3.41 (s, 3H) |
| 2-130 | ¹H-NMR(400.0 MHz, DMSO-d₆): δ = 11.40 (br.s, 1H); 7.71 (d, 1H); 7.54 (d, 1H); 7.54 (s, 1H); 3.97 (s, 3H); 3.80 (m, 1H); 3.59 (m, 1H); 1.17 (t, 3H) |
| 2-202 | ¹H-NMR(400.0 MHz, DMSO-d₆): δ = 11.86 (br s, 1H); 8.04 (d, 1H); 7.94 (d, 1H); 4.00 (s, 3H) |
| 2-238 | ¹H-NMR(400.0 MHz, CDCl₃): δ = 8.21 (dd, 1H); 7.65 (d, 1H); 5.23 (br s, 1H); 5.11 (br s, 1H); 3.97 (s, 3H) |
| 2-253 | ¹H-NMR(400.0 MHz, CDCl₃): δ = 11.02 (br.s, 1H); 7.88 (d, 1H); 7.05 (d, 1H); 4.47 (s, 2H); 4.07 (s, 3H); 2.60 (s, 3H) |
| 2-254 | ¹H-NMR(400.0 MHz, DMSO-d₆): δ = 11.92 (br s, 1H); 7.96 (d, 1H); 7.92 (d, 1H); 4.62 (d, 1H); 4.48 (d, 1H); 3.97 (s, 3H) |
| 2-255 | ¹H-NMR(400.0 MHz, DMSO-d₆): δ = 12.09 (br s, 1H); 8.48 (d, 1H); 7.99 (d, 1H); 4.72 (d, 1H); 4.44 (d, 1H); 3.98 (s, 3H) |
| 2-256 | ¹H-NMR(400.0 MHz, DMSO-d₆): δ = 8.46 (d, 1H); 7.80 (d, 1H); 4.68 (s, 2H); 3.76 (s, 3H) |
| 2-257 | ¹H-NMR(400.0 MHz, DMSO-d₆): δ = 11.97 (br s, 1H); 8.08 (d, 1H); 7.83 (d, 1H); 7.05 (s, 1H); 3.97 (s, 3H); 3.15 (s, 3H) |
| 3-7 | ¹H-NMR(400.0 MHz, DMSO-d₆): δ = 11.55 (br s, 1H); 7.72 (m, 2H); 7.41 (t, 1H); 4.32 (q, 2H); 1.46 (t, 3H) |
| 3-20 | ¹H-NMR(400.0 MHz, DMSO-d₆): δ = 11.78 (br s, 1H); 7.90 (d, 1H); 7.66 (d, 1H); 4.69 (s, 2H); 4.28 (q, 2H); 1.44 (t, 3H) |
| 3-52 | ¹H-NMR(400.0 MHz, DMSO-d₆): δ = 11.12 (br s, 1H); 7.61 (d, 1H); 7.42 (d, 1H); 6.03 (s, 2H); 4.30 (q, 2H); 1.44 (t, 3H) |
| 3-53 | ¹H-NMR(400.0 MHz, DMSO-d₆): δ = 11.00 (br s, 1H); 7.56 (d, 1H); 7.26 (d, 1H); 7.11 (t, 1H); 5.98 (s, 2H); 4.29 (q, 2H); 1.44 (t, 3H) |
| 3-67 | ¹H-NMR(400.0 MHz, DMSO-d₆): δ = 11.50 (br s, 1H); 8.28 (d, 1H); 7.79 (d, 1H); 5.93 (d, 1H); 5.41 (d, 1H); 4.36 (q, 2H); 1.48 (t, 3H) |
| 3-82 | ¹H-NMR(400.0 MHz, DMSO-d₆): δ = 11.50 (br s, 1H); 8.29 (d, 1H); 7.85 (d, 1H); 5.63 (s, 2H); 4.34 (q, 2H); 1.45 (t, 3H) |
| 4-16 | ¹H-NMR(400.0 MHz, DMSO-d₆): δ = 11.77 (br s, 1H); 7.88 (d, 1H); 7.65 (d, 1H); 4.70 (s, 2H); 4.24 (t, 2H); 2.86 (tq, 2H); 0.86 (t, 3H) |
| 5-64 | ¹H-NMR(400.0 MHz, DMSO-d₆): δ = 7.67 (d, 1H); 7.45 (d, 1H); 5.96 (d, 1H); 3.84 (s, 3H), 3.24 (s, 3H) |
| 6-1 | ¹H-NMR(400.0 MHz, DMSO-d₆): δ = 13.37 (br s, 1H); 7.63 (d, 1H); 7.31 (d, 1H); 5.99 (s, 2H) |
| 6-2 | ¹H-NMR(400.0 MHz, DMSO-d₆): δ = 13.18 (br s, 1H); 7.58 (d, 1H); 7.16 (d, 1H); 7.06 (t, 1H); 5.93 (s, 2H) |
| 6-3 | ¹H-NMR(400.0 MHz, DMSO-d₆): δ = 13.43 (br s, 1H); 7.65 (d, 1H); 7.23 (d, 1H); 5.96 (s, 2H) |
| 6-4 | ¹H-NMR(400.0 MHz, DMSO-d₆): δ = 13.78 (br s, 1H); 8.28 (d, 1H); 7.68 (d, 1H); 5.87 (d, 1H); 5.36 (d, 1H) |
| 6-5 | ¹H-NMR(400.0 MHz, DMSO-d6): δ = 13.56 (br s, 1H); 8.23 (d, 1H); 7.50 (d, 1H); 7.43 (t, 1H); 5.84 (d, 1H); 5.30 (d, 1H) |
| 6-7 | ¹H-NMR(400.0 MHz, DMSO-d₆): δ = 13.92 (br s, 1H); 8.30 (d, 1H); 7.74 (d, 1H); 5.63 (s, 2H) |
| 6-8 | ¹H-NMR(400.0 MHz, DMSO-d₆): δ = 8.23 (d, 1H); 7.54 (d, 1H); 7.33 (t, 1H); 5.55 (s, 2H) |
| 6-10 | ¹H-NMR(400.0 MHz, DMSO-d₆): δ = 13.31 (br s, 1H); 7.63(d, 1H); 7.30 (d, 1H); 6.53 (q, 1H); 1.75 (d, 3H) |
| 6-64 | ¹H-NMR(400.0 MHz, DMSO-d₆): δ = 7.65 (d, 1H); 7.43 (d, 1H); 5.94 (d, 1H); 3.23 (s, 3H) |

### B. Formulierungsbeispiele

### 1. Stäubemittel

Ein Stäubemittel wird erhalten, indem man 10 Gew.-Teile einer Verbindung der Formel (I) und 90% Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der Formel (I), 6 Gew.-Teile Alkylphenolpolyglykolether (^{®}Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man 75 Gew.-Teile einer Verbindung der Formel (I),

| | | |
|---|---|---|
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man 25 Gew.-Teile einer Verbindung der Formel (I),

| | | |
|---|---|---|
| 5 | " | 2,2'-dinaphthylmethan-6,6'-disulfonsaures Natrium, |
| 2 | " | oleoylmethyltaurinsaures Natrium, |
| 1 | " | Polyvinylalkohol, |
| 17 | " | Calciumcarbonat und |
| 50 | " | Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### Versuchsbeschreibung

In den nachfolgenden Tabellen werden folgende Abkürzungen verwendet:

### Unerwünschte Pflanzen / Weeds:

| | | | |
|---|---|---|---|
| ABUTH: | *Abutilon* theo*p*hrasti | ALOMY | *Alopecurus myosuroides* |
| AMARE: | *Amaranthus retroflexus* | AVEFA: | *Avena fatua* |
| CYPES: | *Cyperus esculentus* | ECHCG: | *Echinochloa crus-galli* |
| LOLMU: | *Lolium multiflorum* | MATIN: | *Matricaria inodora* |
| PHBPU: | *Ipomoea purpurea* | POLCO: | *Polygonum convolvulus* |
| SETVI: | *Setaria viridis* | STEME: | *Stellaria media* |
| VERPE: | *Veronica persica* | VIOTR: | *Viola tricolor* |

### 1. Herbizide Wirkung und Kulturverträglichkeit im Vorauflauf

Samen von mono- bzw. dikotylen Unkraut und Kulturpflanzen werden in Kunststoff- oder organischen Pflanztöpfen ausgelegt und mit Erde abgedeckt. Die in Form von benetzbaren Pulvern (WP) oder als Emulsionskonzentrate (EC) formulierten erfindungsgemäßen Verbindungen werden dann als wässrige Suspension bzw. Emulsion unter Zusatz von 0,5% Additiv mit einer Wasseraufwandmenge von umgerechnet 600 l/ha auf die Oberfläche der Abdeckerde appliziert. Nach der Behandlung werden die Töpfe im Gewächshaus aufgestellt und unter guten Wachstumsbedingungen für die Testpflanzen gehalten. Nach ca. 3 Wochen wird die Wirkung der Präparate visuell im Vergleich zu unbehandelten Kontrollen in Prozentwerten bonitiert. In den nachfolgenden Tabellen ist die herbizide Wirkung zahlreicher erfindungsgemäßer Verbindungen gegen wichtige Schadpflanzen angegeben. Beispielsweise bedeutet 100% Wirkung = Pflanzen sind abgestorben, 0% Wirkung = wie Kontrollpflanzen.

| Beispiel Nummer | Dosierung [g/ha] | CYPES |
|---|---|---|
| 2-65 | 320 | 90% |
| 2-68 | 320 | 90% |
| 2-83 | 320 | 100% |
| 2-106 | 320 | 90% |
| 3-67 | 320 | 80% |
| 3-82 | 320 | 90% |

| Beispiel Nummer | Dosierung [g/ha] | ECHCG |
|---|---|---|
| 2-68 | 320 | 90% |
| 2-69 | 320 | 90% |
| 2-83 | 320 | 100% |
| 2-106 | 320 | 90% |
| 3-52 | 320 | 90% |
| 3-67 | 320 | 100% |
| 3-82 | 320 | 100% |

| Beispiel Nummer | Dosierung [g/ha] | SETVI |
|---|---|---|
| 2-68 | 320 | 100% |
| 2-69 | 320 | 90% |
| 2-83 | 320 | 100% |
| 2-84 | 320 | 90% |
| 2-94 | 320 | 100% |
| 2-106 | 320 | 90% |
| 3-52 | 320 | 90% |
| 3-67 | 320 | 80% |

| Beispiel Nummer | Dosierung [g/ha] | ABUTH |
|---|---|---|
| 2-68 | 320 | 90% |
| 2-69 | 320 | 100% |
| 2-83 | 320 | 100% |
| 2-84 | 320 | 90% |
| 2-94 | 320 | 90% |
| 2-106 | 320 | 100% |
| 3-52 | 320 | 90% |
| 3-67 | 320 | 90% |
| 3-82 | 320 | 90% |

| Beispiel Nummer | Dosierung [g/ha] | ABUTH |
|---|---|---|
| 2-43 | 80 | 100% |
| 2-112 | 80 | 100% |
| 2-130 | 80 | 100% |

| Beispiel Nummer | Dosierung [g/ha] | AMARE |
|---|---|---|
| 2-54 | 320 | 80% |
| 2-65 | 320 | 90% |
| 2-68 | 320 | 100% |
| 2-69 | 320 | 100% |
| 2-80 | 320 | 90% |
| 2-83 | 320 | 100% |
| 2-84 | 320 | 100% |
| 2-94 | 320 | 100% |
| 2-106 | 320 | 100% |
| 3-52 | 320 | 100% |
| 3-53 | 320 | 90% |
| 3-67 | 320 | 100% |
| 3-82 | 320 | 100% |

| Beispiel Nummer | Dosierung [g/ha] | AMARE |
|---|---|---|
| 2-43 | 80 | 100% |
| 2-112 | 80 | 90% |
| 2-254 | 80 | 80% |

| Beispiel Nummer | Dosierung [g/ha] | MATIN |
|---|---|---|
| 2-68 | 320 | 90% |
| 2-69 | 320 | 90% |
| 2-80 | 320 | 80% |
| 2-83 | 320 | 90% |
| 2-84 | 320 | 100% |
| 2-106 | 320 | 100% |
| 3-52 | 320 | 90% |
| 3-67 | 320 | 90% |
| 3-82 | 320 | 100% |

| Beispiel Nummer | Dosierung [g/ha] | MATIN |
|---|---|---|
| 1-50 | 80 | 90% |
| 2-43 | 80 | 80% |

| Beispiel Nummer | Dosierung [g/ha] | STEME |
|---|---|---|
| 2-68 | 320 | 90% |
| 2-69 | 320 | 90% |
| 2-83 | 320 | 100% |
| 2-84 | 320 | 90% |
| 2-94 | 320 | 100% |
| 2-106 | 320 | 90% |
| 3-52 | 320 | 100% |
| 3-67 | 320 | 100% |
| 3-82 | 320 | 100% |

| Beispiel Nummer | Dosierung [g/ha] | STEME |
|---|---|---|
| 2-21 | 80 | 90% |
| 2-43 | 80 | 90% |
| 2-130 | 80 | 100% |

| Beispiel Nummer | Dosierung [g/ha] | VIOTR |
|---|---|---|
| 2-68 | 320 | 100% |
| 2-69 | 320 | 100% |
| 2-83 | 320 | 100% |
| 2-84 | 320 | 100% |
| 2-94 | 320 | 100% |
| 2-106 | 320 | 100% |
| 3-52 | 320 | 90% |
| 3-53 | 320 | 100% |
| 3-67 | 320 | 100% |
| 3-82 | 320 | 100% |

| Beispiel Nummer | Dosierung [g/ha] | VIOTR |
|---|---|---|
| 2-43 | 80 | 100% |
| 2-112 | 80 | 100% |
| 2-130 | 80 | 100% |

| Beispiel Nummer | Dosierung [g/ha] | VERPE |
|---|---|---|
| 2-13 | 320 | 90% |
| 2-15 | 320 | 90% |
| 2-65 | 320 | 80% |
| 2-68 | 320 | 100% |
| 2-69 | 320 | 90% |
| 2-83 | 320 | 100% |
| 2-84 | 320 | 100% |
| 3-52 | 320 | 80% |
| 3-67 | 320 | 100% |
| 3-82 | 320 | 100% |

| Beispiel Nummer | Dosierung [g/ha] | VERPE |
|---|---|---|
| 2-112 | 80 | 80% |

In einem Vergleichsversuch wurde exemplarisch die herbizide Wirkung der erfindungsgemäßen Verbindung Nr. 2-53 mit der der aus WO 2013/076315 A2 bekannten Verbindung Nr. A-117 verglichen. Dabei zeigte sich anhand zahlreicher Schadpflanzen eine deutliche Überlegenheit der erfindungsgemäßen Verbindung:

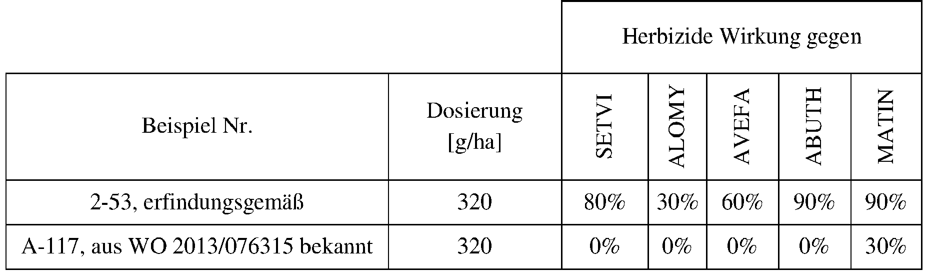

### 2. Herbizide Wirkung und Kulturverträglichkeit im Nachauflauf

Samen von mono- bzw. dikotylen Unkraut- bzw. Kulturpflanzen werden in Kunststoff- oder organischen Pflanztöpfen in sandigem Lehmboden ausgelegt, mit Erde abgedeckt und im Gewächshaus unter kontrollierten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion unter Zusatz von 0,5% Additiv mit einer WasserDosierung von umgerechnet 600 l/ha auf die grünen Pflanzenteile gesprüht. Nach ca. 3 Wochen Standzeit der Versuchspflanzen im Gewächshaus, unter optimalen Wachstumsbedingungen, wird die Wirkung der Präparate visuell im Vergleich zu unbehandelten Kontrollen bonitiert. In den nachfolgenden Tabellen ist die herbizide Wirkung zahlreicher erfindungsgemäßer Verbindungen gegen wichtige Schadpflanzen angegeben. Beispielsweise bedeutet 100% Wirkung = Pflanzen sind abgestorben, 0% Wirkung = wie Kontrollpflanzen.

| Beispiel Nummer | Dosierung [g/ha] | ECHCG |
|---|---|---|
| 2-54 | 80 | 100% |
| 2-68 | 80 | 100% |
| 2-69 | 80 | 100% |
| 2-83 | 80 | 90% |
| 2-84 | 80 | 90% |
| 2-94 | 80 | 80% |
| 2-106 | 80 | 100% |
| 3-52 | 80 | 100% |
| 3-53 | 80 | 80% |
| 3-67 | 80 | 100% |
| 3-82 | 80 | 80% |

| Beispiel Nummer | Dosierung [g/ha] | ECHCG |
|---|---|---|
| 2-112 | 20 | 80% |
| 2-257 | 20 | 100% |

| Beispiel Nummer | Dosierung [g/ha] | SETVI |
|---|---|---|
| 2-54 | 80 | 90% |
| 2-83 | 80 | 90% |
| 2-68 | 80 | 90% |
| 2-69 | 80 | 80% |
| 2-84 | 80 | 100% |
| 2-94 | 80 | 80% |
| 2-106 | 80 | 100% |
| 3-52 | 80 | 100% |
| 3-53 | 80 | 80% |

| Beispiel Nummer | Dosierung [g/ha] | SETVI |
|---|---|---|
| 2-112 | 20 | 80% |

| Beispiel Nummer | Dosierung [g/ha] | ABUTH |
|---|---|---|
| 2-13 | 80 | 80% |
| 2-54 | 80 | 80% |
| 2-69 | 80 | 90% |
| 2-83 | 80 | 90% |
| 2-84 | 80 | 90% |
| 2-94 | 80 | 90% |
| 2-106 | 80 | 90% |
| 3-52 | 80 | 80% |
| 3-53 | 80 | 90% |
| 3-67 | 80 | 80% |

| Beispiel Nummer | Dosierung [g/ha] | ABUTH |
|---|---|---|
| 1-35 | 20 | 80% |
| 1-50 | 20 | 80% |
| 2-58 | 20 | 80% |
| 2-112 | 20 | 90% |
| 2-130 | 20 | 90% |
| 2-253 | 20 | 90% |
| 2-257 | 20 | 90% |

| Beispiel Nummer | Dosierung [g/ha] | AMARE |
|---|---|---|
| 2-13 | 80 | 80% |
| 2-15 | 80 | 80% |
| 2-54 | 80 | 90% |
| 2-68 | 80 | 100% |
| 2-69 | 80 | 90% |
| 2-80 | 80 | 90% |
| 2-83 | 80 | 100% |
| 2-84 | 80 | 100% |
| 2-94 | 80 | 90% |
| 2-106 | 80 | 100% |
| 3-52 | 80 | 100% |
| 3-53 | 80 | 100% |
| 3-67 | 80 | 100% |
| 3-82 | 80 | 100% |

| Beispiel Nummer | Dosierung [g/ha] | AMARE |
|---|---|---|
| 1-50 | 20 | 80% |
| 1-65 | 20 | 80% |
| 2-43 | 20 | 80% |
| 2-58 | 20 | 80% |
| 2-112 | 20 | 80% |

| Beispiel Nummer | Dosierung [g/ha] | MATIN |
|---|---|---|
| 2-68 | 80 | 90% |
| 2-69 | 80 | 90% |
| 2-80 | 80 | 80% |
| 2-83 | 80 | 100% |
| 2-84 | 80 | 100% |
| 2-106 | 80 | 90% |
| 3-52 | 80 | 90% |
| 3-53 | 80 | 80% |
| 3-67 | 80 | 90% |
| 3-82 | 80 | 100% |

| Beispiel Nummer | Dosierung [g/ha] | PHBPU |
|---|---|---|
| 2-50 | 80 | 80% |
| 2-54 | 80 | 80% |
| 2-68 | 80 | 90% |
| 2-69 | 80 | 80% |
| 2-83 | 80 | 90% |
| 2-84 | 80 | 90% |
| 2-106 | 80 | 80% |
| 3-52 | 80 | 100% |
| 3-67 | 80 | 100% |
| 3-82 | 80 | 90% |

| Beispiel Nummer | Dosierung [g/ha] | PHBPU |
|---|---|---|
| 1-65 | 20 | 80% |
| 2-22 | 20 | 80% |

| Beispiel Nummer | Dosierung [g/ha] | STEME |
|---|---|---|
| 2-21 | 20 | 80% |
| 2-255 | 20 | 80% |

| Beispiel Nummer | Dosierung [g/ha] | STEME |
|---|---|---|
| 2-13 | 80 | 80% |
| 2-68 | 80 | 100% |
| 2-69 | 80 | 100% |
| 2-80 | 80 | 90% |
| 2-83 | 80 | 100% |
| 2-84 | 80 | 100% |
| 2-94 | 80 | 100% |
| 2-106 | 80 | 100% |
| 3-52 | 80 | 100% |
| 3-53 | 80 | 90% |
| 3-67 | 80 | 100% |
| 3-82 | 80 | 100% |

| Beispiel Nummer | Dosierung [g/ha] | VIOTR |
|---|---|---|
| 2-54 | 80 | 100% |
| 2-68 | 80 | 100% |
| 2-69 | 80 | 100% |
| 2-80 | 80 | 90% |
| 2-83 | 80 | 100% |
| 2-84 | 80 | 100% |
| 2-94 | 80 | 80% |
| 2-106 | 80 | 100% |
| 3-52 | 80 | 100% |
| 3-53 | 80 | 90% |
| 3-67 | 80 | 100% |
| 3-82 | 80 | 100% |

| Beispiel Nummer | Dosierung [g/ha] | VIOTR |
|---|---|---|
| 1-35 | 20 | 80% |
| 1-50 | 20 | 80% |
| 2-21 | 20 | 80% |
| 2-22 | 20 | 100% |
| 2-43 | 20 | 90% |
| 2-130 | 20 | 90% |
| 2-255 | 20 | 80 |
| 2-257 | 20 | 80% |

| Beispiel Nummer | Dosierung [g/ha] | VERPE |
|---|---|---|
| 2-13 | 80 | 90% |
| 2-54 | 80 | 90% |
| 2-68 | 80 | 100% |
| 2-69 | 80 | 90% |
| 2-83 | 80 | 90% |
| 2-84 | 80 | 100% |
| 3-52 | 80 | 100% |
| 3-53 | 80 | 80% |
| 3-67 | 80 | 100% |
| 3-82 | 80 | 100% |

| Beispiel Nummer | Dosierung [g/ha] | VERPE |
|---|---|---|
| 2-112 | 20 | 80% |

In einem Vergleichsversuch wurde exemplarisch die herbizide Wirkung der erfindungsgemäßen Verbindung Nr. 2-53 mit der der aus WO 2013/076315 A2 bekannten Verbindung Nr. A-117 verglichen. Dabei zeigte sich anhand zahlreicher Schadpflanzen eine deutliche Überlegenheit der erfindungsgemäßen Verbindung.

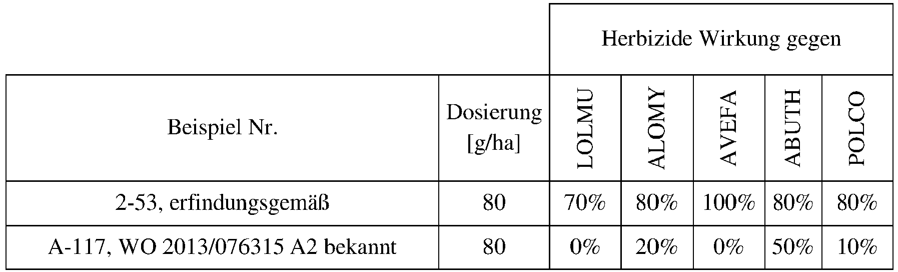

## Patentansprüche

1. Verbindungen der allgemeinen Formel (I) 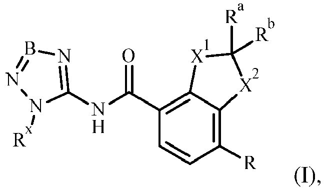 und deren agrochemisch verträglichen Salze, in welchen die Symbole und Indizes folgende Bedeutungen haben:
B bedeutet N oder CH,
X¹, X² bedeuten unabhängig voneinander jeweils jeweils O oder S(O)ₙ,
R bedeutet Wasserstoff, Halogen, (C₁-C₆)-Alkyl, Halogen-(C₁-C₆)-alkyl, (C₃-C₆)-Cycloalkyl, R'O oder R²(O)ₙS,
R^{a}, R^{b} bedeuten unabhängig voneinander jeweils Wasserstoff, Fluor, Chlor, Hydroxy, (C₁-C₆)-Alkyl, Halogen-(C₁-C₆)-alkyl, (C₁-C₆)-Alkyloxy, (C₁-C₆)-Alkylthio, Cyano, oder
R^{a} und R^{b} bilden gemeinsam mit dem Kohlenstoffatom, an dem sie gebunden sind eine Carbonyl- oder eine Thiocarbonylgruppe,
R^{x} bedeutet (C₁-C₆)-Alkyl, (C₁-C₆)-Alkyl-O-(C₁-C₆)-alkyl oder Phenyl,
R¹ bedeutet (C₁-C₆)-Alkyl oder Halogen-(C₁-C₆)-alkyl,
R² bedeutet (C₁-C₆)-Alkyl,
n bedeutet 0, 1 oder 2.

2. Verbindungen gemäß Anspruch 1, worin
B bedeutet N oder CH,
X¹, X² bedeuten unabhängig voneinander jeweils O oder S(O)ₙ,
R bedeutet Wasserstoff, Chlor, Brom, Iod, (C₁-C₃)-Alkyl, Halogen-(C₁-C₃)-alkyl, (C₃-C₆)-Cycloalkyl, R'O oder R²(O)ₙS,
R^{a}, R^{b} bedeuten unabhängig voneinander jeweils Wasserstoff, Fluor, Chlor, Hydroxy, (C₁-C₆)-Alkyl, Halogen-(C₁-C₆)-alkyl, (C₁-C₆)-Alkyloxy, (C₁-C₆)-Alkylthio, Cyano, oder
R^{a} und R^{b} bilden gemeinsam mit dem Kohlenstoffatom, an dem sie gebunden sind eine Carbonyl- oder eine Thiocarbonylgruppe,
R^{x} bedeutet (C₁-C₃)-Alkyl oder (C₁-C₃)-Alkyl-O-(C₁-C₃)-alkyl,
R¹ bedeutet (C₁-C₃)-Alkyl oder Halogen-(C₁-C₃)-alkyl,
R² bedeutet (C₁-C₃)-Alkyl,
n bedeutet 0, 1 oder 2.

3. Verbindungen gemäß Anspruch 1 oder 2, worin
B bedeutet N oder CH,
X¹, X² bedeuten unabhängig voneinander jeweils O oder S(O)ₙ,
R bedeutet Wasserstoff, Chlor, Brom, Iod, Methyl, Ethyl, Cyclopropyl, Trifluormethyl, Difluormethyl, Pentafluorethyl, Methylsulfanyl, Methylsulfinyl, Methylsulfonyl, Ethylsulfonyl oder Methoxy,
R^{a}, R^{b} bedeuten unabhängig voneinander jeweils Wasserstoff, Fluor, Chlor, Methyl, Ethyl, Trifluormethyl, Difluormethyl, Methyoxy, Ethoxy, Methylthio, Ethylthio, Cyano, oder
R^{a} und R^{b} bilden gemeinsam mit dem Kohlenstoffatom, an dem sie gebunden sind eine Carbonyl- oder eine Thiocarbonylgruppe,
R^{x} bedeutet Methyl, Ethyl, Propyl, Methoxymethyl, Methoxyethyl oder 2-Methoxy-2-methyl-1-propyl,
n bedeutet 0, 1 oder 2.

4. Herbizide Mittel enthaltend mindestens eine Verbindung gemäß einem der Ansprüche 1 bis 3 in Mischung mit Formulierungshilfsmitteln.

5. Herbizide Mittel gemäß Anspruch 4 enthaltend mindestens einen weiteren pestizid wirksamen Stoff aus der Gruppe Insektizide, Akarizide, Herbizide, Fungizide, Safener und Wachstumsregulatoren.

6. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder von herbiziden Mitteln nach Anspruch 4 oder 5 auf die Pflanzen oder auf den Ort des unerwünschten Pflanzenwachstums appliziert.

7. Verwendung von Verbindungen der Formel (I) gemäß einem der Ansprüche 1 bis 3 oder von herbiziden Mitteln nach Anspruch 4 oder 5 zur Bekämpfung unerwünschter Pflanzen.

8. Verwendung nach Anspruch 7, **dadurch gekennzeichnet, daß** die Verbindungen der Formel (I) zur Bekämpfung unerwünschter Pflanzen in Kulturen von Nutzpflanzen eingesetzt werden.

9. Verwendung nach Anspruch 8, **dadurch gekennzeichnet, daß** die Nutzpflanzen transgene Nutzpflanzen sind.

10. Verbindungen der Formel (II) 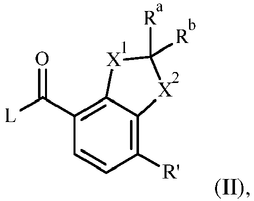 worin die Symbole und Indizes folgende Bedeutungen haben:
L bedeutet Chlor, Methoxy, Ethoxy oder Hydroxy,
X¹, X² bedeuten unabhängig voneinander jeweils O oder S(O)ₙ, wobei X¹ und X² nicht beide gleichzeitig O oder S(O)ₙ sind,
R' bedeutet SO₂Me, SO₂Et, Trifluormethyl, Difluormethyl oder Pentafluorethyl,
R^{a}, R^{b} bedeuten unabhängig voneinander jeweils Wasserstoff, Fluor, Chlor, Methyl, Ethyl, Trifluormethyl, Difluormethyl, Methyoxy, Ethoxy, Methylthio, Ethylthio, Cyano, oder
R^{a} und R^{b} bilden gemeinsam mit dem Kohlenstoffatom, an dem sie gebunden sind eine Carbonyl- oder eine Thiocarbonylgruppe,
n bedeutet 0, 1 oder 2.

11. Verbindungen der Formel (V) 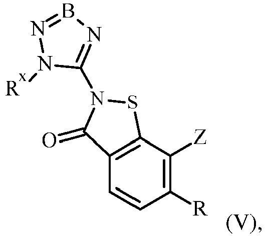 worin die Symbole und Indizes folgende Bedeutungen haben:
B bedeutet N oder CH,
Z bedeutet OH, SH,
R bedeutet Wasserstoff, Chlor, Brom, Iod, Methyl, Ethyl, Cyclopropyl, Trifluormethyl, Difluormethyl, Pentafluorethyl, Methylsulfanyl, Methylsulfinyl, Methylsulfonyl, Ethylsulfonyl, Methoxy,
R^{x} bedeutet Methyl, Ethyl, Propyl, Methoxymethyl, Methoxyethyl, 2-Methoxy-2-methyl-1-propyl.

## Claims

1. Compounds of the general formula (I) 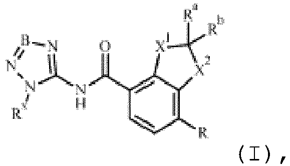 and agrochemically acceptable salts thereof, in which the symbols and indices are defined as follows:
B is N or CH,
X¹, X² are each independently O or S(O)ₙ,
R is hydrogen, halogen, (C₁-C₆) -alkyl, halo-(C₁-C₆)-alkyl, (C₃-C₆) -cycloalkyl, R¹O or R²(O)ₙS, R^{a}, R^{b} are each independently hydrogen, fluorine, chlorine, hydroxyl, (C₁-C₆) -alkyl, halo- (C₁-C₆) -alkyl, (C₁-C₆)-alkyloxy, (C₁-C₆)-alkylthio, cyano, or
R^{a} and R^{b} together with the carbon atom to which they are bonded form a carbonyl or a thiocarbonyl group,
R^{x} is (C₁-C₆)-alkyl, (C₁-C₆)-alkyl-O-(C₁-C₆)-alkyl or phenyl,
R¹ is (C₁-C₆)-alkyl or halo- (C₁-C₆)-alkyl,
R² is (C₁-C₆)-alkyl,
n is 0, 1 or 2.

2. Compounds according to Claim 1, in which
B is N or CH,
X¹, X² are each independently O or S(O)ₙ,
R is hydrogen, chlorine, bromine, iodine, (C₁-C₃)-alkyl, halo- (C₁-C₃) -alkyl, (C₃-C₆) -cycloalkyl, R¹O or R²(O)ₙS,
R^{a}, R^{b} are each independently hydrogen, fluorine, chlorine, hydroxyl, (C₁-C₆) -alkyl, halo-(C₁-C₆)-alkyl, (C₁-C₆)-alkyloxy, (C₁-C₆)-alkylthio, cyano, or
R^{a} and R^{b} together with the carbon atom to which they are bonded form a carbonyl or a thiocarbonyl group,
R^{x} is (C₁-C₃)-alkyl or (C₁-C₃)-alkyl-O-(C₁-C₃)-alkyl,
R¹ is (C₁-C₃)-alkyl or halo-(C₁-C₃)-alkyl,
R² is (C₁-C₃) -alkyl,
n is 0, 1 or 2.

3. Compounds according to Claim 1 or 2, in which
B is N or CH,
X¹, X² are each independently O or S(O)ₙ,
R is hydrogen, chlorine, bromine, iodine, methyl, ethyl, cyclopropyl, trifluoromethyl, difluoromethyl, pentafluoroethyl, methylsulfanyl, methylsulfinyl, methylsulfonyl, ethylsulfonyl or methoxy, R^{a}, R^{b} are each independently hydrogen, fluorine, chlorine, methyl, ethyl, trifluoromethyl, difluoromethyl, methoxy, ethoxy, methylthio, ethylthio, cyano, or
R^{a} and R^{b} together with the carbon atom to which they are bonded form a carbonyl or a thiocarbonyl group,
R^{x} is methyl, ethyl, propyl, methoxymethyl, methoxyethyl or 2-methoxy-2-methyl-1-propyl,
n is 0, 1 or 2.

4. Herbicidal compositions comprising at least one compound according to any of Claims 1 to 3, mixed with formulation auxiliaries.

5. Herbicidal compositions according to Claim 4, comprising at least one further pesticidally active substance from the group of insecticides, acaricides, herbicides, fungicides, safeners and growth regulators.

6. Method of controlling unwanted plants, **characterized in that** an effective amount of at least one compound of the formula (I) according to any of Claims 1 to 3 or of herbicidal compositions according to Claim 4 or 5 is applied to the plants or to the site of the unwanted vegetation.

7. Use of compounds of the formula (I) according to any of Claims 1 to 3 or of herbicidal compositions according to Claim 4 or 5 for controlling unwanted plants.

8. Use according to Claim 7, **characterized in that** the compounds of the formula (I) are used for controlling unwanted plants in crops of useful plants.

9. Use according to Claim 8, **characterized in that** the useful plants are transgenic useful plants.

10. Compounds of the formula (II) 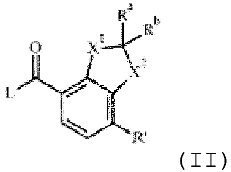 in which the symbols and indices are defined as follows:
L is chlorine, methoxy, ethoxy or hydroxyl, X¹, X² are each independently O or S(O)ₙ, where X¹ and
X² are not both simultaneously O or S(O)ₙ,
R' is SO₂Me, SO₂Et, trifluoromethyl, difluoromethyl or pentafluoroethyl,
R^{a}, R^{b} are each independently hydrogen, fluorine, chlorine, methyl, ethyl, trifluoromethyl, difluoromethyl, methoxy, ethoxy, methylthio, ethylthio, cyano, or
R^{a} and R^{b} together with the carbon atom to which they are bonded form a carbonyl or a thiocarbonyl group,
n is 0, 1 or 2.

11. Compounds of the formula (V) 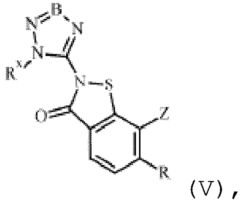 in which the symbols and indices are defined as follows:
B is N or CH,
Z is OH, SH,
R is hydrogen, chlorine, bromine, iodine, methyl, ethyl, cyclopropyl, trifluoromethyl, difluoromethyl, pentafluoroethyl, methylsulfanyl, methylsulfinyl, methylsulfonyl, ethylsulfonyl, methoxy,
R^{x} is methyl, ethyl, propyl, methoxymethyl, methoxyethyl, 2-methoxy-2-methyl-1-propyl.

## Revendications

1. Composés de formule générale (I) 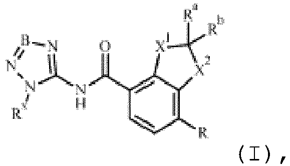 et leurs sels agrochimiquement acceptables, dans lesquels les symboles et les indices ont les significations suivantes :
B signifie N ou CH,
X¹, X² signifient chacun indépendamment l'un de l'autre O ou S(O)ₙ,
R signifie hydrogène, halogène, alkyle en (C₁-C₆), halogéno-alkyle en (C₁-C₆), cycloalkyle en (C₃-C₆), R¹O ou R²(O)ₙS,
R^{a}, R^{b} signifient chacun indépendamment l'un de l'autre hydrogène, fluor, chlore, hydroxy, alkyle en (C₁-C₆), halogéno-alkyle en (C₁-C₆),alkyloxy en (C₁-C₆), alkylthio en (C₁-C₆), cyano, ou
R^{a} et R^{b} forment, conjointement avec l'atome de carbone auquel ils sont liés, un groupe carbonyle ou un groupe thiocarbonyle,
R^{x} signifie alkyle en (C₁-C₆), alkyle en (C₁-C₆)-O-alkyle en (C₁-C₆) ou phényle,
R¹ signifie alkyle en (C₁-C₆) ou halogéno-alkyle en (C₁-C₆),
R² signifie alkyle en (C₁-C₆).
n signifie 0, 1 ou 2.

2. Composés selon la revendication 1, dans lesquels
B signifie N ou CH,
X¹, X² signifient chacun indépendamment l'un de l'autre O ou S(O)ₙ,
R signifie hydrogène, chlore, brome, iode, alkyle en (C₁-C₃), halogéno-alkyle en (C₁-C₃), cycloalkyle en (C₃-C₆), R¹O ou R²(O)ₙS,
R^{a}, R^{b} signifient chacun indépendamment l'un de l'autre hydrogène, fluor, chlore, hydroxy, alkyle en (C₁-C₆), halogéno-alkyle en (C₁-C₆), alkyloxy en (C₁-C₆), alkylthio en (C₁-C₆), cyano, ou
R^{a} et R^{b} forment, conjointement avec l'atome de carbone auquel ils sont liés, un groupe carbonyle ou un groupe thiocarbonyle,
R^{x} signifie alkyle en (C₁-C₃) ou alkyle en (C₁-C₃)-O-alkyle en (C₁-C₃),
R¹ signifie alkyle en (C₁-C₃) ou halogéno-alkyle en (C₁-C₃),
R² signifie alkyle en (C₁-C₃).
n signifie 0, 1 ou 2.

3. Composés selon la revendication 1 ou 2, dans lesquels
B signifie N ou CH,
X¹, X² signifient chacun indépendamment l'un de l'autre O ou S(O)ₙ,
R signifie hydrogène, chlore, brome, iode, méthyle, éthyle, cyclopropyle, trifluorométhyle, difluorométhyle, pentafluoroéthyle, méthylsulfanyle, méthylsulfinyle, méthylsulfonyle, éthylsulfonyle ou méthoxy, R^{a}, R^{b} signifient chacun indépendamment l'un de l'autre hydrogène, fluor, chlore, méthyle, éthyle, trifluorométhyle, difluorométhyle, méthoxy, éthoxy, méthylthio, éthylthio, cyano, ou
R^{a} et R^{b} forment, conjointement avec l'atome de carbone auquel ils sont liés, un groupe carbonyle ou un groupe thiocarbonyle,
R^{x} signifie méthyle, éthyle, propyle, méthoxyméthyle, méthoxyéthyle ou 2-méthoxy-2-méthyl-1-propyle,
n signifie 0, 1 ou 2.

4. Agent herbicide contenant au moins un composé selon l'une quelconque des revendications 1 à 3 en mélange avec des adjuvants de formulation.

5. Agent herbicide selon la revendication 4, contenant au moins une autre substance à activité pesticide du groupe des insecticides, des acaricides, des herbicides, des fongicides, des phytoprotecteurs et des régulateurs de croissance.

6. Procédé de lutte contre des plantes indésirables, **caractérisé en ce que** l'on applique sur les plantes ou sur le site de croissance des plantes indésirables une quantité efficace d'au moins un composé de formule (I) selon l'une quelconque des revendications 1 à 3 ou d'agents herbicides selon la revendication 4 ou 5.

7. Utilisation de composés de formule (I) selon l'une quelconque des revendications 1 à 3 ou d'agents herbicides selon la revendication 4 ou 5 pour lutter contre des plantes indésirables.

8. Utilisation selon la revendication 7, **caractérisée en ce que** les composés de formule (I) sont utilisés pour lutter contre des plantes indésirables dans des cultures de plantes utiles.

9. Utilisation selon la revendication 8, **caractérisée en ce que** les plantes utiles sont des plantes utiles transgéniques.

10. Composés de formule (II) 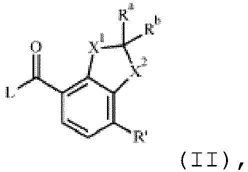 dans lesquels les symboles et les indices ont les significations suivantes :
L signifie chlore, méthoxy, éthoxy ou hydroxy, X¹, X² signifient chacun indépendamment l'un de l'autre O ou S(O)ₙ, X¹ et X² n'étant pas tous les deux simultanément O ou S(O)ₙ,
R' signifie SO₂Me, SO₂Et, trifluorométhyle, difluorométhyle ou pentafluoroéthyle,
R^{a}, R^{b} signifient chacun indépendamment l'un de l'autre hydrogène, fluor, chlore, méthyle, éthyle, trifluorométhyle, difluorométhyle, méthoxy, éthoxy, méthylthio, éthylthio, cyano, ou
R^{a} et R^{b} forment, conjointement avec l'atome de carbone auquel ils sont liés, un groupe carbonyle ou un groupe thiocarbonyle,
n signifie 0, 1 ou 2.

11. Composés de formule (V) 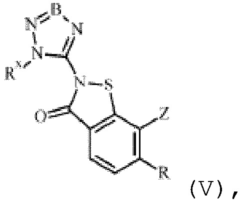 dans lesquels les symboles et les indices ont les significations suivantes :
B signifie N ou CH,
Z signifie OH, SH,
R signifie hydrogène, chlore, brome, iode, méthyle, éthyle, cyclopropyle, trifluorométhyle, difluorométhyle, pentafluoroéthyle, méthylsulfanyle, méthylsulfinyle, méthylsulfonyle, éthylsulfonyle, méthoxy,
R^{x} signifie méthyle, éthyle, propyle, méthoxyméthyle, méthoxyéthyle, 2-méthoxy-2-méthyl-1-propyle.
